(19) Europäisches Patentamt
European Patent Office
Office européen des brevets

(11) EP 3 920 187 A1

# (12) EUROPEAN PATENT APPLICATION

(43) Date of publication:
**08.12.2021 Bulletin 2021/49**

(51) Int Cl.:
***G16B 15/20*** *(2019.01)* ***G16C 20/40*** *(2019.01)*

(21) Application number: **21158937.9**

(22) Date of filing: **24.02.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.06.2020 JP 2020098832**

(71) Applicant: **FUJITSU LIMITED**
**Kanagawa 211-8588 (JP)**

(72) Inventors:
- **TERASHIMA, Chieko**
  **Kanagawa, 211-8588 (JP)**
- **TANIDA, Yoshiaki**
  **Kanagawa, 211-8588 (JP)**
- **SATO, Hiroyuki**
  **Kanagawa, 211-8588 (JP)**

(74) Representative: **Hoffmann Eitle**
**Patent- und Rechtsanwälte PartmbB**
**Arabellastraße 30**
**81925 München (DE)**

## (54) STRUCTURE SEARCH METHOD, STRUCTURE SEARCH APPARATUS, AND STRUCTURE SEARCH PROGRAM

(57) A structure search apparatus of searching for a stable structure of a compound in which a plurality of compound residues are bonded together includes: a memory; and a processor circuit coupled to the memory, the processor circuit being configured to perform processing, the processing including: executing an interaction potential identification processing configured to identify an interaction potential between a compound residue x and a compound residue y among the plurality of compound residues; executing a steric structure identification processing configured to identify a steric structure of the compound in a three-dimensional lattice space which is a set of lattice points by arranging the plurality of compound residues at lattice points in the three-dimensional lattice space in consideration of the interaction potential identified by the interaction potential identification processing; and in response to an identification result obtained, outputting the identification result indicating the identified steric structure of the compound.

FIG. 7

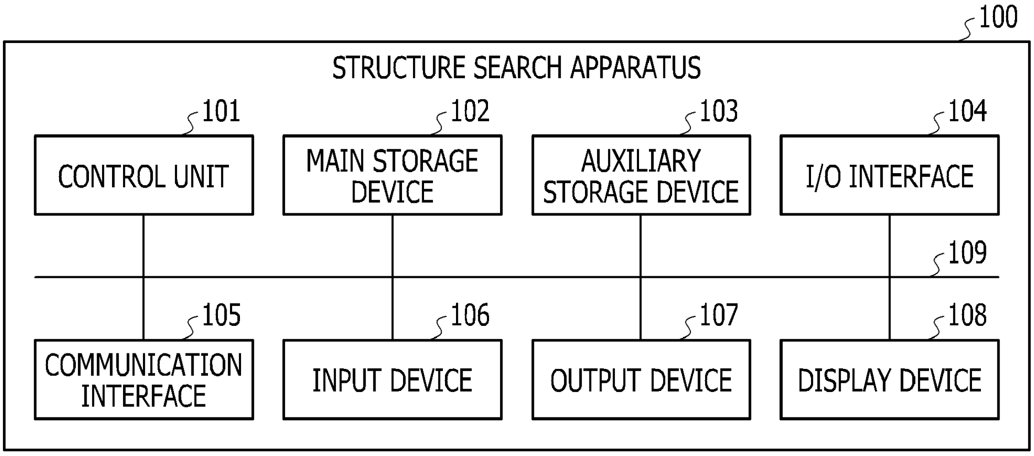


EP 3 920 187 A1

## Description

FIELD

The embodiments discussed herein are related to a structure search method, a structure search apparatus, a structure search program, and an interaction potential identification method.

BACKGROUND

In recent years, in a situation such as a drug discovery, there has been a case where a stable structure of a molecule relatively large in size has to be obtained by using a calculator (a computer). However, for example, all-atom calculation may have difficulty in searching for a stable structure of a molecule relatively large in size such as a peptide or protein within a practical time.

Therefore, for reducing the calculation time, a technique of roughly capturing the structure of a molecule (coarse graining) has been studied. As a technique for coarse graining of a molecular structure, for example, there has been studied a technique in which a protein is coarse-grained into a simple cubic lattice structure in a linear chain (single chain) based on information on a one-dimensional sequence of amino acid residues in the protein and is treated as a lattice protein. As for techniques using the lattice protein, there has been reported a technique of searching for a stable structure at high speed by using a quantum annealing technique (see, for example, NPL 1).

In such a technique using the lattice protein, a stable structure of a molecule is usually searched for in consideration of the magnitude of interaction between coarsely-grained amino acid residues. The interaction between coarsely-grained amino acid residues may be calculated by using, for example, an interaction potential between the amino acid residues.

The interaction potential is a sum of energy changes in coarsely-grained amino acid residues depending on the arrangement places of the amino acid residues. In the technique using the lattice protein described above, for example, the search for a stable structure of a molecule is performed by searching for a structure in which the energy obtained from the interaction potential is most stable.

As interaction potentials for, for example, normal natural amino acid residues forming a peptide or a protein, interaction potentials created by statistically processing the relative positions between the amino acid residues based on a database of proteins may be used (see, for example, NPL 2). Thus, in a case where amino acid residues forming a molecule are normal natural amino acid residues (20 types of natural amino acids), it is possible to use the commonly known interaction potentials created based on the database of proteins as described above.

Regarding amino acid residues other than the natural amino acid residues (such as chemically modified unnatural amino acid residues), there has been proposed a method of obtaining an interaction potential by molecular dynamics calculation on structures of extracted side chain portions of amino acid residues (see, for example, NPL 3). In a case where the amino acid residues forming the molecule include an amino acid residue other than the natural amino acid residues, it is not possible to use the commonly known interaction potentials as described above and it is requested to individually obtain an interaction potential for the amino acid residue other than the natural amino acid residues.

In recent years, in a situation such as drug discovery, there has been a case where a medium molecular compound or a polymer compound such as a peptide or protein is used as a drug. In this case, in order to improve the physiological activity and stability of the compound, an amino acid residue other than the natural amino acid residues is sometimes introduced into the compound. Therefore, in a situation such as drug discovery, it seems to be useful to search for a stable structure of a compound containing an amino acid residue other than the natural amino acid residues (hereinafter, also referred to as a "modified amino acid residue").

In the related art such as NPL 3, however, it is impossible to obtain an interaction potential by appropriately considering the structures of amino acid residues in a molecule, which results in an insufficient accuracy of the interaction potential, and accordingly does not allow an accurate search for a stable structure of the molecule.

SUMMARY

[Technical Problem]

In one aspect, an object of the present disclosure is to provide a structure search method, a structure search apparatus, and a structure search program which are capable of, in searching for a stable structure of a compound in which a plurality of compound residues are bonded together, accurately searching for the stable structure of the compound even when the compound contains a compound group having an unknown interaction potential.

In another aspect, an object of the present disclosure is to provide an interaction potential identification method which is capable of, in identifying an interaction potential between compound residues in a compound in which a plurality of compound residues are bonded together, accurately identifying an interaction potential for a compound group having an unknown interaction potential.

[Solution to Problem]

According to an aspect of the embodiments, there is provided a structure search apparatus of searching for a stable structure of a compound in which a plurality of compound residues are bonded together. In an example, the apparatus includes: a memory; and a processor circuit coupled to the memory, the processor circuit being configured to perform processing, the processing including: executing an interaction potential identification processing configured to identify an interaction potential between a compound residue x and a compound residue y among the plurality of compound residues; executing a steric structure identification processing configured to identify a steric structure of the compound in a three-dimensional lattice space which is a set of lattice points by arranging, based on the interaction potential identified by the interaction potential identification processing, the plurality of compound residues at lattice points in the three-dimensional lattice space; and in response to an identification result obtained by the steric structure identification processing, outputting the identification result indicating the identified steric structure of the compound.

In an example, the interaction potential identification processing identifies the interaction potential between the compound residue x and the compound residue y by using: first parameters for the compound residue x; and second parameters for the compound residue y.

In an example, the first parameters is configured such that a saturated group-containing structure portion x-1 and a saturated group-containing structure portion x+1 in a compound derivative x containing the compound residue x will not cause interaction.

In an example, the second parameters is configured such that a saturated group-containing structure portion y-1 and a saturated group-containing structure portion y+1 in a compound derivative y containing the compound residue y will not cause interaction.

In an example, the compound derivative x is a compound derivative obtained by adding the saturated group-containing structure portion x-1 and the saturated group-containing structure portion x+1 to the compound residue x,

In an example, the compound derivative y is a compound derivative obtained by adding the saturated group-containing structure portion y-1 and the saturated group-containing structure portion y+1 to the compound residue y.

In an example, the saturated group-containing structure portion x-1 is a saturated group-containing structure portion included in a compound residue x-1 adjacent to the compound residue x, the compound residue x-1 having a functional group x-1 bonded to a group involved in a linking bond in the compound residue x.

In an example, the saturated group-containing structure portion x-1 is composed of the functional group x-1 and a saturated group x-1, the saturated group x-1 being obtained by bonding hydrogen atoms to an atom bonded to the functional group x-1 to saturate a valence of that atom.

In an example, the saturated group-containing structure portion x+1 is a saturated group-containing structure portion included in a compound residue x+1 adjacent to the compound residue x, the compound residue x+1 having a functional group x+1 bonded to a group involved in a linking bond in the compound residue x.

In an example, the saturated group-containing structure portion x+1 is composed of the functional group x+1 and a saturated group x+1, the saturated group x+1 being obtained by bonding hydrogen atoms to an atom bonded to the functional group x+1 to saturate a valence of that atom.

In an example, the saturated group-containing structure portion y-1 is a saturated group-containing structure portion included in a compound residue y-1 adjacent to the compound residue y, the compound residue y-1 having a functional group y-1 bonded to a group involved in a linking bond in the compound residue y.

In an example, the saturated group-containing structure portion y-1 is composed of the functional group y-1 and a saturated group y-1, the saturated group y-1 being obtained by bonding hydrogen atoms to an atom bonded to the functional group y-1 to saturate a valence of that atom.

In an example, the saturated group-containing structure portion y+1 is a saturated group-containing structure portion included in a compound residue y+1 adjacent to the compound residue y, the compound residue y+1 having a functional group y+1 bonded to a group involved in a linking bond in the compound residue y.

In an example, the saturated group-containing structure portion y+1 is composed of the functional group y+1 and a saturated group y+1, the saturated group y+1 being obtained by bonding hydrogen atoms to an atom bonded to the functional group y+1 to saturate a valence of that atom.

[Effects of Invention]

In one aspect, the present disclosure may provide a structure search method, a structure search apparatus, and a structure search program which are capable of, in searching for a stable structure of a compound in which a plurality of compound residues are bonded together, accurately searching for the stable structure of the compound even when the compound contains a compound group having an unknown interaction potential.

In another aspect, the present disclosure may provide an interaction potential identification method which is capable of, in identifying an interaction potential between compound residues in a compound in which a plurality of compound residues are bonded together, accurately identifying an interaction potential for a compound group having an unknown interaction potential.

BRIEF DESCRIPTION OF DRAWINGS

FIG. 1A is a schematic diagram illustrating an example in a search for a stable structure of a protein by coarse-graining the protein;
FIG. 1B is a schematic diagram illustrating the example in the search for the stable structure of the protein by coarse-graining the protein;
FIG. 1C is a schematic diagram illustrating the example in the search for the stable structure of the protein by coarse-graining the protein;
FIG. 2A is a schematic diagram for explaining an example of a diamond encoding method;
FIG. 2B is a schematic diagram for explaining the example of the diamond encoding method;
FIG. 2C is a schematic diagram for explaining the example of the diamond encoding method;
FIG. 2D is a schematic diagram for explaining the example of the diamond encoding method;
FIG. 2E is a schematic diagram for explaining the example of the diamond encoding method;
FIG. 3 illustrates an example of a structure of an amino acid residue for use in molecular dynamics calculation for calculating an interaction potential between amino acid residues in the related art;
FIG. 4 illustrates an example of a state where amino acid residues are bonded together in a peptide;
FIG. 5 illustrates an example of a structure of an amino acid derivative x prepared in one example of the technique disclosed herein;
FIG. 6 illustrates an example of interactions between an amino acid derivative x and an amino acid derivative y in the example of the technique disclosed herein;
FIG. 7 illustrates a hardware configuration example of a structure search apparatus disclosed herein;
FIG. 8 illustrates another hardware configuration example of a structure search apparatus disclosed herein;
FIG. 9 illustrates a functional configuration example of a structure search apparatus disclosed herein;
FIG. 10 illustrates an example of a flowchart for identifying an interaction potential for use to search for a stable structure of a peptide by using one example of the technique disclosed herein;
FIG. 11 illustrates an example of a flowchart for searching for a stable structure of a peptide in consideration of an interaction potential identified by using the example of the technique disclosed in the above;
FIG. 12 is a diagram illustrating an example of a case where lattice points within an area having a radius r are each denoted by $S_r$;
FIG. 13A is a diagram illustrating an example of a set of destination lattice points for an amino acid residue;
FIG. 13B is a diagram illustrating an example of a set of destination lattice points for amino acid residues;
FIG. 13C is a diagram illustrating an example of a set of destination lattice points for amino acid residues;
FIG. 13D is a diagram illustrating an example of a set of destination lattice points for amino acid residues;
FIG. 14 illustrates an example of a three-dimensional representation of $S_1$, $S_2$, and $S_3$;
FIG. 15A illustrates an example of allocation of space information to bits $X_1$ to $X_n$;
FIG. 15B illustrates the example of the allocation of the space information to the bits $X_1$ to $X_n$;
FIG. 15C illustrates the example of the allocation of the space information to the bits $X_1$ to $X_n$;
FIG. 16 is a diagram for explaining an example of $H_{one}$;
FIG. 17 is a diagram for explaining an example of $H_{olap}$;
FIG. 18 is a diagram for explaining an example of $H_{conn}$;
FIG. 19A is a diagram for explaining the example of $H_{pair}$;
FIG. 19B is a diagram for explaining an example of $H_{pair}$;
FIG. 20A illustrates an example of particles representing a main chain and side chains in coarse-grained amino acid residues;
FIG. 20B illustrates an example of a set of lattice points for arranging the coarse-grained amino acid residues illustrated in FIG. 20A;
FIG. 20C illustrates an example of a state where the coarse-grained amino acid residues illustrated in FIG. 20A are arranged at lattice points;
FIG. 21 illustrates an example of a functional configuration of an annealing machine for use in an annealing method;

FIG. 22 illustrates an example of an operation flow of a transition control unit;
FIG. 23 illustrates PMF between leucine residues calculated in Example 1;
FIG. 24A illustrates an example of a chemical formula of a N-methylphenylalanine residue in a peptide;
FIG. 24B illustrates an example of a structure of a N-methylphenylalanine derivative prepared in Example 2;
FIG. 25 illustrates a PMF between a N-methylphenylalanine residue and a valine residue calculated in Example 2;
FIG. 26 illustrates a superposition of a search result of a stable structure of a cyclic peptide searched out in Example 3 on a structure of a cyclic peptide identified by NMR; and
FIG. 27 illustrates an example of a relationship in searching for a stable structure of a peptide by identifying an interaction potential between amino acid residues in one embodiment of the technique disclosed herein and in the related art.

DESCRIPTION OF EMBODIMENT(S)

(Structure Search Method)

The technique disclosed herein is based on the finding by the present inventors that a search for a stable structure of a compound in which multiple compound residues are bonded together in the related art results in an insufficient accuracy of the search for the stable structure of the compound in a case where the compound contains a compound group having an unknown interaction potential. Prior to detailed description of the technique disclosed herein, problems and others in the related art will be described by taking, as an example, a case where a compound as a stable structure search target is a peptide (protein).

In recent years, in a case of using a peptide (protein) as a drug in a situation such as drug discovery, an amino acid residue other than the natural amino acid residues (modified amino acid residue) has been sometimes introduced into the peptide (protein) in order to improve the physiological activity and stability. For this reason, in a situation such as drug discovery, it seems to be useful to search for a stable structure of a peptide (protein) containing a modified amino acid residue having an unknown interaction potential.

A search for a stable structure of a peptide (protein) may use a technique in which amino acid residues forming the protein are coarse-grained and treated as the lattice protein as described above. As one of techniques using the lattice protein, description will be given of a method of obtaining a folding structure as a stable structure of a protein by a diamond encoding method.

In a structure search for a protein (or peptide) using the lattice protein, coarse graining of the protein is firstly performed. The coarse graining of the protein is performed by, for example, creating a coarse-grained model in which atoms 2 constituting the protein are coarse-grained into coarse-grained particles 1A, 1B, and 1C, each of which is a unit per amino acid residue, as illustrated in FIG. 1A.

Next, the created coarse-grained model is used to search for a stable bonding structure. FIG. 1B illustrates an example of a case where a bonding structure in which the coarse-grained particle 1C is located at an end point of an arrow is stable. The search for a stable bonding structure is performed by the diamond encoding method to be described later.

As illustrated in FIG. 1C, the coarse-grained model is restored to an all-atom model based on the stable bonding structure searched out by using the diamond encoding method.

The diamond encoding method is a method in which each of coarse-grained particles (a coarse-grained model) in a chain of amino acids forming a protein is allocated to one of lattice points in a diamond lattice, and is capable of representing the three-dimensional structure of the protein.

For simplification of explanation, the diamond encoding method for a two-dimensional case will be described below by way of example.

FIG. 2A illustrates an example of a structure in which a linear pentapeptide having five amino acid residues bonded together has a linear structure. In FIGs. 2A to 2E, a number in each circle represents a number of the corresponding amino acid residue in the linear pentapeptide.

In the diamond encoding method, once an amino acid residue No. 1 is firstly arranged at the center of the diamond lattice, a place where an amino acid residue No. 2 is arrangeable as illustrated in FIG. 2A is limited to places (places with No. 2) adjacent to the center as illustrated in FIG. 2B. Next, a place where an amino acid residue No. 3 bonded to the amino acid residue No. 2 is arrangeable is limited to places (places with No. 3) adjacent to the places with No. 2 in FIG. 2B, as illustrated in FIG. 2C.

A place where an amino acid residue No. 4 bonded to the amino acid residue No. 3 is arrangeable is limited to places (places with No. 4) adjacent to the places with No. 3 in FIG. 2C, as illustrated in FIG. 2D. A place where an amino acid residue No. 5 bonded to the amino acid residue No. 4 is arrangeable is limited to places (places with No. 5) adjacent to the places with No. 4 in FIG. 2D, as illustrated in FIG. 2E.

Thus, it is possible to represent the coarse-grained structure of the protein by linking the identified places where the amino acid residues are arrangeable in the order of the numbers of the amino acid residues.

Using the Diamond encoding method or the like, coarse-grained amino acid residues are sequentially arranged at corresponding lattice points in a three-dimensional lattice space which is a set of lattice points as described above, so that it is possible to create the steric structure of the protein (peptide) in the three-dimensional lattice space.

In searching for a stable structure of a protein (peptide) by creating a steric structure of the protein in a three-dimensional lattice space, it is requested to appropriately select a combination of arrangement places of coarse-grained amino acid residues in the three-dimensional lattice space. In order to appropriately select the combination of the arrangement places of the coarse-grained amino acid residues, for example, it is preferable to determine the arrangement places of the amino acid residues such that the arrangement places of the amino acid residues satisfy predetermined conditions.

The conditions for the arrangement places of the amino acid residues may be, for example, conditions that allow a steric structure created by arranging the amino acid residues in a three-dimensional lattice space to be a structure that may exist consistently as a protein (peptide) and to be an energetically stable structure. Such conditions may be, for example, conditions including the following three constraints and an interaction between amino acid residues.

[Constraints]

- Each of amino acid residues forming a protein (peptide) exists alone;
- Two or more of the amino acid residues forming the protein (peptide) do not coexist at one lattice point; and
- Among the amino acid residues forming the protein (peptide), amino acid residues peptide-bonded to each other exist at lattice points adjacent to each other in the three-dimensional lattice space.

[Interaction]

- An interaction between amino acid residues not peptide-bonded to each other among the amino acid residues forming the protein (peptide)

For example, in searching for a stable structure of a protein by creating a steric structure of the protein in a three-dimensional lattice space, it is preferable to search for a structure which satisfies the above-described three constraints and has a stable interaction between the amino acid residues not peptide-bonded to each other (energy is low).

The interaction between amino acid residues not peptide-bonded to each other may be calculated by using, for example, an interaction potential between the amino acid residues as described above.

The interaction potential is a sum of energy changes in coarsely-grained amino acid residues depending on the arrangement places of the amino acid residues.

As interaction potentials for, for example, normal natural amino acid residues (20 types of natural amino acids) forming a protein, interaction potentials created by statistically processing the relative positions between the amino acid residues based on a database of proteins may be used. For example, in a case where amino acid residues forming a molecule are normal natural amino acid residues (20 types of natural amino acids), it is possible to use the commonly known interaction potentials created based on the database of proteins.

On the other hand, for amino acid residues other than the natural amino acid residues (such as chemically modified unnatural amino acid residues), there is known a technique of using an interaction potential obtained by molecular dynamics calculation on structures of extracted side chain portions of amino acid residues as in NPL 3. In a case where the amino acid residues forming the molecule include an amino acid residue other than the natural amino acid residues, it is not possible to use the commonly known interaction potentials and it is requested to individually calculate and obtain the interaction potential as described above.

As described above, the related art such as NPL 3 is a technique of calculating an interaction potential between amino acid residues by performing molecular dynamics calculation on the structures of the extracted side chain portions of the amino acids (side chain analogs) when calculating the interaction potential. In this way, in the related art, an interaction potential is calculated by performing molecular dynamics calculation on a structure of an extracted side chain portion of an amino acid (side chain analog) and a structure of an amino acid molecule alone.

FIG. 3 illustrates an example of a structure of an amino acid residue for use in molecular dynamics calculation for calculating an interaction potential between amino acid residues in the related art.

As illustrated in FIG. 3, an amino acid residue in a peptide forms peptide bonds with amino acid residues present adjacent to the concerned amino acid residue. For example, the amino acid residue surrounded by a broken line in FIG.

3 is present in the peptide while being peptide-bonded to the adjacent amino acid residues in the peptide. Therefore, a structure that the amino acid residue surrounded by the broken line in FIG. 3 may have in the peptide is influenced by the peptide bonds with the amino acid residues adjacent to the concerned amino acid residue. For example, the structure of a portion of an amino acid residue as a calculation target (for which the interaction potential is to be calculated), the portion forming the main chain in a peptide, is influenced by the peptide bonds between the concerned amino acid residue and the adjacent amino acid residues.

However, in the related art, molecular dynamics calculation is performed on a structure of an extracted side chain portion of an amino acid (side chain analog) surrounded by a solid line in FIG. 3 and a structure of an amino acid molecule alone surrounded by a broken line in FIG. 3. For this reason, the related art does not take into consideration the influence of the peptide bonds with the amino acid residues adjacent to the amino acid residue as the calculation target. As a result, in the related art, it is not possible to take into consideration the influence that a structure of a portion forming the main chain in the peptide in an amino acid residue as a calculation target (for which an interaction potential is to be calculated) receives from peptide bonds between the concerned amino acid residue and its adjacent amino acid residues.

For this reason, in the related art, it is not possible to appropriately evaluate an interaction between the main chain in the peptide in the amino acid residue as the calculation target (for which the interaction potential is to be calculated) and the side chain of the concerned amino acid residue. In the related art, for example, it is not possible to appropriately evaluate the interaction between the main chain in the peptide in the amino acid residue and the side chain of the concerned amino acid residue, which results in an insufficient accuracy of the interaction potential, and accordingly does not allow an accurate search for the steric structure of the peptide.

As the case where the compound is a peptide and the compound residues are amino acid residues has been described as an example, the interaction potential for a compound containing a compound residue having an unknown interaction potential is calculated by using a structure of an extracted portion of the compound and so on in the related art. Therefore, in the related art, it is not possible to take into consideration an influence that a structure of a portion forming a main chain of a compound in a compound residue receives from linking bonds between the concerned compound residue and its adjacent compound residues. As a result, in the related art, the accuracy of the calculated interaction potential is so low that it is not possible to accurately search for the steric structure of the compound.

Under these circumstances, the inventors have made extensive studies on an apparatus and so on capable of, in searching for a stable structure of a compound in which multiple compound residues are bonded together, accurately searching for a stable structure of the compound even in a case where the compound contains a compound group having an unknown interaction potential and have obtained the following findings.

For example, the inventors have found that the following structure search method and the like are capable of, in searching for a stable structure of a compound in which multiple compound residues are bonded together, searching for the stable structure of the compound with high accuracy even when the compound contains a compound group having an unknown interaction potential.

A structure search method as an example of the technique disclosed herein is a computer-based structure search method of searching for a stable structure of a compound in which a plurality of compound residues are bonded together, the method comprising:

identifying an interaction potential between a compound residue x and a compound residue y among the plurality of compound residues; and

identifying a steric structure of the compound in a three-dimensional lattice space which is a set of lattice points by sequentially arranging the plurality of compound residues at certain lattice points in the three-dimensional lattice space in consideration of the interaction potential obtained in the identifying an interaction potential, wherein

the identifying an interaction potential includes identifying the interaction potential between the compound residue x and the compound residue y by

setting parameters for the compound residue x such that a saturated group-containing structure portion x-1 and a saturated group-containing structure portion x+1 in a compound derivative x containing the compound residue x will not cause interaction,

the saturated group-containing structure portion x-1 included in a compound residue x-1 being adjacent to the compound residue x and having a functional group bonded to a group involved in a linking bond in the compound residue x,

the saturated group-containing structure portion x-1 composed of the functional group and a saturated group obtained by bonding hydrogen atoms to an atom bonded to the functional group to saturate the valence of the atom,

the saturated group-containing structure portion x+1 included in a compound residue x+1 being adjacent to the compound residue x and having a functional group bonded to a group involved in a linking bond in the compound residue x,

the saturated group-containing structure portion x+1 composed of the functional group and a saturated group obtained by bonding hydrogen atoms to an atom bonded to the functional group to saturate the valence of the atom,
the compound derivative x obtained by adding the saturated group-containing structure portion x-1 and the saturated group-containing structure portion x+1 to the compound residue x, and
setting parameters for the compound residue y such that a saturated group-containing structure portion y-1 and a saturated group-containing structure portion y+1 in a compound derivative y containing the compound residue y will not cause interaction,
the saturated group-containing structure portion y-1 included in a compound residue y-1 being adjacent to the compound residue x and having a functional group bonded to a group involved in a linking bond in the compound residue y,
the saturated group-containing structure portion y-1 composed of the functional group and a saturated group obtained by bonding hydrogen atoms to an atom bonded to the functional group to saturate the valence of the atom,
the saturated group-containing structure portion y+1 included in a compound residue y+1 being adjacent to the compound residue y and having a functional group bonded to a group involved in a linking bond in the compound residue y,
the saturated group-containing structure portion y+1 composed of the functional group and a saturated group obtained by bonding hydrogen atoms to an atom bonded to the functional group to saturate the valence of the atom,
the compound derivative y obtained by adding the saturated group-containing structure portion y-1 and the saturated group-containing structure portion y+1 to the compound residue y.

In a case where the above-described structure search method is applied to a peptide (protein) formed of amino acid residues, the structure search method may be rephrased as follows.

For example, another example of a structure search method disclosed herein is a computer-based structure search method of searching for a stable structure of a peptide in which a plurality of amino acid residues are bonded together, the method comprising:

identifying an interaction potential between an amino acid residue x and an amino acid residue y among the plurality of amino acid residues; and
identifying a steric structure of the peptide in a three-dimensional lattice space which is a set of lattice points by sequentially arranging the plurality of amino acid residues at certain lattice points in the three-dimensional lattice space in consideration of the interaction potential obtained in the identifying an interaction potential, wherein
the identifying an interaction potential includes identifying the interaction potential between the compound residue x and the compound residue y by
setting parameters for the amino acid residue x such that an acetyl structure portion and a N-methyl structure portion in an amino acid derivative x containing the amino acid residue x will not cause interaction,
the acetyl structure portion included in an amino acid residue x-1 being adjacent to the amino acid residue x and having a carbonyl group bonded to an amino group involved in a peptide bond in the amino acid residue x,
the acetyl structure portion composed of the carbonyl group and a methyl group obtained by bonding hydrogen atoms to a carbon atom bonded to the carbonyl group to saturate the valence of the carbon atom,
the N-methyl structure portion included in an amino acid residue x+1 being adjacent to the amino acid residue x and having an amino group bonded to a carbonyl group involved in a peptide bond in the amino acid residue x,
the N-methyl structure portion composed of the amino group and a methyl group obtained by bonding hydrogen atoms to a carbon atom bonded to the amino group to saturate the valence of the carbon atom,
the amino acid derivative x obtained by adding the acetyl structure portion and the N-methyl structure portion to the amino acid residue x, and
setting parameters for the amino acid residue y such that an acetyl structure portion and a N-methyl structure portion in an amino acid derivative y containing the amino acid residue y will not cause interaction,
the acetyl structure portion included in an amino acid residue y-1 being adjacent to the amino acid residue y and having a carbonyl group bonded to an amino group involved in a peptide bond in the amino acid residue y,
the acetyl structure portion composed of the carbonyl group and a methyl group obtained by bonding hydrogen atoms to a carbon atom bonded to the carbonyl group to saturate the valence of the carbon atom,
the N-methyl structure portion included in an amino acid residue y+1 being adjacent to the amino acid residue y and having an amino group bonded to a carbonyl group involved in a peptide bond in the compound residue y,
the N-methyl structure portion composed of the amino group and a methyl group obtained by bonding hydrogen atoms to a carbon atom bonded to the amino group to saturate the valence of the carbon atom,
the amino acid derivative y obtained by adding the acetyl structure portion and the N-methyl structure portion to the amino acid residue y.

Hereinafter, an example of the technique disclosed herein in which a search for a stable structure of a compound achieves an accurate search for the stable structure of the compound even when the compound contains a compound group having an unknown interaction potential will be descried by taking a case where the compound is a peptide as an example.

First, in the example of the technique disclosed herein, an interaction potential between an amino acid residue x and an amino acid residue y among multiple amino acid residues is identified (calculated). In the example of the technique disclosed herein, the multiple amino acid residues are sequentially arranged at lattice points in a three-dimensional lattice space which is a set of lattice points in consideration of the interaction potential obtained in a step of identifying an interaction potential. In the example of the technique disclosed herein, a steric structure of the peptide is identified (created) in the three-dimensional lattice space by sequentially arranging the multiple amino acid residues.

As described above, the technique disclosed herein searches for the stable structure of the peptide by identifying the interaction potential between the amino acid residue x and the amino acid residue y, and searching for the steric structure of the peptide in consideration of the identified interaction potential.

In the example of the technique disclosed herein, what is captured for the amino acid residue x in the step of identifying an interaction potential includes not only a portion composed of solely the amino acid residue but also partial structures of amino acid residues bonded adjacent to the concerned amino acid residue.

In the example of the technique disclosed herein, for example, a portion of an amino acid residue adjacent by the side of an amino group involved in a peptide bond at one end of the amino acid residue x and a portion of an amino acid residue adjacent by the side of a carbonyl group involved in a peptide bond at the other end of the amino acid residue x are captured for the amino acid residue x.

With reference to the drawings, detailed description regarding an amino acid residue x will be given of how to capture the partial structures of the amino acid residues bonded adjacent to the concerned amino acid residue x in the example of the technique disclosed herein.

FIG. 4 illustrates an example of how amino acid residues are bonded together in a peptide. In FIG. 4, an amino acid residue x located in the center of FIG. 4 is bonded to an amino acid residue x+1 adjacent on the left side of the amino acid residue x and an amino acid residue x-1 adjacent on the right side of the amino acid residue x. The amino acid residue x has a side chain R, a carbon atom (Ca atom) bonded to R, an amino group bonded to the Ca atom, and a carbonyl group bonded to the Ca atom.

As illustrated in FIG. 4, the amino group (NH group) in the amino acid residue x is bonded to a carbonyl group (CO group) in the amino acid residue x-1 adjacent on the right side of the amino acid residue x, and the carbonyl group in the amino acid residue x is bonded to an amino group in the amino acid residue x+1 adjacent on the left side of the amino acid residue x.

In the example of the technique disclosed herein, an amino acid derivative x is prepared in such a way that partial structures included in a region surrounded by a solid rectangular frame line in FIG. 4 are taken out from the structures of the amino acid residue x+1 and the amino acid residue x-1 which are bonded adjacent to the amino acid residue x, and the partial structures are added to the amino acid residue x.

In the example of the technique disclosed herein, in order to consider two amino acid residues bonded adjacent to the amino acid residue x, the amino acid derivative x containing the amino acid residue x is prepared for the amino acid residue x by performing the following two processes:

- to acetylate the amino group side of the amino acid residue x involved in the peptide bond; and
- to N-methylate the carbonyl group-side of the amino acid residue x involved in the peptide bond.

Here, two amino acid residues bonded adjacent to the amino acid residue x as illustrated in FIG. 4 are referred to as an amino acid residue x-1 and an amino acid residue x+1, respectively.

The amino acid residue x-1 is an amino acid residue being adjacent to the amino acid residue x and having a carbonyl group bonded to the amino group involved in the peptide bond in the amino acid residue x. The amino acid residue x+1 is an amino acid residue being adjacent to the amino acid residue x and having an amino group bonded to the carbonyl group involved in the peptide bond in the amino acid residue x.

Under these conditions, a structure portion in the amino acid residue x-1 composed of the carbonyl group and a methyl group obtained by bonding hydrogen atoms to the carbon atom bonded to the carbonyl group to saturate the valence of the carbon atom is referred to as an acetyl structure portion. Then, a structure portion in the amino acid residue x+1 composed of the amino group and a methyl group obtained by bonding hydrogen atoms to the carbon atom bonded to the amino group to saturate the valence of the carbon atom is referred to as a N-methyl structure portion.

For example, in the example of the technique disclosed herein, the amino group-side terminal portion of the amino acid residue x involved in the peptide bond is acetylated, whereas the carbonyl group-side terminal portion of the amino acid residue x involved in the peptide bond is N-methylated, thereby enabling the structure of the main chain in the peptide in the amino acid residue x to be considered.

FIG. 5 illustrates an example of the structure of the amino acid derivative x prepared in the example of the technique disclosed herein. In FIG. 5, a region surrounded by a right frame line represents the acetyl structure portion composed of the carbonyl group and the methyl group obtained by bonding the hydrogen atoms to the carbon atom bonded to the carbonyl group to saturate the valence of the carbon atom (the structure in which the amino group-side terminal of the amino acid residue x is acetylated). In FIG. 5, a region surrounded by a left frame line represents the N-methyl structure portion composed of the amino group and the methyl group obtained by bonding the hydrogen atoms to the carbon atom bonded to the amino group to saturate the valence of the carbon atom (the structure in which the carbonyl group-side terminal of the amino acid residue x is N-methylated).

In the example of the technique disclosed herein, prepared is the amino acid derivative x containing the amino acid residue x and obtained by adding the acetyl structure portion and the N-methyl structure portion to the amino acid residue x as illustrated in FIG. 5. This preparation enables the influence of the peptide bonds with the two amino acid residues bonded adjacent to the amino acid residue x to be taken into consideration.

In the example of the technique disclosed herein, the aforementioned processes for the amino acid residue x are also performed for the amino acid residue y. For example, in the example of the technique disclosed herein, an amino acid derivative y containing the amino acid residue y and obtained by adding an acetyl structure portion and a N-methyl structure portion to the amino acid residue y is prepared by performing the same processes as the aforementioned processes for the amino acid residue x. This preparation enables the influence of the peptide bonds with the two amino acid residues bonded adjacent to the amino acid residue y to be taken into consideration.

In the example of the technique disclosed herein, an interaction potential between the amino acid residue x and the amino acid residue y among the multiple amino acid residues is identified (calculated), and the steric structure of the peptide is identified (created) in the three-dimensional lattice space in consideration of the identified interaction potential. Therefore, in the identification of the interaction potential, it is preferable to identify the interaction potential by accurately evaluating the interaction between the amino acid residue x and the amino acid residue y.

For this reason, in order to identify the interaction potential between the amino acid residue x and the amino acid residue y, it is preferable to keep the added structure portions from interacting with other molecules in evaluating the interaction between the amino acid derivative x and the amino acid derivative y thus prepared.

To this end, in the example of the technique disclosed herein, parameters are set such that the acetyl structure portion and the N-methyl structure portion in the amino acid derivative x will not cause interaction. Similarly, in the example of the technique disclosed herein, parameters are set such that the acetyl structure portion and the N-methyl structure portion in the amino acid derivative y will not cause interaction. For example, in the example of the technique disclosed herein, the parameters are set such that none of the acetyl structure portions and the N-methyl structure portions in the amino acid derivative x and the amino acid derivative y will cause interaction.

In the example of the technique disclosed herein, the acetyl structure portion and the N-methyl structure portion in the amino acid derivative x cause action due to chemical bonds with a portion composed of the amino acid residue x (main portion of the amino acid derivative x) in the molecule of the amino acid derivative x. Similarly, in the example of the technique disclosed herein, the acetyl structure portion and the N-methyl structure portion in the amino acid derivative y cause action due to chemical bonds with a portion composed of the amino acid residue y (main portion of the amino acid derivative y) in the molecule of the amino acid derivative y.

FIG. 6 illustrates an example of interactions between the amino acid derivative x and the amino acid derivative y in the example of the technique disclosed herein.

In the example of the technique disclosed herein, for example, the parameters are set such that the acetyl structure portions and the N-methyl structure portions in the amino acid derivative x and the amino acid derivative y will not cause interaction with another molecule as illustrated in FIG. 6. In FIG. 6, portions between which the interaction is to be taken into consideration are linked by a solid line arrow, and portions between which the interaction is not to be taken into consideration (portions for which the parameters are set such that the portions will not cause interaction) are linked by a dotted line arrow.

As illustrated in FIG. 6, in the example of the technique disclosed herein, for example, the parameters are set such that the amino acid residue x in the amino acid derivative x will cause interaction with the amino acid residue y in the amino acid derivative y. In the example of the technique disclosed herein, in the case of identifying the interaction potential in consideration of an influence of a solvent molecule (such as a water molecule), for example, the parameters are set such that the amino acid residue x in the amino acid derivative x will also interact with the solvent molecule as illustrated in FIG. 6.

In the example of the technique disclosed herein, the parameters are set such that the acetyl structure portion and the N-methyl structure portion in the amino acid derivative x will not cause interaction with any of the amino acid residue y, the acetyl structure portion, and the N-methyl structure portion in the amino acid derivative y. In the example of the technique disclosed herein, in the case of identifying the interaction potential in consideration of an influence of a solvent molecule, for example, the parameters are set such that none of the acetyl structure portion and the N-methyl structure portion in the amino acid derivative x will interact with the solvent molecule as illustrated in FIG. 6.

In the example of the technique disclosed herein, in the case of identifying the interaction potential in consideration of an influence of solvent molecules, for example, the parameters are preferably set such that the solvent molecules will interact with each other as illustrated in FIG. 6.

In the example of the technique disclosed herein, by setting the parameters such that none of the acetyl structure portions and the N-methyl structure portions will cause interaction as in the example in FIG. 6, it is possible to evaluate the interaction between the amino acid residue x in the amino acid derivative x and the amino acid residue y in the amino acid derivative y. In the example of the technique disclosed herein, this makes it possible to accurately identify the interaction potential between the amino acid residue x and the amino acid residue y by appropriately evaluating the interaction between the amino acid residue x and the amino acid residue y.

In the example of the technique disclosed herein, the amino acid derivative x containing the amino acid residue x and obtained by adding the acetyl structure portion and the N-methyl structure portion to the amino acid residue x is prepared as described above. In the example of the technique disclosed herein, the amino acid derivative y containing the amino acid residue y and obtained by adding the acetyl structure portion and the N-methyl structure portion to the amino acid residue y is prepared.

In this way, in the example of the technique disclosed herein, for each of the amino acid residue x and the amino acid residue y, it is possible to consider the influence of the peptide bonds with the two adjacent bonded amino acid residues. For example, in the example of the technique disclosed herein, it is possible to appropriately evaluate the interaction between the main chain in the peptide in each amino acid residue as a calculation target (for which the interaction potential is to be calculated) and the side chain of the concerned amino acid residue.

In the example of the technique disclosed herein, the parameters are set such that none of the acetyl structure portions and the N-methyl structure portions in the amino acid derivative x and the amino acid derivative y will cause interaction.

In this way, in the example of the technique disclosed herein, it is possible to appropriately evaluate the interaction between the amino acid residue x in the amino acid derivative x and the amino acid residue y in the amino acid derivative y.

Therefore, in the example of the technique disclosed herein, it is possible to accurately evaluate the interaction between the main chain in the peptide in each amino acid residue and the side chain of the concerned amino acid residue, and the interaction between amino acid residues, and accordingly identify a highly accurate interaction potential.

In the example of the technique disclosed herein, since a steric structure of a peptide is identified in consideration of the highly accurate interaction potential as described above, it is possible to accurately search for a stable structure of the peptide even when the peptide contains an amino acid residue having an unknown interaction potential.

In the example of the technique disclosed herein, the above description has been provided by taking, as an example, the case where a compound as a stable structure search target is a peptide and compound residues forming the compound are amino acid residues. The technique disclosed herein is not limited to the above-described example, but is also capable of, in searching for the stable structure of a compound in which multiple compound residues are bonded together, highly accurately searching for the stable structure of the compound in the same way as described above.

Hereinafter, steps in an example of a structure search method disclosed herein will be described in detail.

The structure search method disclosed herein includes, for example, a step of identifying an interaction potential and a step of identifying a steric structure, and further includes other steps as requested.

First, the structure search method disclosed herein may be a computer-based structure search method of searching for a stable structure of a compound in which multiple compound residues are bonded together.

Here, a compound as a stable structure search target is not particularly limited as long as it is a compound in which multiple compound residues are bonded together, but may be selected according to the intended purpose as appropriate.

The compound residues are not particularly limited as long as the compound residues are capable of bonding together, but may be selected according to the intended purpose as appropriate. Examples of the compound residues include amino acid residues and reactive monomers. For example, in the case where the compound residues are amino acid residues, the compound may be a peptide (protein). Instead, in the case where the compound residues are reactive monomers, the compound may be a polymer. Among these, it is preferable that the compound residues be amino acid residues and the compound be a peptide (protein) in the example of the technique disclosed herein.

The compound in which multiple compound residues are bonded together is not limited to a linear compound (single chain), but may be a compound having a branched structure.

An amino acid from which the amino acid residue is to be obtained may be a natural amino acid or an unnatural amino acid (a modified amino acid or an artificial amino acid). Examples of the natural amino acid include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, β-alanine, β-phenylalanine, and the like. The number of amino acid residues in a peptide (protein) is not particularly limited, but may be selected according to the intended purpose as appropriate and be, for example, about 10 to 50 or several hundreds.

An example of the modified amino acid is an amino acid obtained by modifying (substituting) a part of the structure of any of the natural amino acids as listed above. As the modified amino acid, for example, an amino acid obtained by methylating a part of the structure of a natural amino acid or the like may be used. Here, the example of the technique disclosed herein is capable of accurately searching for a stable structure of a compound even when the compound contains a compound group having an unknown interaction potential such as a modified amino acid as described above.

Since it seems to be often the case that the interaction potential is unknown when the compound residues are reactive monomers, it is preferable that each of the monomers that may be contained in the compound be treated as a compound residue having an unknown interaction potential.

Each compound residue is preferably treated as a particle arrangeable at one of lattice points in a three-dimensional lattice space in the step of identifying a steric structure. To this end, it is preferable to treat each compound residue as, for example, a coarse-grained particle of an amino acid residue, a coarse-grained particle of a monomer, or the like.

For example, in the case of treating amino acid residues as coarse-grained particles, each amino acid residue in the peptide may be treated as one coarse-grained particle or each amino acid residue may be divided into a main chain and a side chain in a peptide, and be treated as separate particles (a main chain particle and a side chain particle). In the case where each amino acid residue is divided into a main chain and a side chain in a peptide and the main chain and the side chain are treated as separate particles, the main chain particle of an amino acid having no side chain (such as, for example, glycine) is preferably treated as a side chain particle.

<Step of Identifying Interaction Potential (Interaction Potential Identification Step)>

In the step of identifying an interaction potential (interaction potential identification step), for example, an interaction potential between a compound residue x and a compound residue y among multiple compound residues is identified. In the following description, "identifying an interaction potential" will be referred to as "calculating an interaction potential" and "the step of identifying an interaction potential" will be referred to as "the step of calculating an interaction potential" in some cases.

In the step of identifying an interaction potential, a compound derivative x having the compound residue x and obtained by adding a saturated group-containing structure portion x-1 and a saturated group-containing structure portion x+1 to the compound residue x is prepared as described above.

Here, the compound residue x is adjacent to the compound residue x-1 and the compound residue x+1. Each of the compound residue x-1 and the compound residue x+1 has a functional group bonded to a group involved in a linking bond in the compound residue x.

In each of the compound residue x-1 and the compound residue x+1, the functional group bonded to the group involved in the linking bond in the compound residue x is not particularly limited, but may be selected according to the intended purpose as appropriate. Examples of the functional group bonded to the group involved in the linking bond in the compound residue x in each of the compound residue x-1 and the compound residue x+1 include a hydroxy group, an aldehyde group, a carbonyl group, a carboxy group, a sulfo group, an amino group, and the like.

In the step of identifying an interaction potential, for example, the saturated group-containing structure portion x-1 and the saturated group-containing structure portion x+1 are added to the compound residue x, the saturated group-containing structure portion x-1 and the saturated group-containing structure portion x+1 each composed of one of the aforementioned functional groups and a saturated group obtained by bonding hydrogen atoms to an atom bonded to the concerned functional group to saturate the valence of the atom.

The atom bonded to the functional group is not particularly limited as long as it is an atom capable of bonding to another atom, but may be selected according to the intended purpose as appropriate. Examples thereof include a carbon atom, an oxygen atom, a nitrogen atom, a sulfur atom, a phosphorus atom, and the like.

Examples of the saturated group obtained by saturating the valence of the atom include a methyl group, a hydroxy group, an amino group, and the like.

In the step of identifying an interaction potential, for example, known molecular modeling software may be used to add the saturated group-containing structure portion x-1 and the saturated group-containing structure portion x+1 to the compound residue x (preparation of the compound derivative x).

In the case where the compound is a peptide, the amino group involved in the peptide bond in the amino acid residue x and the carbonyl group involved in the peptide bond in the amino acid residue x may be partially chemically modified. For example, in the example of the technique disclosed herein, the amino group involved in the peptide bond in the amino acid residue x may be, for example, one in which hydrogen in the amino group is substituted with a methyl group (methylated). In the example of the technique disclosed herein, for example, the acetyl structure portion may be added to the amino group-side terminal methylated as described above.

In the example of the technique disclosed herein, for example, the amino acid residue in which the amino group involved in the peptide bond or the carbonyl group involved in the peptide bond is modified as described above is treated as a modified amino acid residue having an unknown interaction potential.

In the step of identifying an interaction potential, for example, parameters for the prepared compound derivative x are set such that none of the saturated group-containing structure portion x-1 and the saturated group-containing structure portion x+1 will cause interaction. When the compound is a peptide, the parameters are set such that none of the acetyl structure portion and the N-methyl structure portion in the amino acid derivative x will cause interaction.

In the step of identifying an interaction potential, for example, a compound derivative y having a compound residue y and obtained by adding a saturated group-containing structure portion y-1 and a saturated group-containing structure portion y+1 to the compound residue y is prepared in the same method as the method of preparing the compound derivative x.

In the step of identifying an interaction potential, for example, parameters for the compound derivative y are set such that none of the saturated group-containing structure portion y-1 and the saturated group-containing structure portion y+1 will cause interaction in the same method as the method of setting the parameters for the compound derivative x.

In the step of identifying an interaction potential, the interaction potential between the compound residue x and the compound residue y is identified (calculated) by using the parameters set for the compound derivative x and the compound derivative y.

The method of calculating the interaction potential between the compound residue x and the compound residue y is not particularly limited as long as the method is capable of calculating the interaction potential by calculating the interaction between the compound residue x and the compound residue y, but may be selected according to the intended purpose as appropriate. Examples of the method of calculating the interaction potential between the compound residue x and the compound residue y include a method using molecular mechanics, a method using molecular dynamics, and so on. As the method of calculating the interaction potential, for example, a Monte Carlo (MC) method may also be used to calculate the interaction potential by specifying multiple pairs of arrangement places of the compound residue x and the compound residue y, and performing molecular mechanics calculation on the specified pairs of arrangement places.

Among these, the method using molecular dynamics is preferable as the method of calculating the interaction potential between the compound residue x and the compound residue y. In the example of the technique disclosed herein, the step of calculating the interaction potential preferably includes calculating the interaction potential between the compound residue x and the compound residue y by molecular dynamics calculation. In this way, in one aspect of the example of the technique disclosed herein, it is possible to more appropriately evaluate the interaction between the compound residue x and the compound residue y, and to calculate the interaction potential with higher accuracy. In the case where the interaction potential is calculated by the molecular dynamics method, for example, the molecular mechanics method may be used to calculate the energy between the compound residue x and the compound residue y.

Hereinafter, detailed description will be given of the parameter setting and the interaction potential identification (calculation) in a case where the interaction potential between amino acid residues is obtained by using molecular dynamics calculation in the step of identifying an interaction potential.

In the case where the interaction potential is calculated by molecular dynamics calculation, the setting of the parameters for each of the amino acid derivatives may be made, for example, by setting parameters for a molecular force field in the amino acid derivative.

The molecular force field is, for example, a mathematical expression formulated as a function of forces that atoms present in a molecule such as a peptide receive from other atoms. In the molecular mechanics calculation or the molecular dynamics calculation based on a molecular force field, a force acting between atoms is represented by a numerical value by using a potential function determined by the types of atoms and a bond state and including, as variables, for example, parameters representing a bond between the atoms (such as a bond length and a bond angle).

In the example of the technique disclosed herein, for example, the parameters for the molecular force field are adjusted and set such that none of the acetyl structure portions and the N-methyl structure portions in the amino acid derivative x and the amino acid derivative y will cause interaction.

The molecular force field is not particularly limited, but may be selected according to the intended purpose as appropriate. For example, a created molecular force field or an existing molecular force field may be used.

In the case of creating and using a molecular force field, for example, known molecular force field creation software may be used to create the molecular force field.

Regarding the existing molecular force field, examples of the molecular force field for a peptide (protein) include assisted model building with energy refinement (Amber)-based molecular force fields, chemistry at Harvard macromolecular mechanics (CHARMm)-based molecular force fields, optimized potentials for liquid simulations (OPLS)-based molecular force fields, and so on. Examples of the Amber-based molecular force field include Amber ff99SB-ILDN, Amber 12SB, and so on. Examples of the CHARMm-based molecular force field include CHARMm 36, and so on.

Regarding the molecular force field, for example, a general AMBER force field (GAFF), which is a versatile molecular force field for an organic compound, may be used as an existing force field for a compound other than the peptide (protein). When the peptide contains a modified amino acid (unnatural amino acid), for example, a molecular force field created by using molecular force field creation software, GAFF, or the like may be used as the molecular force field for the modified amino acid.

In the example of the technique disclosed herein, parameters representing charges of atoms and parameters representing dispersion forces (Van der Waals forces) are adjusted in the setting of the parameters, so that the acetyl structure portion and the N-methyl structure portion will be kept from causing interaction.

In the example of the technique disclosed herein, for example, charges of the acetyl structure portion and the N-methyl structure portion are fixed such that a total value of the charges of the respective structure portions is an integer by using values of existing molecular force fields, and charges of portions other than these structure portions are obtained by quantum chemical calculation.

For example, in the example of the technique disclosed herein, the charge of the acetyl structure portion is fixed to the charge of the "ACE group", which is a residual type corresponding to the acetyl group, and the charge of the N-methyl structure portion is fixed to the charge of the "NME group", which is a residual type corresponding to the N-methyl group. For these residual types, the charges of the respective atoms are usually set such that the total value of the charges of the residual types is an integer.

In the example of the technique disclosed herein, for example, structure optimization by quantum chemical calculation for each amino acid derivative is performed to calculate the electrostatic potential, and the parameters for the charges in the amino acid derivative is obtained based on the calculated electrostatic potential.

It is preferable to use, for example, restrained electrostatic potential (RESP) charges as the charges in the amino acid derivative set based on the electrostatic potential.

As described above, it is preferable to obtain the parameters for the charges in the amino acid derivative such that the total of the charges of the acetyl structure portion and the N-methyl structure portion is an integer.

For example, in order to use an Ewald method or a particle mesh Ewald method (PME method), which is a method of calculating an electrostatic interaction at high speed in molecular dynamics calculation using periodic boundary conditions, it is preferable that the total sum of charges in a calculation system (calculation cells) be 0. In the molecular dynamics calculation, counter ions such as $Na^+$ ions and $Cl^-$ ions are included in the calculation system to adjust the charges in the calculation system. Therefore, the total sum of the charges in the calculation system (calculation cells) may be easily adjusted to 0 when the total sum of the charges of the acetyl structure portion and the N-methyl structure portion is set to an integer. Usually, as the parameters for the charges of the "ACE group" and the "NME group" in the existing molecular force fields, the charges of the respective atoms are set such that the total sum of the charges of these groups will be 0.

In the example of the technique disclosed herein, among the parameters for the amino acid derivative set as described above, for example, the parameters representing the charges and the parameters representing the dispersion forces for the acetyl structure portion and the N-methyl structure portion are set to 0. The setting of the parameters representing the charges and the parameters representing the dispersion forces for the acetyl structure portion and the N-methyl structure portion to 0 may be done, for example, by correcting a file for setting the parameters for the amino acid derivative.

In the example of the technique disclosed herein, the parameters representing the charges and the parameters representing the dispersion forces for the acetyl structure portion and the N-methyl structure portion are set to 0, so that the interactions of these structure portions with other molecules (forces applied to atoms that are not bonded to these structure portions) may be set to 0. For example, in the example of the technique disclosed herein, the parameters representing the charges and the parameters representing the dispersion forces for the acetyl structure portion and the N-methyl structure portion are set to 0, so that it is possible to set the parameters such that the acetyl structure portion and the N-methyl structure portion will not cause interaction.

In the example of the technique disclosed herein, for example, the interaction potential between the amino acid residue x and the amino acid residue y is calculated by performing molecular dynamics calculation using the parameters which are set as described above and which keep the acetyl structure portion and the N-methyl structure portion from causing interaction.

The molecular dynamics calculation means, for example, to simulate the motions of particles (mass points) such as atoms by numerically solving the Newtonian equation of motion.

The molecular dynamics calculation (molecular dynamics simulation) may be performed by using, for example, a known molecular dynamics calculation program. Examples of the molecular dynamics calculation program include AMBER, CHARMm, Groningen machine for chemical simulations (GROMACS), Groningen molecular simulation (GROMOS), nanoscale molecular dynamics (NAMD), myPresto, massively parallel computation of absolute binding free energy with well-equilibrated system (MAPLECAFEE) (registered trademark), and the like.

In the molecular dynamics calculation, for example, after an initial structure of a calculation target molecule is created, the size of a calculation system (calculation cells) is set, and solvent molecules (for example, water molecules) are arranged around the calculation target molecule. As the model of the water molecule, for example, a transferable intermolecular potential with 3 points (TIP3P) model or the like may be used.

In the molecular dynamics calculation, for example, $Na^+$ ions, $Cl^-$ ions, and the like are inserted into a calculation cell such that the total sum of the charges of atoms in the calculation cell will be 0, and forces acting on the respective atoms under periodic boundary conditions are calculated. In the molecular dynamics calculation, for example, how each atom included in the calculation system moves by receiving force is calculated based on the Newtonian equation of motion.

In the molecular dynamics calculation, it is preferable to perform structural relaxation of the solvent molecules and adjustment of the size of the calculation system in order to perform a more stable simulation. The structural relaxation of the solvent molecules may be performed by, for example, molecular dynamics calculation with a fixed number of particles, a fixed volume, and a fixed temperature (constant number, volume, and temperature (NVT) calculation) under the conditions where the size of the calculation system is fixed and positional constraints are applied to the atoms in the main chain of the target molecule. After the structural relaxation of the solvent molecules is performed, the size of the calculation system may be adjusted by, for example, equilibrating the entire calculation system by molecular dynamics calculation with a fixed number of particles, a fixed pressure, and a fixed temperature (constant number, pressure and temperature (NPT) calculation).

In the molecular dynamics calculation, for example, a more stable simulation may be continuously performed by executing the NVT calculation or the NPT calculation for the calculation cells after the size adjustment as described above.

The molecular dynamics calculation is preferably performed at a set temperature of, for example, about 280 K (kelvin) to 320 K.

In the example of the technique disclosed herein, for example, structure sampling for the amino acid residue x and the amino acid residue y is performed by executing molecular dynamics calculation using the parameters that keep the acetyl structure portions and the N-methyl structure portions from causing interaction.

The structure sampling for the amino acid residue x and the amino acid residue y by the molecular dynamics calculation may be performed, for example, by executing the NPT calculation on a calculation system in which the amino acid residue x and the amino acid residue y are arranged at a predetermined distance. In this way, in the example of the technique disclosed herein, for example, it is possible to analyze the magnitude of the interaction between the amino acid residue x and the amino acid residue y or the like by performing the structure sampling for the amino acid residue x and the amino acid residue y by the molecular dynamics calculation.

In the example of the technique disclosed herein, for example, the free energy for the amino acid residue x and the amino acid residue y may be obtained by analyzing structure sampling data (such as trajectory data) for the amino acid residue x and the amino acid residue y. In the example of the technique disclosed herein, for example, a potential of mean force (PMF) for the distance between the amino acid residue x and the amino acid residue y may be obtained based on the structure sampling data for the amino acid residue x and the amino acid residue y.

The PMF represents, for example, a free energy curved surface of a calculation system (a curved surface obtained by plotting free energy along a certain reaction coordinate). Therefore, for example, it is possible to obtain a change in free energy depending on the distance between the amino acid residue x and the amino acid residue y by obtaining the PMF for the distance between the amino acid residue x and the amino acid residue y.

In the example of the technique disclosed herein, the method of obtaining a PMF based on the structure sampling data for the amino acid residue x and the amino acid residue y is not particularly limited, but may be selected according to the intended purpose as appropriate. Examples of the method of obtaining a PMF based on the structure sampling data for the amino acid residue x and the amino acid residue y include an umbrella sampling method, a replica exchange umbrella sampling method, a multi-canonical method, and so on. Among these, it is preferable to use the umbrella sampling method as the method of obtaining a PMF based on the structure sampling data for the amino acid residue x and the amino acid residue y.

In the case of obtaining the PMF by using the umbrella sampling method, for example, molecular dynamics calculation under the condition where the distance between the amino acid derivative x and the amino acid derivative y is constrained (conserved) is performed multiple times by changing the constrained distance. Then, it is possible to obtain the PMF depending on the distance between the amino acid residue x and the amino acid residue y, for example, by coupling the pieces of the sampling data respectively obtained by the multiple times of molecular dynamics calculation. For example, from the PMF depending on the distance between the amino acid residue x and the amino acid residue y, it is possible to obtain a graph (free energy topography) of the PMF in which the distance between the amino acid residue x and the amino acid residue y is set as a reaction coordinate.

For example, a weighted histogram analysis method (WHAM method) or the like may be used as the method of coupling the pieces of the sampling data respectively obtained by the multiple times of molecular dynamics calculation.

In the example of the technique disclosed herein, for example, the interaction potential between the amino acid residue x and the amino acid residue y is calculated based on the PMF obtained as described above. The interaction potential between the amino acid residue x and the amino acid residue y may be calculated, for example, in such a way that the value of the PMF depending on the distance between the amino acid residue x and the amino acid residue y is converted so as to correspond to the distance between the amino acid residues in the three-dimensional lattice space where the amino acid residues are arranged.

In the step of calculating an interaction potential in the structure search method disclosed herein, the PMF depending on the distance between the amino acid residue x and the amino acid residue y is obtained, for example, based on the molecular dynamics calculation for the system including the amino acid residue x and the amino acid residue y as described above. In the step of calculating an interaction potential, for example, the interaction potential between the amino acid residue x and the amino acid residue y usable in a step of creating a steric structure of a peptide is calculated based on the PMF depending on the distance between the amino acid residue x and the amino acid residue y.

In the example of the technique disclosed herein, it is preferable that the above-described calculation of the interaction potential between the amino acid residue x and the amino acid residue y be performed for all combinations of peptide types as steric structure search targets. In one aspect of the example of the technique disclosed herein, it is preferable to calculate the interaction potentials for all combinations of two amino acid residues among multiple amino acid residues. For example, in one aspect of the example of the technique disclosed herein, it is preferable to calculate the interaction potentials for all combinations of two types of compound residues among multiple compound residues.

Thus, in the example of the technique disclosed herein, it is possible to take the highly accurate interaction potentials into consideration without omission in an operation of arranging each of the multiple amino acid residues forming a compound at one of lattice points in a three-dimensional lattice space, and therefore to further improve the accuracy of searching for a stable structure of the compound.

The technique disclosed herein is not limited to the above example. Instead, for example, the above-described commonly known interaction potentials may be used for combinations of the normal natural amino acid residues (20 types of natural amino acids).

<Step of Identifying Steric Structure (Steric Structure Identification Step) >

In the step of identifying a steric structure, for example, the steric structure of the compound is identified in a three-dimensional lattice space which is a set of lattice points by sequentially arranging multiple compound residues at certain lattice points in the three-dimensional lattice space in consideration of the interaction potential obtained in the step of identifying an interaction potential. In the following description, "identifying a steric structure" will be referred to as "creating a steric structure" and "the step of identifying a steric structure" will be referred to as "the step of creating a steric structure" in some cases.

In the step of identifying a steric structure, a method of identifying (creating) a steric structure of a compound in a three-dimensional lattice space is not particularly limited as long as the interaction potentials obtained in the step of identifying an interaction potential may be taken into consideration, but may be selected according to the intended purpose as appropriate. In the step of identifying a steric structure, for example, a method of identifying the steric structure based on an objective function expression based on arrangement conditions and constraints for the compound may be suitably used as the method of identifying (creating) the steric structure of the compound in the three-dimensional lattice space.

<<Objective Function Expression>>

The objective function expression generally means a function based on conditions and constraints in a combinatorial optimization problem, and is a function which takes a minimum value when a combination of variables (parameters) in the objective function expression is optimum in the combinatorial optimization problem. The objective function expression (objective function) may also be referred to as an energy function, a cost function, the Hamiltonian, or the like.

Here, identifying the steric structure of the compound in a three-dimensional lattice space by sequentially arranging multiple compound residues at certain lattice points in the three-dimensional lattice space may be considered as an optimization problem for optimizing the combination of compound residues arranged at the lattice points. Therefore, for example, by searching for a combination of variables which minimize the value of the objective function expression, it is possible to search for a solution of the combinatorial optimization problem, or to search for the stable steric structure of the compound in the three-dimensional lattice space.

The objective function expression is not particularly limited as long as it allows consideration of the interaction potentials obtained in the step of identifying an interaction potential and takes a small value when the compound has a stable steric structure, but may be selected according to the intended purpose as appropriate.

The objective function expression preferably includes, for example, at least the following four terms:

- a term indicating that each of multiple compound residues exists alone;
- a term indicating that two or more of the multiple compound residues do not coexist at one lattice point;
- a term indicating that compound residues bonded together among the multiple compound residues exist at adjacent lattice points in the three-dimensional lattice space; and

- a term representing the interaction potential obtained in the step of identifying an interaction potential.

Here, among the above-listed four terms, the three terms other than the term representing the interaction potential may be considered to be, for example, constraint terms with which the steric structure of the compound to be created will be a structure that may exist consistently as a compound. These three constraint terms may be formulated as, for example, terms each of which takes a small value (for example, the value is 0) when the constraint represented by the term is satisfied. Thus, the example of the technique disclosed herein is capable of searching for a more appropriate steric structure of a compound, because the objective function expression takes a small value when the searched-out steric structure of the compound is a structure that may exist consistently as a compound, for example.

The term representing the interaction potential in the above objective function expression may be considered as a term representing the interaction with which the steric structure of the compound to be identified will be an energetically stable structure. The term representing the interaction potential may be formulated as, for example, a term that takes a smaller value as the interaction is more stable (energy is lower) depending on the distance between the compound residues arranged at the lattice points in the three-dimensional lattice space. Thus, the example of the technique disclosed herein is capable of searching for a more appropriate steric structure of a compound, because the objective function expression takes a smaller value as the searched-out steric structure of the compound is a more energetically stable structure, for example.

For example, in the example of the technique disclosed herein, the steric structure of the compound is identified based on the objective function expression including the above-listed four terms, so that the searched-out steric structure will be a structure that may exist consistently as a compound and an energetically stable structure.

In the example of the technique disclosed herein, it is preferable to use, for example, an objective function expression represented by the following expression (1). In the example of the technique disclosed herein, for example, it is possible to identify the steric structure of the compound by minimizing (optimizing) the following expression (1), and thereby search for a more stable structure of the compound.

$$E = H_{one} + H_{olap} + H_{conn} + H_{pair}$$

... Expression (1)

In Expression (1), E is an objective function expression.

$H_{one}$ is a term indicating that each of multiple compound residues exists alone.

$H_{olap}$ is a term indicating that two or more of the multiple compound residues do not coexist at one lattice point.

$H_{conn}$ is a term indicating that compound residues bonded together among the multiple compound residues exist at adjacent lattice points in a three-dimensional lattice space.

$H_{pair}$ is a term representing the interaction potential obtained in the step of identifying an interaction potential.

In the above expression (1), $H_{one}$, $H_{olap}$, and $H_{conn}$ are, for example, constraint terms with which the steric structure of the compound to be identified will be a structure that may exist consistently as a compound, and may be formulated as terms that take small values (for example, the values are 0) when the constraints represented by the terms are satisfied.

In the above expression (1), $H_{pair}$ is, for example, a term that represents the interaction with which the steric structure of the compound to be identified will be an energetically stable structure, and may be formulated as a term that takes a smaller value as the interaction is more stable (energy is lower).

More specific expressions and the like for $H_{one}$, $H_{olap}$, $H_{conn}$, and $H_{pair}$ in the above expression (1) will be described later.

Here, a method of minimizing the objective function expression is not particularly limited but may be selected according to the intended purpose as appropriate. However, a preferable method is, for example, a method of performing the minimization based on an Ising model expression converted from the objective function expression and represented by the following expression (2). In the example of the technique disclosed herein, for example, it is preferable that the step of identifying a steric structure be performed by optimization processing based on the Ising model expression converted from the objective function expression and represented by the following expression (2). The Ising model expression represented by the following expression (2) is an Ising model expression in a quadratic unconstrained binary optimization (QUBO) format.

$$E = -\sum_{i,j=0} w_{ij} x_i x_j - \sum_{i=0} b_i x_i \quad \text{... Expression (2)}$$

In the above expression (2), E is the Ising model expression converted from the objective function expression, $w_{ij}$ is a numerical value representing an interaction between an i-th bit and a j-th bit, bi is a numerical value representing a bias for the i-th bit, $x_i$ is a binary variable indicating that the i-th bit is 0 or 1, and $x_j$ is a binary variable indicating that the j-th bit is 0 or 1.

Here, $w_{ij}$ in the above expression (2) may be obtained, for example, by extracting numerical values of respective parameters and so on in the objective function expression before conversion into the Ising model expression, for each combination of $x_i$ and $x_j$, and is usually a matrix.

The first term on the right side of the above expression (2) is the sum of the products of the states and the weight value of two circuits, without omission and duplication, in all combinations of two circuits selectable from all the circuits.

The second term on the right side of the above expression (2) is obtained by adding up the products of the bias values and the states of all the circuits.

For example, it is possible to convert the objective function expression into the Ising model expression represented by the above expression (2) by extracting the parameters of the objective function expression before the conversion into the Ising model expression and obtaining $w_{ij}$ and $b_i$.

The Ising model expression converted from the objective function expression as described above may be optimized (minimized) within a short time by, for example, performing an annealing method using an annealing machine or the like. For example, in the example of the technique disclosed herein, it is preferable that the step of identifying a steric structure be performed by executing the ground state search using the annealing method on the Ising model expression, and thereby identifying (calculating) the lowest energy of the Ising model expression.

Examples of the annealing machine used to optimize the objective function expression include, for example, a quantum annealing machine, a semiconductor annealing machine using semiconductor technology, a machine that performs simulated annealing executed by software using a central processing unit (CPU) and a graphics processing unit (GPU), and so on. As the annealing machine, for example, Digital Annealer (registered trademark) may be used.

Details of the annealing method using the annealing machine will be described later.

<Other Steps>

The other steps are not particularly limited but may be selected according to the intended purpose as appropriate.

(Structure Search Apparatus)

A structure search apparatus disclosed herein is a structure search apparatus that searches for a stable structure of a compound in which a plurality of compound residues are bonded together, the structure search apparatus comprising:

an interaction potential identification unit that identifies an interaction potential between a compound residue x and a compound residue y among the plurality of compound residues; and
a steric structure identification unit that identifies a steric structure of the compound in a three-dimensional lattice space which is a set of lattice points by sequentially arranging the plurality of compound residues at certain lattice points in the three-dimensional lattice space in consideration of the interaction potential identified by using the interaction potential identification unit, wherein
the interaction potential identification unit identifies the interaction potential between the compound residue x and the compound residue y by
setting parameters for the compound residue x such that a saturated group-containing structure portion x-1 and a saturated group-containing structure portion x+1 in a compound derivative x containing the compound residue x will not cause interaction,
the saturated group-containing structure portion x-1 included in a compound residue x-1 being adjacent to the compound residue x and having a functional group bonded to a group involved in a linking bond in the compound residue x,
the saturated group-containing structure portion x-1 composed of the functional group and a saturated group obtained by bonding hydrogen atoms to an atom bonded to the functional group to saturate the valence of the atom,
the saturated group-containing structure portion x+1 included in a compound residue x+1 being adjacent to the compound residue x and having a functional group bonded to a group involved in a linking bond in the compound residue x,
the saturated group-containing structure portion x+1 composed of the functional group and a saturated group obtained by bonding hydrogen atoms to an atom bonded to the functional group to saturate the valence of the atom,
the compound derivative x obtained by adding the saturated group-containing structure portion x-1 and the saturated group-containing structure portion x+1 to the compound residue x, and
setting parameters for the compound residue y such that a saturated group-containing structure portion y-1 and a saturated group-containing structure portion y+1 in a compound derivative y containing the compound residue y will not cause interaction,
the saturated group-containing structure portion y-1 included in a compound residue y-1 being adjacent to the compound residue x and having a functional group bonded to a group involved in a linking bond in the compound residue y,
the saturated group-containing structure portion y-1 composed of the functional group and a saturated group obtained by bonding hydrogen atoms to an atom bonded to the functional group to saturate the valence of the atom,
the saturated group-containing structure portion y+1 included in a compound residue y+1 being adjacent to the compound residue y and having a functional group bonded to a group involved in a linking bond in the compound residue y,
the saturated group-containing structure portion y+1 composed of the functional group and a saturated group obtained by bonding hydrogen atoms to an atom bonded to the functional group to saturate the valence of the atom,
the compound derivative y obtained by adding the saturated group-containing structure portion y-1 and the saturated group-containing structure portion y+1 to the compound residue y.

The structure search apparatus disclosed herein may be, for example, an apparatus that executes the structure search method disclosed herein. A preferred embodiment in the structure search apparatus disclosed herein may be the same as a preferred embodiment in the structure search method disclosed herein.

(Structure Search Program)

A structure search program disclosed herein is a program that causes a computer to perform a structure search for searching for a stable structure of a compound in which a plurality of compound residues are bonded together, the program causing the computer to execute a process comprising:

identifying an interaction potential between a compound residue x and a compound residue y among the plurality of compound residues; and
identifying a steric structure of the compound in a three-dimensional lattice space which is a set of lattice points by sequentially arranging the plurality of compound residues at certain lattice points in the three-dimensional lattice space in consideration of the interaction potential obtained in the identifying an interaction potential, wherein
the identifying an interaction potential includes identifying the interaction potential between the compound residue x and the compound residue y by
setting parameters for the compound residue x such that a saturated group-containing structure portion x-1 and a saturated group-containing structure portion x+1 in a compound derivative x containing the compound residue x will not cause interaction,
the saturated group-containing structure portion x-1 included in a compound residue x-1 being adjacent to the compound residue x and having a functional group bonded to a group involved in a linking bond in the compound residue x,
the saturated group-containing structure portion x-1 composed of the functional group and a saturated group obtained by bonding hydrogen atoms to an atom bonded to the functional group to saturate the valence of the atom,
the saturated group-containing structure portion x+1 included in a compound residue x+1 being adjacent to the compound residue x and having a functional group bonded to a group involved in a linking bond in the compound residue x,
the saturated group-containing structure portion x+1 composed of the functional group and a saturated group obtained by bonding hydrogen atoms to an atom bonded to the functional group to saturate the valence of the atom,
the compound derivative x obtained by adding the saturated group-containing structure portion x-1 and the saturated group-containing structure portion x+1 to the compound residue x, and
setting parameters for the compound residue y such that a saturated group-containing structure portion y-1 and a saturated group-containing structure portion y+1 in a compound derivative y containing the compound residue y will not cause interaction,
the saturated group-containing structure portion y-1 included in a compound residue y-1 being adjacent to the compound residue x and having a functional group bonded to a group involved in a linking bond in the compound residue y,

the saturated group-containing structure portion y-1 composed of the functional group and a saturated group obtained by bonding hydrogen atoms to an atom bonded to the functional group to saturate the valence of the atom,

the saturated group-containing structure portion y+1 included in a compound residue y+1 being adjacent to the compound residue y and having a functional group bonded to a group involved in a linking bond in the compound residue y,

the saturated group-containing structure portion y+1 composed of the functional group and a saturated group obtained by bonding hydrogen atoms to an atom bonded to the functional group to saturate the valence of the atom,

the compound derivative y obtained by adding the saturated group-containing structure portion y-1 and the saturated group-containing structure portion y+1 to the compound residue y.

The structure search program disclosed herein may be, for example, a program that causes a computer to execute the structure search method disclosed herein. A preferred embodiment in the structure search program disclosed herein may be the same as a preferred embodiment in the structure search method disclosed herein.

The structure search program disclosed herein may be created using any of various known program languages depending on conditions such as a configuration of a computer system and a type and a version of an operating system for use.

The structure search program disclosed herein may be recorded on a recording medium such as a built-in hard disk, an external hard disk, or the like, or recorded on a recording medium such as a compact disk read-only memory (CD-ROM), a digital versatile disc read-only memory (DVD-ROM), a magneto-optical (MO) disk, or a Universal Serial Bus (USB) memory.

In a case where the structure search program disclosed herein is recorded on the aforementioned recording medium, the structure search program may be used directly or be used by being installed on a hard disk, as requested, by way of a recording medium reader included in the computer system. The structure search program disclosed herein may be recorded in an external storage area (another computer or the like) accessible from the computer system through an information communication network. In this case, the structure search program disclosed herein, which is recorded in the external storage area, may be used directly or be used by being installed on the hard disk from the external storage area, as requested, by way of the information communication network.

The structure search program disclosed herein may be divided into certain process units, which may be recorded on multiple recording media.

(Computer Readable Recording Medium)

A computer readable recording medium disclosed herein is obtained by recording the structure search program disclosed herein.

The computer readable recording medium disclosed herein is not particularly limited, but may be selected according to the intended purpose as appropriate. Examples thereof include a built-in hard disk, an external hard disk, a CD-ROM, a DVD-ROM, an MO disk, a USB memory, and the like.

The computer readable recording medium disclosed herein may be multiple recording media each of which records therein one of certain process units into which the structure search program disclosed herein is divided.

(Interaction Potential identification Method)

An interaction potential identification method disclosed herein is a computer-based interaction potential identification method of identifying an interaction potential between compound residues in a compound in which a plurality of compound residues are bonded together, the method comprising identifying an interaction potential between a compound residue x and a compound residue y among the plurality of compound residues by

setting parameters for the compound residue x such that a saturated group-containing structure portion x-1 and a saturated group-containing structure portion x+1 in a compound derivative x containing the compound residue x will not cause interaction,

the saturated group-containing structure portion x-1 included in a compound residue x-1 being adjacent to the compound residue x and having a functional group bonded to a group involved in a linking bond in the compound residue x,

the saturated group-containing structure portion x-1 composed of the functional group and a saturated group obtained by bonding hydrogen atoms to an atom bonded to the functional group to saturate the valence of the atom,

the saturated group-containing structure portion x+1 included in a compound residue x+1 being adjacent to the compound residue x and having a functional group bonded to a group involved in a linking bond in the compound residue x,

the saturated group-containing structure portion x+1 composed of the functional group and a saturated group obtained by bonding hydrogen atoms to an atom bonded to the functional group to saturate the valence of the atom,
the compound derivative x obtained by adding the saturated group-containing structure portion x-1 and the saturated group-containing structure portion x+1 to the compound residue x, and
setting parameters for the compound residue y such that a saturated group-containing structure portion y-1 and a saturated group-containing structure portion y+1 in a compound derivative y containing the compound residue y will not cause interaction,
the saturated group-containing structure portion y-1 included in a compound residue y-1 being adjacent to the compound residue x and having a functional group bonded to a group involved in a linking bond in the compound residue y,
the saturated group-containing structure portion y-1 composed of the functional group and a saturated group obtained by bonding hydrogen atoms to an atom bonded to the functional group to saturate the valence of the atom,
the saturated group-containing structure portion y+1 included in a compound residue y+1 being adjacent to the compound residue y and having a functional group bonded to a group involved in a linking bond in the compound residue y,
the saturated group-containing structure portion y+1 composed of the functional group and a saturated group obtained by bonding hydrogen atoms to an atom bonded to the functional group to saturate the valence of the atom,
the compound derivative y obtained by adding the saturated group-containing structure portion y-1 and the saturated group-containing structure portion y+1 to the compound residue y.

The interaction potential identification method disclosed herein may be performed, for example, in the same way as the step of identifying an interaction potential in the structure search method disclosed herein. A preferable embodiment in the interaction potential identification method disclosed herein may be the same as, for example, a preferable embodiment in the step of identifying an interaction potential in the structure search method disclosed herein.

Hereinafter, the example of the technique disclosed herein will be described in more detail by using configuration examples of apparatuses, flowcharts, and so on.

FIG. 7 illustrates a hardware configuration example of a structure search apparatus disclosed herein.

In a structure search apparatus 100, for example, a control unit 101, a main storage device 102, an auxiliary storage device 103, an input/output (I/O) interface 104, a communication interface 105, an input device 106, an output device 107, and a display device 108 are coupled to each other via a system bus 109.

The control unit 101 performs operations (such as four arithmetic operations, comparison operations, and annealing method operations), operation control of hardware and software, and the like. The control unit 101 may be, for example, a central processing unit (CPU), a part of an annealing machine for use in the annealing method, or a combination of them.

The control unit 101 implements various functions by, for example, executing a program (such as, for example, the structure search program disclosed herein) read into the main storage device 102 or the like.

The processes performed by the interaction potential identification unit and the steric structure identification unit in the structure search apparatus disclosed herein may be performed by the control unit 101, for example.

The main storage device 102 stores various programs and stores data and others to be used for executing the various programs. As the main storage device 102, for example, a storage device including at least one of a read-only memory (ROM) and a random-access memory (RAM) may be used.

The ROM stores, for example, various programs such as a Basic Input/Output System (BIOS). The ROM is not particularly limited, but may be selected according to the intended purpose as appropriate, and examples thereof include a mask ROM, a programmable ROM (PROM), and the like.

The RAM functions as, for example, a work area in which the various programs stored in the ROM, the auxiliary storage device 103, and the like are expanded when executed by the control unit 101. The RAM is not particularly limited, but may be selected according to the intended purpose as appropriate, and examples thereof include a dynamic random-access memory (DRAM), a static random-access memory (SRAM), and the like.

The auxiliary storage device 103 is not particularly limited as long as it is capable of storing various kinds of information, but may be selected according to the intended purpose as appropriate. Examples thereof include a solid-state drive (SSD), a hard disk drive (HDD), and the like. The auxiliary storage device 103 may be a portable storage device such as a compact disc (CD) drive, a digital versatile disc (DVD) drive, or a Blu-ray (Registered trademark) disc (BD) drive.

The structure search program disclosed herein is stored, for example, in the auxiliary storage device 103, is loaded into the RAM (main memory) of the main storage device 102 and is executed by the control unit 101.

The I/O interface 104 is an interface for coupling to various external devices. The I/O interface 104 allows input and output of data from and to, for example, a compact disc read-only memory (CD-ROM), a digital versatile disk read-only memory (DVD-ROM), a magneto-optical (MO) disk, a Universal Serial Bus (USB) memory [USB flash drive], or the like.

The communication interface 105 is not particularly limited, and any known interface may be used as appropriate. An example thereof is a wireless or wired communication device or the like.

The input device 106 is not particularly limited as long as it is capable of receiving input of various kinds of requests and information to the structure search apparatus 100, and any known device may be used as appropriate. Examples thereof include a keyboard, a mouse, a touch panel, a microphone, and so on. When the input device 106 is a touch panel (touch display), the input device 106 may also serve as the display device 108.

The output device 107 is not particularly limited, and any known device may be used as appropriate. An example thereof is a printer or the like.

The display device 108 is not particularly limited, and any known display device may be used as appropriate. Example thereof include a liquid crystal display, an organic EL display, and the like

FIG. 8 illustrates another hardware configuration example of a structure search apparatus disclosed herein.

In the example illustrated in FIG. 8, the structure search apparatus 100 is divided into a computer 200 that performs a process of identifying an interaction potential, a process of defining an objective function, and other processes, and an annealing machine 300 that performs optimization (ground state search) of the Ising model expression. In the example illustrated in FIG. 8, the computer 200 and the annealing machine 300 in the structure search apparatus 100 are coupled to each other via a network 400.

In the example illustrated in FIG. 8, for example, a CPU or the like may be used as a control unit 101a in the computer 200, and a device specialized for annealing method (annealing) may be used as a control unit 101b in the annealing machine 300.

In the example illustrated in FIG. 8, for example, the computer 200 defines the objective function expression by performing the process of identifying the interaction potential and making various kinds of settings for defining the objective function expression, and converts the defined objective function expression into the Ising model expression. The computer 200 transmits information on the values of the weight ($w_{ij}$) and the bias ($b_i$) in the Ising model expression to the annealing machine 300 via the network 400.

The annealing machine 300 optimizes (minimizes) the Ising model expression based on the received information on the values of the weight ($w_{ij}$) and the bias ($b_i$), and obtains the minimum value of the Ising model expression and the states of the bits that give the minimum value. The annealing machine 300 transmits the obtained minimum value of the Ising model expression and the obtained states of the bits that give the minimum value to the computer 200 via the network 400.

Subsequently, the computer 200 obtains a stable structure of the compound and so on based on the received states of the bits that give the minimum value to the Ising model expression.

FIG. 9 illustrates a functional configuration example of the structure search apparatus disclosed herein.

As illustrated in FIG. 9, the structure search apparatus 100 includes a communication function unit 120, an input function unit 130, an output function unit 140, a display function unit 150, a storage function unit 160, and a control function unit 170.

The communication function unit 120 transmits and receives various kinds of data to and from an external device, for example. For example, the communication function unit 120 may receive structure data of a compound as a stable structure search target, the data of the bias and the weight in the Ising model expression converted from the objective function expression, and the like from the external device.

The input function unit 130 receives various kinds of instructions to the structure search apparatus 100, for example. For example, the input function unit 130 may receive input of the structure data of the compound as the stable structure search target, the data of the bias and the weight in the Ising model expression converted from the objective function expression, and the like.

The output function unit 140 prints and outputs the data of the searched-out stable structure of the compound and so forth, for example.

The display function unit 150 displays the data of the searched-out stable structure of the compound and so forth on the display, for example.

The storage function unit 160 stores various programs, the structure data of the compound as the stable structure search target, a parameter file (topology file) to be used for molecular dynamics calculation, the data of the identified interaction potential, the data of the searched-out stable structure of the compound, and the like, for example.

The control function unit 170 includes an interaction potential identification unit 171 and a steric structure identification unit 175.

The interaction potential identification unit (interaction potential calculation unit) 171 identifies, for example, an interaction potential between a compound residue x and a compound residue y. The interaction potential identification unit 171 includes a compound derivative production unit 172, a parameter setting unit 173, and a molecular dynamics calculation unit 174.

The compound derivative production unit 172 produces and prepares, for example, a compound derivative x containing the compound residue x and a compound derivative y containing the compound residue y. For example, the parameter setting unit 173 sets parameters such that none of the saturated group-containing structure portions x-1, x+1, y-1, and y+1 in the compound derivative x and the compound derivative y will cause interaction. The molecular dynamics calculation unit 174 performs, for example, molecular dynamics calculation on a calculation system including the compound derivative x and the compound derivative y.

The steric structure identification unit (steric structure creation unit) 175 identifies the steric structure of the compound in a three-dimensional lattice space which is a set of lattice points by sequentially arranging the multiple compound residues at certain lattice points in the three-dimensional lattice space in consideration of the interaction potential identified using the interaction potential identification unit, for example. The steric structure identification unit 175 includes an objective function expression creation unit 176 and an optimization processing unit 177.

For example, the objective function expression creation unit 176 creates an objective function expression to be used to create the steric structure of the compound, and converts the created objective function expression into the Ising model expression. For example, the optimization processing unit 177 calculates the lowest energy of the Ising model expression by executing the ground state search using the annealing method on the Ising model expression.

FIG. 10 illustrates an example of a flowchart for identifying an interaction potential to be used to search for a stable structure of a peptide by using the example of the technique disclosed herein.

First, the interaction potential identification unit 171 identifies a sequence of amino acid residues in a peptide as a stable structure search target (S101). For example, in S101, the interaction potential identification unit 171 identifies the types of amino acid residues included in the peptide as the stable structure search target, and the bonding order of the amino acid residues in the peptide.

Next, the interaction potential identification unit 171 selects an amino acid residue x and an amino acid residue y from the amino acid residues included in the peptide (S102). For example, in S102, the interaction potential identification unit 171 selects two amino acid residues from the amino acid residues included in the peptide.

Next, the interaction potential identification unit 171 adds an acetyl structure portion and a N-methyl structure portion to each of the amino acid residue x and the amino acid residue y to prepare an amino acid derivative x and an amino acid derivative y (S103). For example, in S103, the interaction potential identification unit 171 prepares the amino acid derivative x containing the amino acid residue x, which is obtained by adding the acetyl structure portion and the N-methyl structure portion to the amino acid residue x. In S103, the interaction potential identification unit 171 prepares the amino acid derivative y containing the amino acid residue y, which is obtained by adding the acetyl structure portion and the N-methyl structure portion to the amino acid residue y.

Subsequently, the interaction potential identification unit 171 sets parameters of a molecular force field for the amino acid derivative x and the amino acid derivative y such that none of the acetyl structure portions and the N-methyl structure portions will cause interaction (S104). For example, in S104, the charges of the acetyl structure portion and the N-methyl structure portion are fixed by using the values of an existing molecular force field such that the total value of the charges of the structure portions is an integer, and the charges of the portions other than these structure portions are obtained by quantum chemical calculation. In S104, for example, the parameters representing the charges and the parameters representing the dispersion forces for the acetyl structure portion and the N-methyl structure portion are set to 0.

Then, the interaction potential identification unit 171 performs the molecular dynamics calculation using the molecular force field with the set parameters under the conditions where the distance between the amino acid derivative x and the amino acid derivative y is constrained multiple times by changing the constrained distance (S105). For example, in S105, the molecular dynamics calculation in which the distance between the amino acid derivative x and the amino acid derivative y is changed is performed by using the molecular force field set in S104 a predetermined number of times in the range where the distance is changed, thereby performing structure sampling for the amino acid derivative x and the amino acid derivative y.

Next, the interaction potential identification unit 171 obtains a PMF based on data of a distribution of the amino acid derivative x and the amino acid derivative y obtained by performing the structure sampling for the amino acid derivative x and the amino acid derivative y by the molecular dynamics calculation (S106). For example, in S106, the interaction potential identification unit 171 calculates the PMF depending on the distance between the amino acid residue x and the amino acid residue y by, for example, coupling the pieces of the sampling data obtained by the multiple times of the molecular dynamics calculation in S105.

Next, the interaction potential identification unit 171 identifies (calculates) the interaction potential between the amino acid residue x and the amino acid residue y based on the PMF (S107). In S107, for example, the interaction potential is calculated in such a way that the value of the PMF depending on the distance between the amino acid residue x and the amino acid residue y is converted so as to correspond to the distance between the amino acid residues in the three-dimensional lattice space where the amino acid residues are arranged.

Subsequently, the interaction potential identification unit 171 determines whether or not the interaction potentials have been identified for all the combinations of the two types of amino acid residues included in the peptide (S108). For example, when the interaction potential identification unit 171 determines in S108 that the interaction potentials have not been identified for all the combinations of the two types of the amino acid residues, the interaction potential identification unit 171 returns the process to S102. On the other hand, when the interaction potential identification unit 171 determines in S108 that the interaction potentials have been identified for all the combinations of the two types of amino acid residues, the interaction potential identification unit 171 ends the process. When the interaction potential identification unit 171 returns the process to S102 in S108, the interaction potential identification unit 171 preferably selects the amino acid residue x and the amino acid residue y for which the interaction potential has not been identified yet in S102.

FIG. 11 illustrates an example of a flowchart of searching for a stable structure of a peptide in consideration of an interaction potential identified using the example of the technique disclosed herein.

First, the steric structure identification unit 175 defines a three-dimensional lattice space (S201). In S201, for example, the steric structure identification unit 175 defines a three-dimensional lattice space which is a set of lattice points for sequentially arranging multiple amino acid residues based on the number of amino acid residues in a peptide as a stable structure search target.

Hereinafter, an example of the definition of the three-dimensional lattice space will be described. Although the lattice space is actually three-dimensional, the following description will be given of a two-dimensional case as an example for simplification.

First, a set of lattice points within an area having a radius r in a diamond lattice space is referred to as a Shell, and each lattice point is referred to as $S_r$. The lattice points $S_r$ may be represented as illustrated in FIG. 12.

When the lattice points $S_r$ are defined as in FIG. 12, for example, sets of destination lattice points $V_1$ to $V_5$ for first to fifth amino acid residues are as illustrated in FIGs. 13A to 13D.

In FIG. 13A, $V_1 = S_1$ and $V_2 = S_2$. Similarly, $V_3 = S_3$ in FIG. 13B, $V_4 = S_2$ and $S_4$ in FIG. 13C, and $V_5 = S_3$ and $S_5$ in FIG. 13D.

A three-dimensional representation of $S_1$, $S_2$, and $S_3$ is as illustrated in FIG. 14. In FIG. 14, $A = S_1$, $B = S_2$, and $C = S_3$.

A space $V_i$ requested for an i-th amino acid residue in a peptide having n amino acid residues is represented by the following expression.

$$V_i = \bigcup_{r \in J} S_r$$

Here, i = {1, 2, 3, ..., n}.

Then, J = {1, 3, ..., i} for an odd-numbered (i = an odd number) amino acid residue or J = {2, 4, ..., i} for an even-numbered (i = an even number) amino acid residue.

Subsequently, returning to FIG. 11, the steric structure identification unit 175 defines a set of destination lattice points for an i-th amino acid residue as $V_i$ (S202). As a result of defining a set of destination lattice points for an i-th amino acid residue as $V_i$ in S202, a space for arranging each amino acid residue is defined.

Next, the steric structure identification unit 175 allocates bits for use in calculation to the lattice points (S203). In S203, for example, the steric structure identification unit 175 allocates space information to each of bits $X_1$ to $X_n$.

For example, as illustrated in FIGs. 15A to 15C, bits are allocated to the space for arranging the amino acid residues, the bits each having a value "1" indicating that an amino acid residue exists at the concerned lattice point or a value "0" indicating that no amino acid residue exists at the concerned lattice point. Although multiple bits $X_i$ are allocated for each of the amino acid residues 2 to 4 in FIGs. 15A to 15C for convenience of explanation, one bit $X_i$ is allocated for one amino acid residue in practice.

Next, returning to FIG. 11, the steric structure identification unit 175 defines an objective function expression represented by the following expression (1) in consideration of the calculated interaction potential (S204).

$$E = H_{one} + H_{olap} + H_{conn} + H_{pair} \quad \text{... Expression (1)}$$

In Expression (1), E is an objective function expression.

$H_{one}$ is a term indicating that each of multiple compound residues exists alone.

$H_{olap}$ is a term indicating that two or more of the multiple compound residues do not coexist at one lattice point.

$H_{conn}$ is a term indicating that compound residues bonded together among the multiple compound residues exist at adjacent lattice points in a three-dimensional lattice space.

$H_{pair}$ is a term representing the interaction potential obtained in the step of identifying an interaction potential.

Hereinafter, an example of each of the terms in the above expression (1) will be described.

In FIGs. 16 to 19B to be described below, $X_1$ represents a position at which the amino acid residue No. 1 is arrangeable. $X_2$ to $X_5$ represent positions at which the amino acid residue No. 2 is arrangeable. $X_6$ to $X_{13}$ represent

positions at which the amino acid residue No. 3 is arrangeable. $X_{14}$ to $X_{29}$ represent positions at which the amino acid residue No. 4 is arrangeable.

An example of $H_{one}$ is presented below.

$$H_{one} = \lambda_{one} \sum_{i=0}^{N-1} \sum_{x_a, x_b, \in Q_i, a<b} x_a x_b$$

In $H_{one}$, each of $X_a$ and $X_b$ takes 1 or 0. For example, since only one of $X_2$, $X_3$, $X_4$, and $X_5$ is to take 1 in FIG. 16, $H_{one}$ is a function that increases energy if two or more of $X_2$, $X_3$, $X_4$, and $X_5$ are 1, and is a penalty term that takes 0 if only one of $X_2$, $X_3$, $X_4$, and $X_5$ is 1.

In $H_{one}$, $\lambda_{one}$ is a weighting coefficient.

An example of $H_{olap}$ is presented below.

$$H_{olap} = \lambda_{olap} \sum_{v \in V} \sum_{x_a, x_b, \in \theta(v), a<b} x_a x_b$$

In $H_{olap}$, each of $X_a$ and $X_b$ takes 1 or 0. For example, $H_{olap}$ is a term that generates a penalty if $X_{14}$ is 1 when $X_2$ is 1 in FIG. 17.

In $H_{olap}$, $\lambda_{olap}$ is a weighting coefficient.

An example of $H_{conn}$ is presented below.

$$H_{conn} = \lambda_{conn} \left(N - 1 - \sum_{i=0}^{N-1} \sum_{x_d \in Q_i} \sum_{x_u \in \eta(x_d) \cap Q_{i+1}} x_d x_u\right)$$

$H_{conn}$ is a function for evaluating linking bonds between the amino acid residues, and each of $X_d$ and $X_u$ takes 1 or 0. For example, $H_{conn}$ is an expression that decreases energy if any of $X_{13}$, $X_6$, and $X_7$ is 1 when $X_2$ is 1 in FIG. 18, and is a penalty term that takes 0 if all of the amino acid residues in the peptide are bonded together while satisfying the bonding sequence.

In $H_{conn}$, $\lambda_{conn}$ is a weighting coefficient. For example, a relationship $\lambda_{one} > \lambda_{conn}$ may be set.

By transforming the above expression, $H_{conn}$ may be formed into a function that decreases its value to a negative value if the amino acid residues in the peptide are bonded together.

An example of the $H_{pair}$ is presented below.

$$H_{pair} = \frac{1}{2} \sum_{i=0}^{N-1} \sum_{x_a \in Q_i} \sum_{x_b \in \eta(x_a)} P_{\omega(x_a)\omega(x_b)} x_a x_b$$

In $H_{pair}$, each of $X_a$ and $X_b$ takes 1 or 0. For example, $H_{pair}$ is a function that changes energy due to an interaction $P_{\omega(x1)\omega(x15)}$ acting between an amino acid residue $X_1$ and an amino acid residue $X_{15}$ if $X_{15}$ is 1 when $X_1$ is 1 in FIGs. 19A and 19B. The interaction $P_{\omega(x1)w(x15)}$ is determined, for example, by the step of identifying an interaction potential in the technique disclosed herein.

Subsequently, returning to FIG. 11, the steric structure identification unit 175 converts the objective function expression into an Ising model expression in Expression (2) (S205). For example, in S205, the steric structure identification unit 175 extracts the parameters in the objective function expression, and obtains $b_i$ (bias) and $w_{ij}$ (weight) in the following expression (2), thereby converting the objective function expression into the Ising model expression represented by the following expression (2).

$$E = -\sum_{i,j=0} w_{ij} x_i x_j - \sum_{i=0} b_i x_i \quad \text{... Expression (2)}$$

In the above expression (2), E is the Ising model expression converted from the objective function expression, $w_{ij}$ is a numerical value representing an interaction between an i-th bit and a j-th bit, bi is a numerical value representing a bias for the i-th bit, $x_i$ is a binary variable indicating that the i-th bit is 0 or 1, and $x_j$ is a binary variable indicating that the j-th bit is 0 or 1.

Next, the steric structure identification unit 175 minimizes the above expression (2) by using an annealing machine (S206). For example, in S206, the steric structure identification unit 175 executes a ground state search (optimization calculation) using the annealing method on the above expression (2) to calculate the minimum value of the above expression (2), thereby identifying the states of the bits that give the minimum value to the objective function expression.

Subsequently, the steric structure identification unit 175 identifies (creates) a steric structure of the peptide based on the states of the bits that give the minimum value to the above expression (2), and thereby identifies the stable structure of the peptide (S207). For example, in S207, the steric structure identification unit 175 identifies (creates) the steric structure of the peptide by sequentially arranging the amino acid residues in the three-dimensional lattice space based on the states of the bits that give the minimum value to the above expression (2), and thereby identifies the stable structure of the peptide.

The steric structure identification unit 175 outputs the stable structure of the peptide, and ends the process. The stable structure of the peptide may be output as a steric structure diagram of the peptide, or may be output as coordinate information on the amino acid residues forming the peptide.

Although the sequences of the processes in the example of the technique disclosed herein have been described in accordance with specific orders in FIGs. 10 and 11, the orders of steps in the technique disclosed herein may be changed within a technically possible range as appropriate. In the technique disclosed herein, some of the steps may be collectively performed within a technically possible range.

FIGs. 20A to 20C illustrate another example of the method of calculating an interaction potential between amino acid residues.

As an example, considered is a case where a main chain and side chains of amino acid residues as illustrated in FIG. 20A are coarse-grained and are arranged as separate particles at lattice points in FIG. 20B. In FIG. 20A, particles 1 to 4 are particles forming a main chain in respectively different amino acid residues, a particle 5 is a side chain of the amino acid residue having the particle 1 in the main chain, and a particle 6 is a side chain of the amino acid residue having the particle 3 in the main chain.

For the particles illustrated in FIG. 20A, "$q_i^a$" denotes an i-th bit variable (0 or 1) representing a particle a, and "R(i)" and "R(j)" denotes types of amino acid residues represented by the i-th and j-th bit variables, respectively. Then, "$J_{R(i)R(j)}$" denotes an interaction potential between the amino acid residue R(i) and the amino acid residue R(j).

In this case, if the particles are arranged as illustrated in FIG. 20C as a result of performing the structure search, the following eight pairs of particles cause interactions assuming that the interactions occur between all the pairs of particles not bonded together.

"$q_m^5$-$q_n^6$, $q_m^5$-$q_j^2$, $q_m^5$-$q_k^3$, $q_m^5$-$q_l^4$, $q_n^6$-$q_i^1$, $q_n^6$-$q_j^2$, $q_n^6$-$q_k^3$, and $q_n^6$-$q_l^4$"

The interaction ($H_{pair}$) in the above eight pairs may be calculated, for example, in accordance with the following expression.

$$H_{pair} += J_{R(i)R(j)} \times q_i^a \times q_j^b$$

Thus, in the example of the technique disclosed herein, a stable structure may be searched for by creating a steric structure in consideration of, for example, the interaction potential for all the combinations of particles not bonded together.

One example of an annealing method and an annealing machine will be described below.

The annealing method is a method of obtaining a solution stochastically by using a random number value or a superposition of quantum bits. Hereinafter, a problem of minimizing a value of an evaluation function desired to be optimized will be described as an example, and the value of the evaluation function will be referred to as energy. When the value of an evaluation function is desired to be maximized, a sign of the evaluation function may be changed.

First, starting from initial states where one discrete value is assigned to each of variables, a state transition from current states (a combination of the values of the variables) to selected states close to the current states (for example, the states where only one of the variables is changed) is considered. A change in energy associated with the state transition is calculated, and whether to accept the state transition and change the states or to maintain the original states without accepting the state transition is stochastically determined according to the calculated value of the change in energy. When an acceptance probability for a case where the energy decreases is selected to be higher than the acceptance probability for a case where the energy increases, it is expected that a state change occurs in a direction in which the energy decreases on average and the states transition to more appropriate states over time. Thus, there is a possibility of finally obtaining an approximate solution giving energy at an optimal solution or close to an optimal value.

If the state transition is deterministically accepted in a case where the energy decreases, the change in energy will be weakly decreasing over time. However, once a local solution is reached, a state transition will not occur any more. Since an extraordinarily large number of local solutions exist in a discrete optimization problem as described above, the states are often stuck at a local solution that is not very close to the optimal value. For this reason, in solving a discrete optimization problem, it is important to stochastically determine whether or not to accept the states.

In the annealing method, it has been proved that the states reach the optimal solution in the limit of an infinite number of times (number of iterations) by determining that the acceptance probability of the state transition is determined as follows.

Hereinafter, a sequence of a method of determining an optimal solution using the annealing method will be described.

(1) For an energy change (energy decrease) value (-ΔE) associated with a state transition, the acceptance probability p for the state transition is determined by any of the following functions f().

$$p(\Delta E, T) = f(-\Delta E / T)$$ (Expression 1-1)

$$f_{metro}(x) = \min(1, e^x)$$ (Metropolis Method)(Expression 1-2)

$$f_{Gibbs}(x) = \frac{1}{1 + e^{-x}}$$ (Gibbs method)(Expression 1-3)

Here, T is a parameter called a temperature value and may be changed, for example, as follows.

(2) The temperature value T is logarithmically decreased according to the number of iterations t as represented by the following formula.

$$T = \frac{T_0 \log(c)}{\log(t + c)}$$ (Expression 2)

Here, $T_0$ denotes an initial temperature value and is desirably set to a sufficiently large value depending on the problem.

In a case where the acceptance probability expressed by the expression (1) and the steady states are reached after sufficient iterations, the probability of each state being occupied follows the Boltzmann distribution in a thermal equilibrium state in thermodynamics.

When the temperature gradually decreases from a high temperature, the probability of a low energy state being occupied increases. For this reason, when the temperature decreases sufficiently, it is expected to obtain the low energy states. This method is referred to as an annealing method (or simulated annealing method) because this behavior resembles a state change in annealing of a material. The stochastic occurrence of a state transition where the energy increases is equivalent to thermal excitation in physics.

FIG. 21 illustrates an example of a functional configuration of an annealing machine that performs the annealing method. Although the following description will also explain a case where multiple candidates for the state transition are generated, one transition candidate is generated at one time in the basic annealing method.

An annealing machine 300 includes a state holding unit 111 that holds current states S (values of multiple state variables). The annealing machine 300 also includes an energy calculation unit 112 that calculates an energy change value {-ΔEi} for each of state transitions in a case where the state transition occurs from the current states S as a result of changing any of the values of the multiple state variables. The annealing machine 300 includes a temperature control unit 113 that controls a temperature value T and a transition control unit 114 that controls a state change. The annealing machine 300 may be configured as a part of the structure search apparatus 100 described above.

The transition control unit 114 stochastically determines whether or not to accept any one of multiple state transitions, depending on a relative relationship between the energy change value {-ΔEi} and thermal excitation energy based on the temperature value T, the energy change value {-ΔEi}, and a random number value.

The transition control unit 114 includes a candidate generation unit 114a that generates candidates for a state transition, and an acceptability determination unit 114b that stochastically determines whether or not the state transition in each of the candidates is acceptable based on the energy change value {-ΔEi} and the temperature value T. The transition control unit 114 includes a transition determination unit 114c that determines a candidate to be actually employed from the candidates determined as acceptable, and a random number generation unit 114d that generates a probability variable.

An operation in one iteration by the annealing machine 300 is as follows.

First, the candidate generation unit 114a generates one or more candidates (candidate No. {Ni}) for a state transition to the next states from the current states S held by the state holding unit 111. The energy calculation unit 112 calculates an energy change value {-ΔEi} for the state transition specified in each of the candidates by using the current states S and the candidate for the state transition. The acceptability determination unit 114b determines each of the state transitions as acceptable with the acceptance probability expressed by the above expression (1) according to the energy change value {-ΔEi} for the state transition by using the temperature value T generated in the temperature control unit 113 and the probability variable (random number value) generated in the random number generation unit 114d.

The acceptability determination unit 114b outputs the acceptability {fi} of each of the state transitions. In a case where multiple state transitions are determined as acceptable, the transition determination unit 114c randomly selects one of them by using a random number value. The transition determination unit 114c then outputs the transition number N of the selected state transition, and the transition acceptability f. In a case where there is a state transition accepted, the values of the state variables stored in the state holding unit 111 are updated according to the accepted state transition.

Starting with the initial states, the above-described operation is iterated while causing the temperature control unit 113 to decrease the temperature value, and is ended when reaching a certain number of iterations, or when satisfying an end determination condition such as a condition where the energy becomes lower than a predetermined value. The answer output by the annealing machine 300 is the states at the end.

The annealing machine 300 illustrated in FIG. 21 may be implemented by using, for example, a semiconductor integrated circuit. For example, the transition control unit 114 may include a random number generation circuit that functions as the random number generation unit 114d, a comparator circuit that functions as at least a part of the acceptability determination unit 114b, a noise table to be described later, and so on.

Regarding the transition control unit 114 illustrated in FIG. 21, a mechanism to accept a state transition with the acceptance probability expressed by the expression (1) will be described in more detail.

A circuit that outputs 1 with an acceptance probability p and outputs 0 with an acceptance probability (1-p) may be implemented by using a comparator that has two inputs A and B, and that outputs 1 when A > B and outputs 0 when A < B and by inputting the acceptance probability p to the input A and inputting a uniform random number having a value in the unit interval [0, 1) to the input B. Thus, it is possible to achieve the above function when the value of the acceptance probability p calculated by using the expression (1) based on the energy change value and the temperature value T is input to the input A of the comparator.

For example, provided that f denotes a function used in the expression (1), and u denotes a uniform random number having a value in the unit interval [0, 1), a circuit that outputs 1 when f(ΔE/T) is greater than u achieves the above function.

The circuit may achieve the same function as described above even when modified as follows.

Even when the same monotonically increasing function is applied to two numbers, the two numbers maintain the same magnitude relationship. Therefore, even when the same monotonically increasing function is applied to the two inputs of the comparator, the same output is obtained. When an inverse function $f^{-1}$ of f is used as this monotonically increasing function, it is seen that the circuit may be modified to a circuit that outputs 1 when -ΔE/T is greater than $f^{-1}(u)$. Since the temperature value T is positive, it is seen that the circuit may be one that outputs 1 when -ΔE is greater than $Tf^{-1}(u)$.

The transition control unit 114 in FIG. 21 may include a noise table which is a conversion table for realizing the inverse function $f^{-1}(u)$, and which outputs a value of any of the following functions for an input of each discrete value within the unit interval [0, 1).

$$f_{metro}^{-1}(u) = \log(u) \quad \text{(Expression 3-1)}$$

$$f_{Gibbs}^{-1}(u) = \log\left(\frac{u}{1-u}\right) \quad \text{(Expression 3-2)}$$

FIG. 22 illustrates one example of an operation flow of the transition control unit 114. The operation flow illustrated in FIG. 22 includes a step of selecting one state transition as a candidate (S0001), a step of determining whether the state transition is acceptable or not by comparing the energy change value for the state transition with a product of a temperature value and a random number value (S0002), and a step of accepting the state transition when the state transition is acceptable or rejecting the state transition when the state transition is not acceptable (S0003).

[Examples]

Although some examples of the technique disclosed herein will be described, the technique disclosed herein is not limited to these examples at all.

(Example 1)

As Example 1, an interaction potential between leucine residues was identified (calculated) by using an example of the structure search apparatus disclosed herein.

In Example 1, the interaction potential was identified by using a structure search apparatus having a hardware configuration as illustrated in FIG. 8, and by using leucine residues as the amino acid residue x and the amino acid residue y according to the flowchart of FIG. 10.

In Example 1, by using molecular modeling software, an acetyl structure portion composed of a carbonyl group and a methyl group obtained by bonding hydrogen atoms to a carbon atom bonded to the carbonyl group to saturate the valence of the carbon atom was added to the amino group-side terminal of each of the leucine residues. In the same manner, in Example 1, a N-methyl structure portion composed of an amino group and a methyl group obtained by bonding hydrogen atoms to a carbon atom bonded to the amino group to saturate the valence of the carbon atom was added to the carbonyl group-side terminal of each of the leucine residues. In this manner, the leucine derivatives were prepared in Example 1.

In Example 1, the structure optimization of the leucine derivatives was performed by using quantum chemical calculation software Gaussian 09 and using HF/6-31g* as a basis function, and the electrostatic potential of each of the leucine derivatives was calculated. In the structure optimization, the charge of the acetyl structure portion was fixed to the value of the "ACE group", which is a residual type corresponding to the acetyl group in the Amber ff99SB-ILDN in the molecular force field. Similarly, in the structure optimization, the charge of the N-methyl structure portion was fixed to the value of the "NME group", which is a residual type corresponding to the N-methyl group in the Amber ff99SB-ILDN in the molecular force field. Thus, in Example 1, the total value of the charges of the acetyl structure portion and the N-methyl structure portion was fixed to be an integer.

Next, in Example 1, by using the calculated electrostatic potential, atomic charge parameters (RESP charges) in the acetyl structure portion and the N-methyl structure portion in each of the leucine derivatives were obtained by a module antechamber equipped in Amber Tools. In Example 1, molecular force field creation software FF-FOM was used to create parameters (parameters concerning the bond angle and others) other than the charge parameters in the molecular force field for use in molecular dynamics calculation.

In Example 1, by setting the parameters representing the charges and the parameters representing the dispersion forces for the acetyl structure portion and the N-methyl structure portion to 0, the created molecular force field was corrected such that the acetyl structure portion and the N-methyl structure portion would not cause interaction.

Subsequently, in Example 1, the molecular dynamics calculation using the corrected molecular force field was executed for calculating a PMF depending on the distance between the leucine residues. The initial structure in the molecular dynamics calculation was created by randomly arranging the two leucine derivatives and filling the inside of a cubic lattice of $4.5 \times 4.5 \times 4.5$ $nm^3$ with water molecules (TIP3P).

In the molecular dynamics calculation of Example 1, first, energy minimization calculation on the created initial structure with 1000 + 50000 steps was performed by using molecular dynamics simulation software GROMACS with "steep" designated in a setting file.

As the molecular dynamics calculation of Example 1, multiple times of molecular dynamics calculation under the condition where the distance between the leucine derivatives was constrained (conserved) were performed by changing the constrained distance. The distance between the leucine derivatives was constrained in such a way that the distance between the centers of gravity of the Cα carbons or side chains of the leucine derivatives was constrained to be fixed. In the multiple times of molecular dynamics calculation, a harmonic potential having a strength of a spring constant k = 6000 was added in each molecular dynamics calculation (each window) such that the distance between the leucine derivatives was changed in steps of 0.05 nm within a range of 0.4 nm to 1.4 nm. For example, in Example 1, umbrella sampling was performed in the range of 0.4 nm to 1.4 nm of the distance between the leucine derivatives.

In each molecular dynamics calculation (each window), the temperature was set to 298K, NVT calculation of 100 ps was performed for equilibration of the calculation system, and thereafter NPT calculation of 300 ps was performed. Subsequently, in Example 1, sampling for 51 ns (NPT calculation with the distance between the leucine derivatives conserved at a predetermined distance) was performed for each molecular dynamics calculation (each window).

Next, in Example 1, a PMF (free energy) was calculated by using pieces of sampling data at 1st ns to 51st ns in the sampling for 51 ns performed as described above and coupling the pieces of the data in each window by the WHAM method. In consideration of the fact that the degree of freedom varies among the distances r between the leucine derivatives, the calculation of PMF was corrected with addition of $\beta^{-1}\ln(4\pi r^2)$ in order to cancel the stability term due to entropy. In order to maintain the convergence value of the PMF at a long distance at 0, $-\beta^{-1}\ln(4\pi\times1.4^2)$ was added to the calculation for the correction described above.

FIG. 23 illustrates a PMF between leucine residues calculated in Example 1. In FIG. 23, the vertical axis represents the PMF (kcal/mol) and the horizontal axis represents the distance (nm) between the leucine residues. From FIG. 23, it is seen that the PMF is low and the structure is stabilized, for example, when the distance between the leucine residues is 0.5 nm to 0.6 nm.

In Example 1, the calculated PMF was converted so as to correspond to the distance between the amino acid residues in the three-dimensional lattice space, and thereby the interaction potential between the leucine residues was identified.

(Example 2)

In Example 2, an interaction potential between a N-methylphenylalanine residue and a valine residue was identified in the same manner as in Example 1 except that the two leucine residues in Example 1 were changed to the N-methylphenylalanine residue and the valine residue. For example, in Example 2, the interaction potential between the N-methylphenylalanine residue, which is a modified amino acid residue, and the valine residue was identified.

FIG. 24A illustrates an example of a chemical formula of the N-methylphenylalanine residue in a peptide. As illustrated in a circle in FIG. 24A, the N-methylphenylalanine residue is a modified amino acid residue in which an amino group (NH-group) involved in a peptide bond is modified with a methyl group.

FIG. 24B illustrates an example of the structure of a N-methylphenylalanine derivative prepared in Example 2. In Example 2, the N-methylphenylalanine derivative was produced and prepared by adding an acetyl structure portion and a N-methyl structure portion to the N-methylphenylalanine residue.

Similarly, a valine derivative was also produced and prepared by adding an acetyl structure portion and a N-methyl structure portion to the valine residue.

FIG. 25 illustrates a PMF between the N-methylphenylalanine residue and the valine residue calculated in Example 2. In FIG. 25, the vertical axis represents the PMF(kcal/mol), and the horizontal axis represents the distance (nm) between the N-methylphenylalanine residue and the valine residue. From FIG. 25, it is seen that the PMF is low and the structure is stabilized, for example, when the distance between the residues is around 0.5 nm.

In Example 2, the calculated PMF was converted so as to correspond to the distance between the amino acid residues in the three-dimensional lattice space, and thereby the interaction potential between the N-methylphenylalanine residue and the valine residue was identified.

(Example 3)

In Example 3, a stable structure of a cyclic peptide formed by eight amino acid residues and having a steric structure already identified by nuclear magnetic resonance (NMR (nuclear magnetic resonance apparatus)) was searched for by calculating an interaction potential.

Example 3 used a cyclic peptide (Protein Data Bank (PDB) ID: 6AXI) having an amino acid residue sequence "aspartic acid (D) - leucine (L) - phenylalanine (F) - valine (V) - proline (P) - proline (P) - isoleucine (I) - aspartic acid (D)".

In Example 3, the interaction potential was identified for all the combinations of the types of amino acid residues in the cyclic peptide in the same manner as in Examples 1 and 2. In Example 3, each amino acid residue was coarse-grained into the main chain and the side chain as separate particles, and these particles were arranged in the three-dimensional lattice space in a face-centered cubic lattice (FCC).

In Example 3, the stable structure was searched for in consideration of the interaction between not only the closest (adjacent) amino acid residues but also amino acid residues having a distance of 9 Å (1 Å is 0.1 nm) or less therebetween. In Example 3, the distance between lattice points adjacent to each other in the three-dimensional lattice space was set to 3.8 Å.

Example 3 used the objective function expression represented by the above expression (1), and minimized the expression (2) that is the Ising model expression converted from the expression (1) by using a digital annealer (registered trademark) to search for the stable structure.

FIG. 26 illustrates a superposition of the search result of the stable structure of the cyclic peptide searched out in Example 3 on the structure of the cyclic peptide identified by NMR. In FIG. 26, a dark-colored circle having a small diameter indicates the position of the main chain of each amino acid residue in the stable structure obtained in Example 3, and a light-colored circle having a large diameter indicates the position of a Cα carbon atom of each amino acid residue in PDB ID: 6AXI identified by NMR.

The root mean square deviation (RMSD) between the position of the main chain of each amino acid residue in the stable structure obtained in Example 3 and the position of the Cα carbon atom of the corresponding amino acid residue in PDB ID: 6AXI was 0.91 Å. This result means that the stable structure searched out in Example 3 is well matched with the experimental structure identified by NMR.

FIG. 27 illustrates an example of a relationship in searching for a stable structure of a peptide by identifying an interaction potential between amino acid residues in one embodiment of the technique disclosed herein and in the related art.

As illustrated in FIG. 27, for example, an amino acid residue in a peptide forms peptide bonds with amino acid residues adjacent to the concerned amino acid residue. For this reason, the amino acid residue surrounded by a broken line in FIG. 27 is present in the peptide while being peptide-bonded to the adjacent amino acid residues. Therefore, the structure that an amino acid residue may have in the peptide is influenced by, for example, the peptide bonds between the amino acid residue and the adjacent amino acid residues.

In the related art, for example, the interaction potential is identified by performing molecular dynamics calculation on a structure of a side chain portion (side chain analog) extracted from an amino acid and surrounded by a solid line in FIG. 27 or a structure of an amino acid molecule alone surrounded by a broken line in FIG. 27.

For this reason, the related art does not take into consideration the influence of the peptide bonds with the amino acid residues adjacent to the amino acid residue as the calculation target. Therefore, in the related art, since it is not possible to appropriately evaluate the interaction between the main chain in the peptide in the amino acid residue and the side chain of the concerned amino acid residue, the accuracy of the interaction potential is not sufficient, and it is not possible to accurately search for the steric structure of the peptide.

On the other hand, in one embodiment of the technique disclosed herein, for example, an amino acid derivative is prepared by adding an acetyl structure portion and a N-methyl structure portion to an amino acid residue in consideration of an amino acid residue structure in a peptide. In this way, in one embodiment of the technique disclosed herein, it is possible to take into consideration the influence of the peptide bonds with the two amino acid residues bonded adjacent to the amino acid residue. For example, in one embodiment of the technique disclosed herein, it is possible to appropriately evaluate the interaction between the main chain in the peptide in an amino acid residue as the calculation target (for which the interaction potential is to be calculated) and the side chain of the concerned amino acid residue.

In one embodiment of the technique disclosed herein, for example, the parameters are set such that none of the acetyl structure portions and the N-methyl structure portions in the amino acid derivative x and the amino acid derivative y will cause interaction. In this way, in one embodiment of the technique disclosed herein, it is possible to appropriately evaluate the interaction between the amino acid residue x in the amino acid derivative x and the amino acid residue y in the amino acid derivative y.

As described above, in one embodiment of the technique disclosed herein, it is possible to accurately evaluate both the interaction between the main chain in the peptide in the amino acid residue and the side chain of the concerned amino acid residue and the interaction between the amino acid residues, and to accordingly identify (calculate) a highly accurate interaction potential.

In one embodiment of the technique disclosed herein, since a steric structure of a peptide is identified (created) in consideration of a highly accurate interaction potential as described above, it is possible to accurately search for a stable structure of the peptide even when the peptide contains an amino acid residue having an unknown interaction potential.

The following appendices are further disclosed regarding the above embodiments.

[Reference Signs List]

100 structure search apparatus
101 control unit
102 main storage device
103 auxiliary storage device
104 I/O interface
105 communication interface
106 input device
107 output device
108 display device
109 bus
120 communication function unit
130 input function unit
140 output function unit
150 display function unit
160 storage function unit
170 control function unit
171 interaction potential identification unit
172 compound derivative production unit
173 parameter setting unit
174 molecular dynamics calculation unit
175 steric structure identification unit
176 objective function expression creation unit
177 optimization processing unit

[Citation List]

[Non Patent Literature]

NPL 1 : Babbush Ryan, et al., "Construction of Energy Functions for Lattice Heteropolymer Models: A Case Study in Constraint Satisfaction Programming and Adiabatic Quantum Optimization", Advances in Chemical Physics, 155, 201-244.
NPL 2 : Dror Tobi, et.al. "Distance-Dependent, Pair Potential for Protein Folding: Results From Linear Optimization", PROTEINS: Structure, Function, and Genetics, 41: 40-46 (2000).
NPL 3 : Andrew Pohorille, "Good Practices in Free-Energy Calculations", J. Phys. Chem. B, 2010, 114, 10235-10253.

## Claims

**1.** A structure search apparatus of searching for a stable structure of a compound in which a plurality of compound residues are bonded together, the apparatus comprising:

a memory; and
a processor circuit coupled to the memory, the processor circuit being configured to perform processing, the processing including:

executing an interaction potential identification processing configured to identify an interaction potential between a compound residue x and a compound residue y among the plurality of compound residues;
executing a steric structure identification processing configured to identify a steric structure of the compound in a three-dimensional lattice space which is a set of lattice points by arranging, based on the interaction potential identified by the interaction potential identification processing, the plurality of compound residues at lattice points in the three-dimensional lattice space; and
in response to an identification result obtained by the steric structure identification processing, outputting the identification result indicating the identified steric structure of the compound,
wherein the interaction potential identification processing identifies the interaction potential between the compound residue x and the compound residue y by using: first parameters for the compound residue x; and second parameters for the compound residue y,
the first parameters being configured such that a saturated group-containing structure portion x-1 and a saturated group-containing structure portion x+1 in a compound derivative x containing the compound residue x will not cause interaction,
the second parameters being configured such that a saturated group-containing structure portion y-1 and a saturated group-containing structure portion y+1 in a compound derivative y containing the compound residue y will not cause interaction,
the compound derivative x being a compound derivative obtained by adding the saturated group-containing structure portion x-1 and the saturated group-containing structure portion x+1 to the compound residue x,
the compound derivative y being a compound derivative obtained by adding the saturated group-containing structure portion y-1 and the saturated group-containing structure portion y+1 to the compound residue y,
the saturated group-containing structure portion x-1 being a saturated group-containing structure portion included in a compound residue x-1 adjacent to the compound residue x, the compound residue x-1 having a functional group x-1 bonded to a group involved in a linking bond in the compound residue x,
the saturated group-containing structure portion x-1 being composed of the functional group x-1 and a saturated group x-1, the saturated group x-1 being obtained by bonding hydrogen atoms to an atom bonded to the functional group x-1 to saturate a valence of that atom,
the saturated group-containing structure portion x+1 being a saturated group-containing structure portion included in a compound residue x+1 adjacent to the compound residue x, the compound residue x+1 having a functional group x+1 bonded to a group involved in a linking bond in the compound residue x,
the saturated group-containing structure portion x+1 being composed of the functional group x+1 and a saturated group x+1, the saturated group x+1 being obtained by bonding hydrogen atoms to an atom bonded to the functional group x+1 to saturate a valence of that atom,
the saturated group-containing structure portion y-1 being a saturated group-containing structure portion included in a compound residue y-1 adjacent to the compound residue y, the compound residue y-1 having a functional group y-1 bonded to a group involved in a linking bond in the compound residue y,
the saturated group-containing structure portion y-1 being composed of the functional group y-1 and a saturated group y-1, the saturated group y-1 being obtained by bonding hydrogen atoms to an atom bonded to the functional group y-1 to saturate a valence of that atom,
the saturated group-containing structure portion y+1 being a saturated group-containing structure portion included in a compound residue y+1 adjacent to the compound residue y, the compound residue y+1 having a functional group y+1 bonded to a group involved in a linking bond in the compound residue y,
the saturated group-containing structure portion y+1 being composed of the functional group y+1 and a saturated group y+1, the saturated group y+1 being obtained by bonding hydrogen atoms to an atom bonded to the functional group y+1 to saturate a valence of that atom.

**2.** The structure search apparatus according to claim 1, wherein the interaction potential identification processing identifies the interaction potential for all combinations of two types of compound residues among the plurality of compound residues.

**3.** The structure search apparatus according to claim 1 or 2, wherein the interaction potential identification processing identifies the interaction potential between the compound residue x and the compound residue y by molecular dynamics calculation.

**4.** The structure search apparatus according to any one of claims 1 to 3, wherein
the steric structure identification processing identifies the steric structure of the compound by performing a calculation based on an objective function expression, the objective function expression including:

a term indicating that each of the plurality of compound residues exists alone;
a term indicating that two or more of the plurality of compound residues do not coexist at any one of the lattice points;
a term indicating that compound residues bonded together among the plurality of compound residues exist at adjacent lattice points in the three-dimensional lattice space; and
a term representing the interaction potential identified by using the interaction potential identification processing.

**5.** The structure search apparatus according to claim 4, wherein the steric structure identification processing performs an optimization processing based on the objective function expression represented by the following expression (1):

$$E = H_{one} + H_{olap} + H_{conn} + H_{pair} \quad \text{... Expression (1),}$$

where

E is the objective function expression,
$H_{one}$ is a term indicating that each of the plurality of compound residues exists alone,
$H_{olap}$ is a term indicating that two or more of the plurality of compound residues do not coexist at any one of the lattice points,
$H_{conn}$ is a term representing that compound residues bonded together among the plurality of compound residues exist at adjacent lattice points in the three-dimensional lattice space, and
$H_{pair}$ is a term representing the interaction potential identified by using the interaction potential identification processing.

**6.** The structure search apparatus according to claim 5, wherein the steric structure identification processing performs an optimization processing by using an Ising model expression converted from the objective function expression and represented by the following expression (2):

$$E = -\sum_{i,j=0} w_{ij} x_i x_j - \sum_{i=0} b_i x_i \quad \text{... Expression (2),}$$

where

E is the Ising model expression converted from the objective function expression,
$w_{ij}$ is a numerical value representing an interaction between an i-th bit and a j-th bit,
bi is a numerical value representing a bias for the i-th bit,
$x_i$ is a binary variable indicating the i-th bit is 0 or 1, and
$x_j$ is a binary variable indicating the j-th bit is 0 or 1.

**7.** The structure search apparatus according to claim 6, wherein the steric structure identification processing identifies a lowest energy of the Ising model expression by performing a ground state search using an annealing method on the Ising model expression.

**8.** The structure search apparatus according to claim 1, wherein
the compound is a peptide,
the plurality of compound residues is a plurality of amino acid residues,
each of the compound residue x, x-1, x+1, y, y-1, and y+1 is an amino acid residue,
the linking bond is a peptide bond,
the group is an amino group,
the compound derivative is an amino acid derivative,
each of the functional group x-1, x+1, y-1, and y+1 is a carbonyl group, the atom is a carbon atom,
each of the saturated group x-1, x+1, y-1, and y+1 is a methyl group,
the saturated group-containing structure portion x-1 is an acetyl structure portion, and
the saturated group-containing structure portion x+1 is a N-methyl structure portion.

**9.** The structure search apparatus according to claim 8, wherein
the interaction potential identification processing is configured to identify the interaction potential between an amino acid residue x and an amino acid residue y among the plurality of amino acid residues; and
the steric structure identification processing is configured to identify a steric structure of the peptide in the three-dimensional lattice space by arranging, based on the interaction potential identified by the interaction potential identification processing, the plurality of amino acid residues at the lattice points in the three-dimensional lattice space, wherein the interaction potential identification processing identifies the interaction potential between the amino acid residue x and the amino acid residue y by using: the first parameters for the amino acid residue x; and the second parameters for the amino acid residue y,
the first parameters being configured such that an acetyl structure portion x-1 and a N-methyl structure portion x+1 in an amino acid derivative x containing the amino acid residue x will not cause interaction,
the second parameters being configured such that an acetyl structure portion y-1 and a N-methyl structure portion y+1 in an amino acid derivative y containing the amino acid residue y will not cause interaction,
the amino acid derivative x being an amino acid derivative obtained by adding the acetyl structure portion x-1 and the N-methyl structure portion x+1 to the amino acid residue x,
the amino acid derivative y being an amino acid derivative obtained by adding the acetyl structure portion y-1 and the N-methyl structure portion y+1 to the amino acid residue y,
the acetyl structure portion x-1 being an acetyl structure portion included in an amino acid residue x-1 adjacent to the amino acid residue x, the amino acid residue x-1 having a carbonyl group x-1 bonded to an amino group involved in a peptide bond in the amino acid residue x,
the acetyl structure portion x-1 being composed of the carbonyl group x-1 and a methyl group x-1, the methyl group x-1 being obtained by bonding hydrogen atoms to a carbon atom bonded to the carbonyl group x-1 to saturate a valence of that carbon atom,
the N-methyl structure portion x+1 being a N-methyl structure portion included in an amino acid residue x+1 adjacent to the amino acid residue x, the amino acid residue x+1 having a carbonyl group x+1 bonded to an amino group involved in a peptide bond in the amino acid residue x,
the N-methyl structure portion x+1 being composed of the carbonyl group x+1 and a methyl group x+1, the methyl group x+1 being obtained by bonding hydrogen atoms to a carbon atom bonded to the carbonyl group x+1 to saturate a valence of that carbon atom,
the acetyl structure portion y-1 being an acetyl structure portion included in an amino acid residue y-1 adjacent to the amino acid residue y, the amino acid residue y-1 having a carbonyl group y-1 bonded to an amino group involved in a peptide bond in the amino acid residue y,
the acetyl structure portion y-1 being composed of the carbonyl group y-1 and a methyl group y-1, the methyl group y-1 being obtained by bonding hydrogen atoms to a carbon atom bonded to the carbonyl group y-1 to saturate a valence of that carbon atom,
the N-methyl structure portion y+1 being a N-methyl structure portion included in an amino acid residue y+1 adjacent to the amino acid residue y, the amino acid residue y+1 having a carbonyl group y+1 bonded to an amino group involved in a peptide bond in the amino acid residue y,
the N-methyl structure portion y+1 being composed of the carbonyl group y+1 and a methyl group y+1, the methyl group y+1 being obtained by bonding hydrogen atoms to a carbon atom bonded to the carbonyl group y+1 to saturate a valence of that carbon atom.

**10.** A computer-based structure search method of searching for a stable structure of a compound in which a plurality of compound residues are bonded together, the method comprising:

executing an interaction potential identification processing configured to identify an interaction potential between a compound residue x and a compound residue y among the plurality of compound residues;
executing a steric structure identification processing configured to identify a steric structure of the compound in a three-dimensional lattice space which is a set of lattice points by arranging, based on the interaction potential identified by the interaction potential identification processing, the plurality of compound residues at lattice points in the three-dimensional lattice space; and
in response to an identification result obtained by the steric structure identification processing, outputting the identification result indicating the identified steric structure of the compound,
wherein the interaction potential identification processing identifies the interaction potential between the compound residue x and the compound residue y by using: first parameters for the compound residue x; and second parameters for the compound residue y,
the first parameters being configured such that a saturated group-containing structure portion x-1 and a saturated group-containing structure portion x+1 in a compound derivative x containing the compound residue x will not cause interaction,
the second parameters being configured such that a saturated group-containing structure portion y-1 and a saturated group-containing structure portion y+1 in a compound derivative y containing the compound residue y will not cause interaction,
the compound derivative x being a compound derivative obtained by adding the saturated group-containing structure portion x-1 and the saturated group-containing structure portion x+1 to the compound residue x,
the compound derivative y being a compound derivative obtained by adding the saturated group-containing structure portion y-1 and the saturated group-containing structure portion y+1 to the compound residue y,
the saturated group-containing structure portion x-1 being a saturated group-containing structure portion included in a compound residue x-1 adjacent to the compound residue x, the compound residue x-1 having a functional group x-1 bonded to a group involved in a linking bond in the compound residue x,
the saturated group-containing structure portion x-1 being composed of the functional group x-1 and a saturated group x-1, the saturated group x-1 being obtained by bonding hydrogen atoms to an atom bonded to the functional group x-1 to saturate a valence of that atom,
the saturated group-containing structure portion x+1 being a saturated group-containing structure portion included in a compound residue x+1 adjacent to the compound residue x, the compound residue x+1 having a functional group x+1 bonded to a group involved in a linking bond in the compound residue x,
the saturated group-containing structure portion x+1 being composed of the functional group x+1 and a saturated group x+1, the saturated group x+1 being obtained by bonding hydrogen atoms to an atom bonded to the functional group x+1 to saturate a valence of that atom,
the saturated group-containing structure portion y-1 being a saturated group-containing structure portion included in a compound residue y-1 adjacent to the compound residue y, the compound residue y-1 having a functional group y-1 bonded to a group involved in a linking bond in the compound residue y,
the saturated group-containing structure portion y-1 being composed of the functional group y-1 and a saturated group y-1, the saturated group y-1 being obtained by bonding hydrogen atoms to an atom bonded to the functional group y-1 to saturate a valence of that atom,
the saturated group-containing structure portion y+1 being a saturated group-containing structure portion included in a compound residue y+1 adjacent to the compound residue y, the compound residue y+1 having a functional group y+1 bonded to a group involved in a linking bond in the compound residue y,
the saturated group-containing structure portion y+1 being composed of the functional group y+1 and a saturated group y+1, the saturated group y+1 being obtained by bonding hydrogen atoms to an atom bonded to the functional group y+1 to saturate a valence of that atom.

**11.** The computer-based structure search method according to claim 10, wherein the interaction potential identification processing identifies the interaction potential for all combinations of two types of compound residues among the plurality of compound residues.

**12.** The computer-based structure search method according to claim 10 or 11, wherein the interaction potential identification processing identifies the interaction potential between the compound residue x and the compound residue y by molecular dynamics calculation.

**13.** The computer-based structure search method according to any one of claims 10 to 12, wherein
the steric structure identification processing identifies the steric structure of the compound by performing a calculation based on an objective function expression, the objective function expression including:

a term indicating that each of the plurality of compound residues exists alone;
a term indicating that two or more of the plurality of compound residues do not coexist at any one of the lattice points;
a term indicating that compound residues bonded together among the plurality of compound residues exist at adjacent lattice points in the three-dimensional lattice space; and
a term representing the interaction potential identified by using the interaction potential identification unit.

**14.** The computer-based structure search method according to claim 13, wherein the steric structure identification processing performs an optimization processing based on the objective function expression represented by the following expression (1):

$$E = H_{one} + H_{olap} + H_{conn} + H_{pair} \quad \text{... Expression (1),}$$

where

E is the objective function expression,
$H_{one}$ is a term indicating that each of the plurality of compound residues exists alone,
$H_{olap}$ is a term indicating that two or more of the plurality of compound residues do not coexist at any one of the lattice points,
$H_{conn}$ is a term representing that compound residues bonded together among the plurality of compound residues exist at adjacent lattice points in the three-dimensional lattice space, and
$H_{pair}$ is a term representing the interaction potential identified by using the interaction potential identification processing.

**15.** The computer-based structure search method according to claim 10, wherein
the compound is a peptide,
the plurality of compound residues is a plurality of amino acid residues,
each of the compound residue x, x-1, x+1, y, y-1, and y+1 is an amino acid residue,
the linking bond is a peptide bond,
the group is an amino group,
the compound derivative is an amino acid derivative,
each of the functional group x-1, x+1, y-1, and y+1 is a carbonyl group,
the atom is a carbon atom,
each of the saturated group x-1, x+1, y-1, and y+1 is a methyl group,
the saturated group-containing structure portion x-1 is an acetyl structure portion, and
the saturated group-containing structure portion x+1 is a N-methyl structure portion.

**16.** The computer-based structure search method according to claim 15, wherein
the interaction potential identification processing is configured to identify the interaction potential between an amino acid residue x and an amino acid residue y among the plurality of amino acid residues; and
the steric structure identification processing is configured to identify a steric structure of the peptide in the three-dimensional lattice space by arranging, based on the interaction potential identified by the interaction potential identification processing, the plurality of amino acid residues at the lattice points in the three-dimensional lattice space,
wherein the interaction potential identification processing identifies the interaction potential between the amino acid residue x and the amino acid residue y by using: the first parameters for the amino acid residue x; and the second parameters for the amino acid residue y,
the first parameters being configured such that an acetyl structure portion x-1 and a N-methyl structure portion x+1 in an amino acid derivative x containing the amino acid residue x will not cause interaction,
the second parameters being configured such that an acetyl structure portion y-1 and a N-methyl structure portion y+1 in an amino acid derivative y containing the amino acid residue y will not cause interaction,
the amino acid derivative x being an amino acid derivative obtained by adding the acetyl structure portion x-1 and the N-methyl structure portion x+1 to the amino acid residue x,
the amino acid derivative y being an amino acid derivative obtained by adding the acetyl structure portion y-1 and the N-methyl structure portion y+1 to the amino acid residue y,
the acetyl structure portion x-1 being an acetyl structure portion included in an amino acid residue x-1 adjacent to the amino acid residue x, the amino acid residue x-1 having a carbonyl group x-1 bonded to an amino group involved in a peptide bond in the amino acid residue x,
the acetyl structure portion x-1 being composed of the carbonyl group x-1 and a methyl group x-1, the methyl group x-1 being obtained by bonding hydrogen atoms to a carbon atom bonded to the carbonyl group x-1 to saturate a valence of that carbon atom,
the N-methyl structure portion x+1 being a N-methyl structure portion included in an amino acid residue x+1 adjacent to the amino acid residue x, the amino acid residue x+1 having a carbonyl group x+1 bonded to an amino group involved in a peptide bond in the amino acid residue x,
the N-methyl structure portion x+1 being composed of the carbonyl group x+1 and a methyl group x+1, the methyl group x+1 being obtained by bonding hydrogen atoms to a carbon atom bonded to the carbonyl group x+1 to saturate a valence of that carbon atom,
the acetyl structure portion y-1 being an acetyl structure portion included in an amino acid residue y-1 adjacent to the amino acid residue y, the amino acid residue y-1 having a carbonyl group y-1 bonded to an amino group involved in a peptide bond in the amino acid residue y,
the acetyl structure portion y-1 being composed of the carbonyl group y-1 and a methyl group y-1, the methyl group y-1 being obtained by bonding hydrogen atoms to a carbon atom bonded to the carbonyl group y-1 to saturate a valence of that carbon atom,
the N-methyl structure portion y+1 being a N-methyl structure portion included in an amino acid residue y+1 adjacent to the amino acid residue y, the amino acid residue y+1 having a carbonyl group y+1 bonded to an amino group involved in a peptide bond in the amino acid residue y,
the N-methyl structure portion y+1 being composed of the carbonyl group y+1 and a methyl group y+1, the methyl group y+1 being obtained by bonding hydrogen atoms to a carbon atom bonded to the carbonyl group y+1 to saturate a valence of that carbon atom.

**17.** A structure search program comprising instructions which, when the program is executed by a computer, cause the computer to perform processing of searching for a stable structure of a compound in which a plurality of compound residues are bonded together, the processing comprising:

executing an interaction potential identification processing configured to identify an interaction potential between a compound residue x and a compound residue y among the plurality of compound residues;
executing a steric structure identification processing configured to identify a steric structure of the compound in a three-dimensional lattice space which is a set of lattice points by arranging, based on the interaction potential identified by the interaction potential identification processing, the plurality of compound residues at lattice points in the three-dimensional lattice space; and
in response to an identification result obtained by the steric structure identification processing, outputting the identification result indicating the identified steric structure of the compound,
wherein the interaction potential identification processing identifies the interaction potential between the compound residue x and the compound residue y by using: first parameters for the compound residue x; and second parameters for the compound residue y,
the first parameters being configured such that a saturated group-containing structure portion x-1 and a saturated group-containing structure portion x+1 in a compound derivative x containing the compound residue x will not cause interaction,
the second parameters being configured such that a saturated group-containing structure portion y-1 and a saturated group-containing structure portion y+1 in a compound derivative y containing the compound residue y will not cause interaction,
the compound derivative x being a compound derivative obtained by adding the saturated group-containing structure portion x-1 and the saturated group-containing structure portion x+1 to the compound residue x,
the compound derivative y being a compound derivative obtained by adding the saturated group-containing structure portion y-1 and the saturated group-containing structure portion y+1 to the compound residue y,
the saturated group-containing structure portion x-1 being a saturated group-containing structure portion included in a compound residue x-1 adjacent to the compound residue x, the compound residue x-1 having a functional group x-1 bonded to a group involved in a linking bond in the compound residue x,
the saturated group-containing structure portion x-1 being composed of the functional group x-1 and a saturated group x-1, the saturated group x-1 being obtained by bonding hydrogen atoms to an atom bonded to the functional group x-1 to saturate a valence of that atom,
the saturated group-containing structure portion x+1 being a saturated group-containing structure portion included in a compound residue x+1 adjacent to the compound residue x, the compound residue x+1 having a functional group x+1 bonded to a group involved in a linking bond in the compound residue x,
the saturated group-containing structure portion x+1 being composed of the functional group x+1 and a saturated group x+1, the saturated group x+1 being obtained by bonding hydrogen atoms to an atom bonded to the functional group x+1 to saturate a valence of that atom,
the saturated group-containing structure portion y-1 being a saturated group-containing structure portion included in a compound residue y-1 adjacent to the compound residue y, the compound residue y-1 having a functional group y-1 bonded to a group involved in a linking bond in the compound residue y,
the saturated group-containing structure portion y-1 being composed of the functional group y-1 and a saturated group y-1, the saturated group y-1 being obtained by bonding hydrogen atoms to an atom bonded to the functional group y-1 to saturate a valence of that atom,
the saturated group-containing structure portion y+1 being a saturated group-containing structure portion included in a compound residue y+1 adjacent to the compound residue y, the compound residue y+1 having a functional group y+1 bonded to a group involved in a linking bond in the compound residue y,
the saturated group-containing structure portion y+1 being composed of the functional group y+1 and a saturated group y+1, the saturated group y+1 being obtained by bonding hydrogen atoms to an atom bonded to the functional group y+1 to saturate a valence of that atom.

**18.** The structure search program according to claim 17, wherein the interaction potential identification processing identifies the interaction potential for all combinations of two types of compound residues among the plurality of compound residues.

**19.** The structure search program according to claim 17 or 18, wherein the interaction potential identification processing identifies the interaction potential between the compound residue x and the compound residue y by molecular dynamics calculation.

**20.** The structure search program according to any one of claims 17 to 19, wherein
the steric structure identification processing identifies the steric structure of the compound by performing a calculation based on an objective function expression, the objective function expression including:

a term indicating that each of the plurality of compound residues exists alone;
a term indicating that two or more of the plurality of compound residues do not coexist at any one of the lattice points;
a term indicating that compound residues bonded together among the plurality of compound residues exist at adjacent lattice points in the three-dimensional lattice space; and
a term representing the interaction potential identified by using the interaction potential identification processing.

FIG. 1A

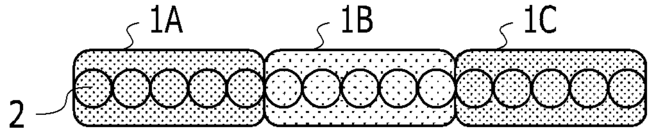


FIG. 1B

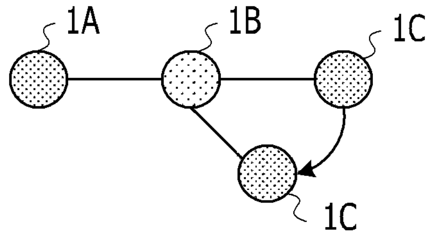


FIG. 1C

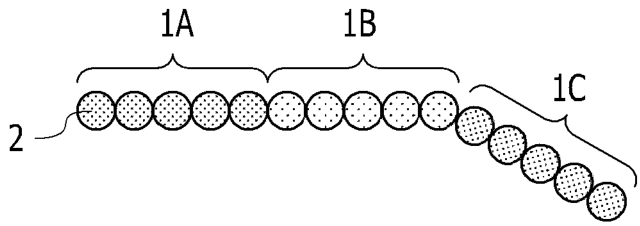

FIG. 2A

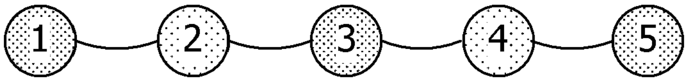


FIG. 2B

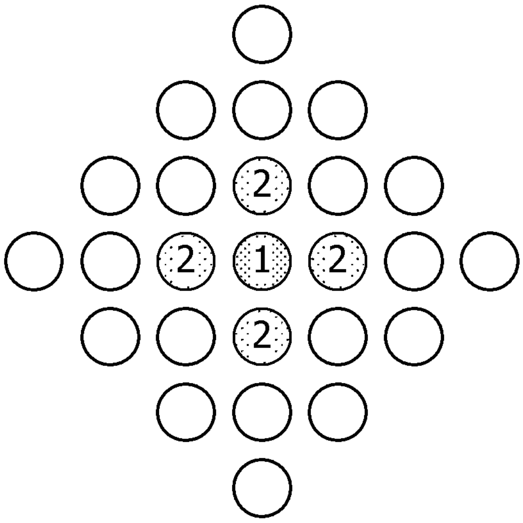


FIG. 2C

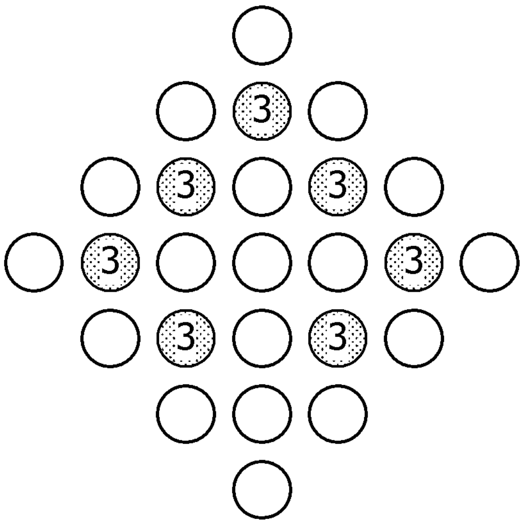

FIG. 2D

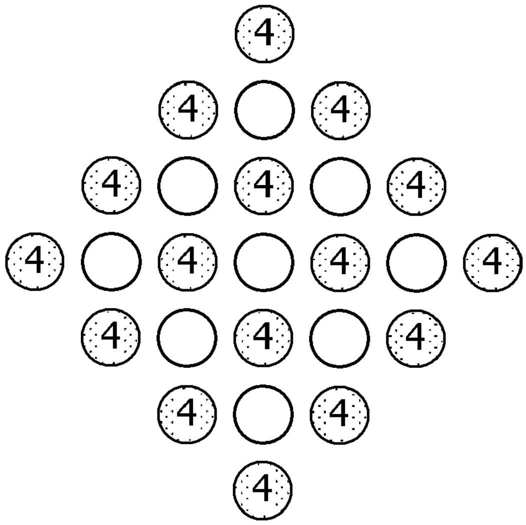

FIG. 2E

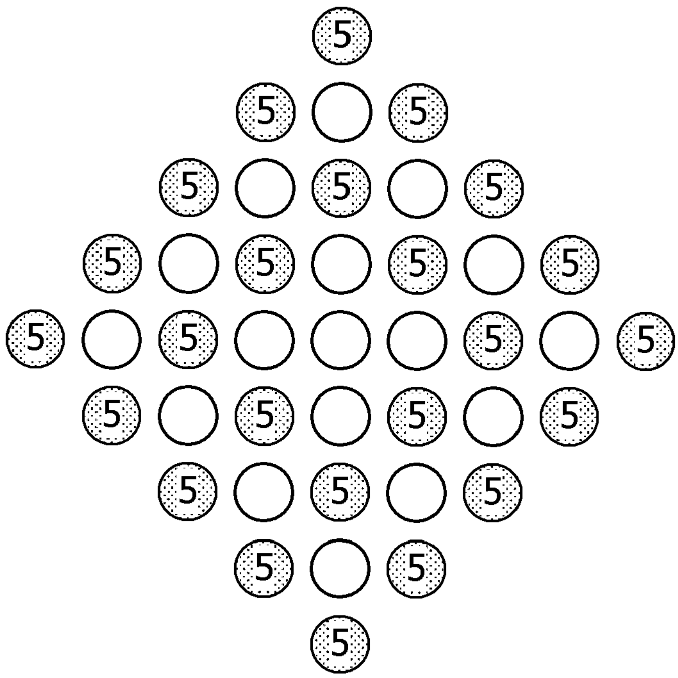

FIG. 3

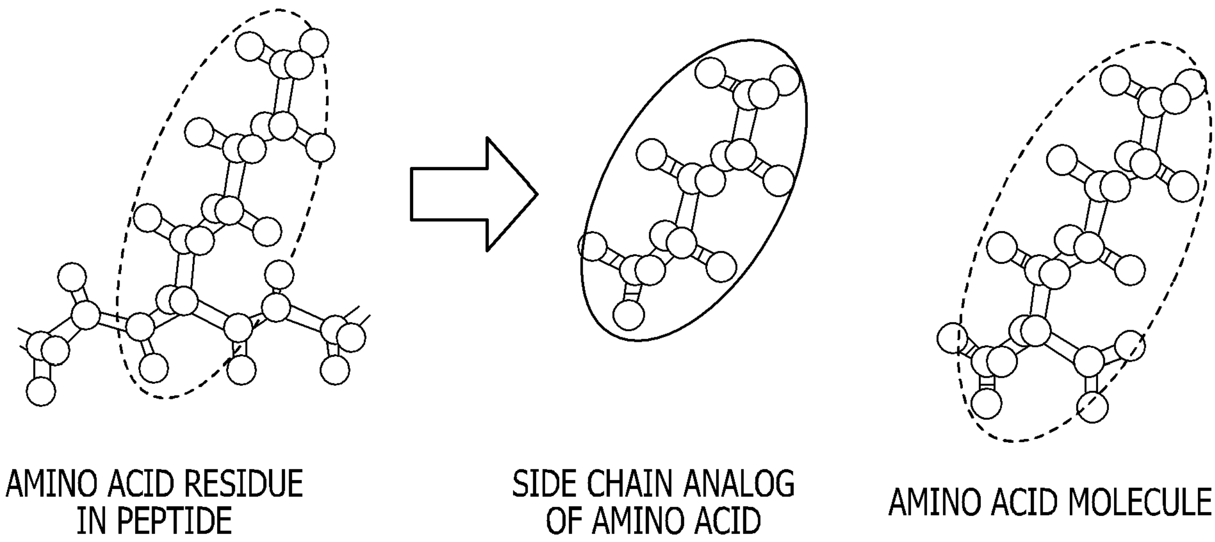
AMINO ACID RESIDUE
IN PEPTIDE
SIDE CHAIN ANALOG
OF AMINO ACID
AMINO ACID MOLECULE

# FIG. 4

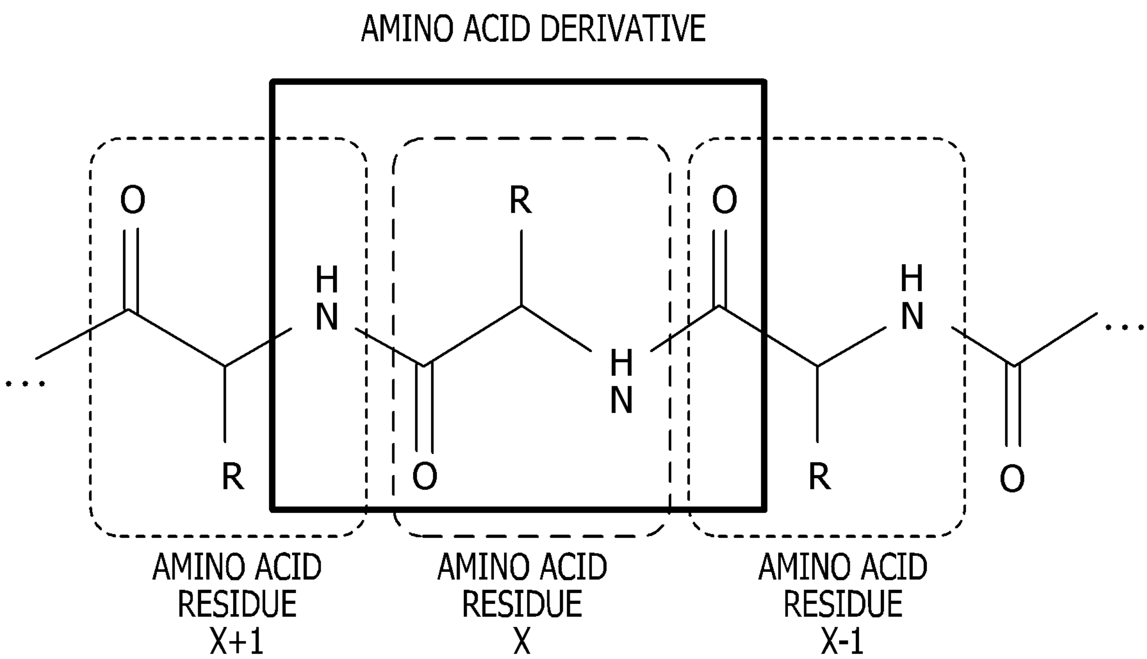
AMINO ACID DERIVATIVE
AMINO ACID RESIDUE X+1
AMINO ACID RESIDUE X
AMINO ACID RESIDUE X-1

FIG. 5

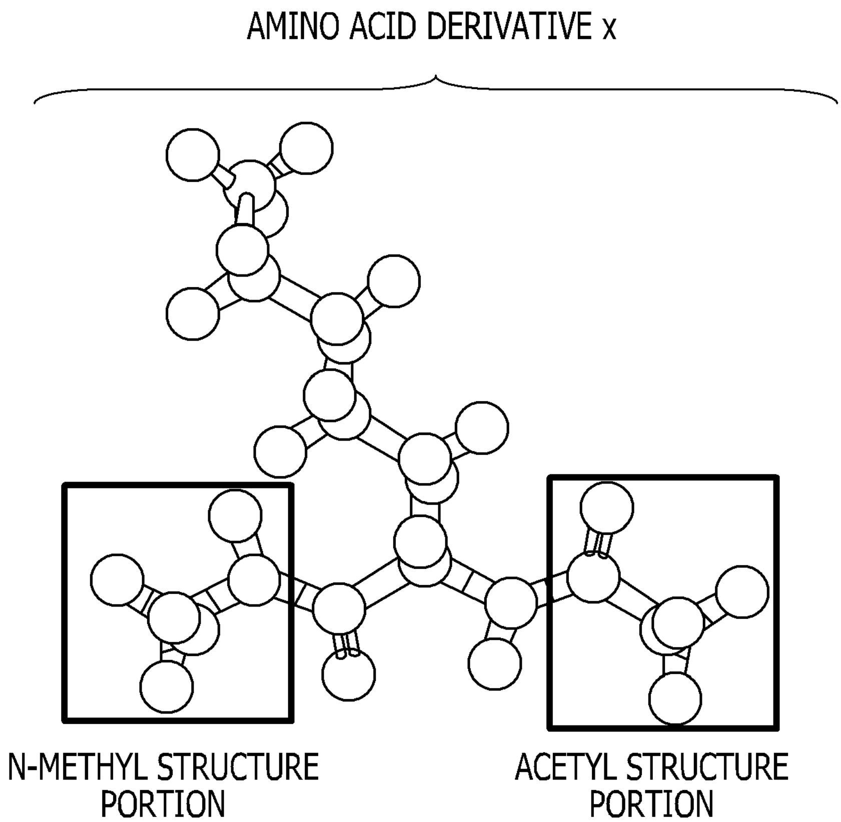
AMINO ACID DERIVATIVE x
N-METHYL STRUCTURE PORTION
ACETYL STRUCTURE PORTION

FIG. 6

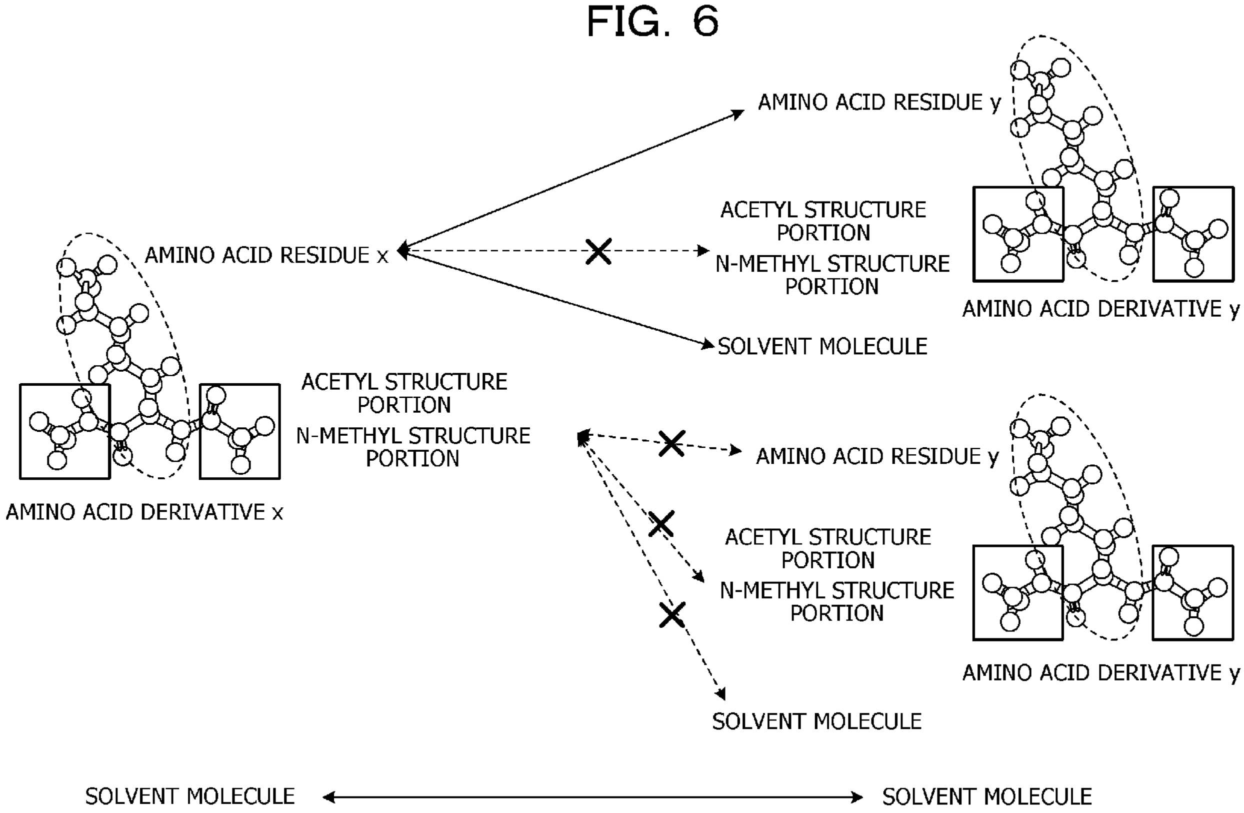
AMINO ACID RESIDUE y
ACETYL STRUCTURE PORTION
N-METHYL STRUCTURE PORTION
AMINO ACID RESIDUE x
SOLVENT MOLECULE
AMINO ACID DERIVATIVE y
ACETYL STRUCTURE PORTION
N-METHYL STRUCTURE PORTION
AMINO ACID DERIVATIVE x
AMINO ACID RESIDUE y
ACETYL STRUCTURE PORTION
N-METHYL STRUCTURE PORTION
AMINO ACID DERIVATIVE y
SOLVENT MOLECULE
SOLVENT MOLECULE
SOLVENT MOLECULE

FIG. 7

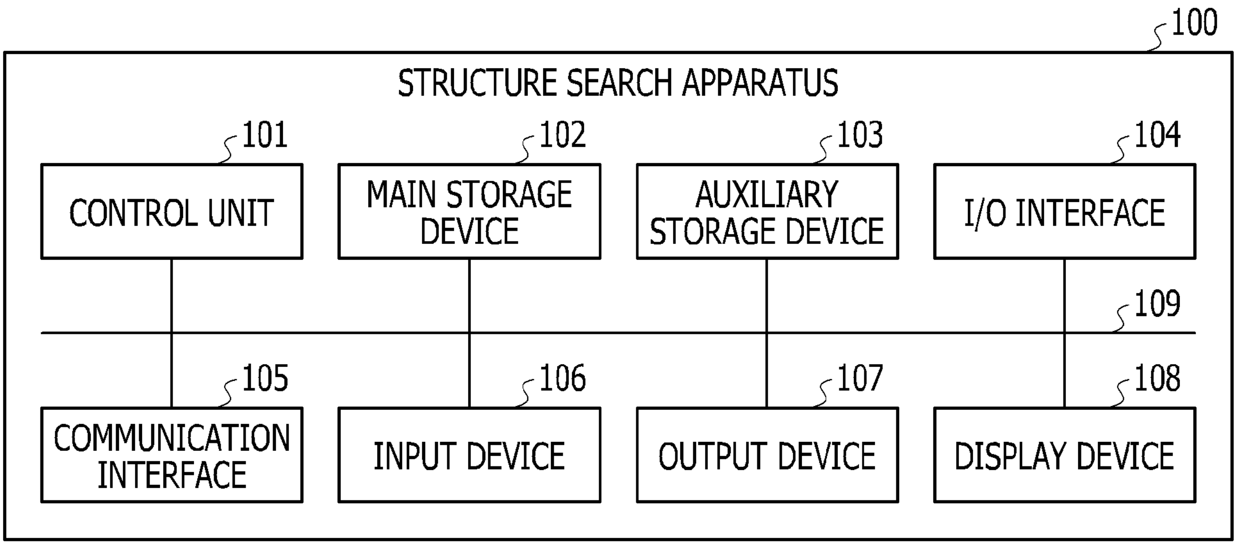
100
STRUCTURE SEARCH APPARATUS
101
CONTROL UNIT
102
MAIN STORAGE DEVICE
103
AUXILIARY STORAGE DEVICE
104
I/O INTERFACE
109
105
COMMUNICATION INTERFACE
106
INPUT DEVICE
107
OUTPUT DEVICE
108
DISPLAY DEVICE

FIG. 8

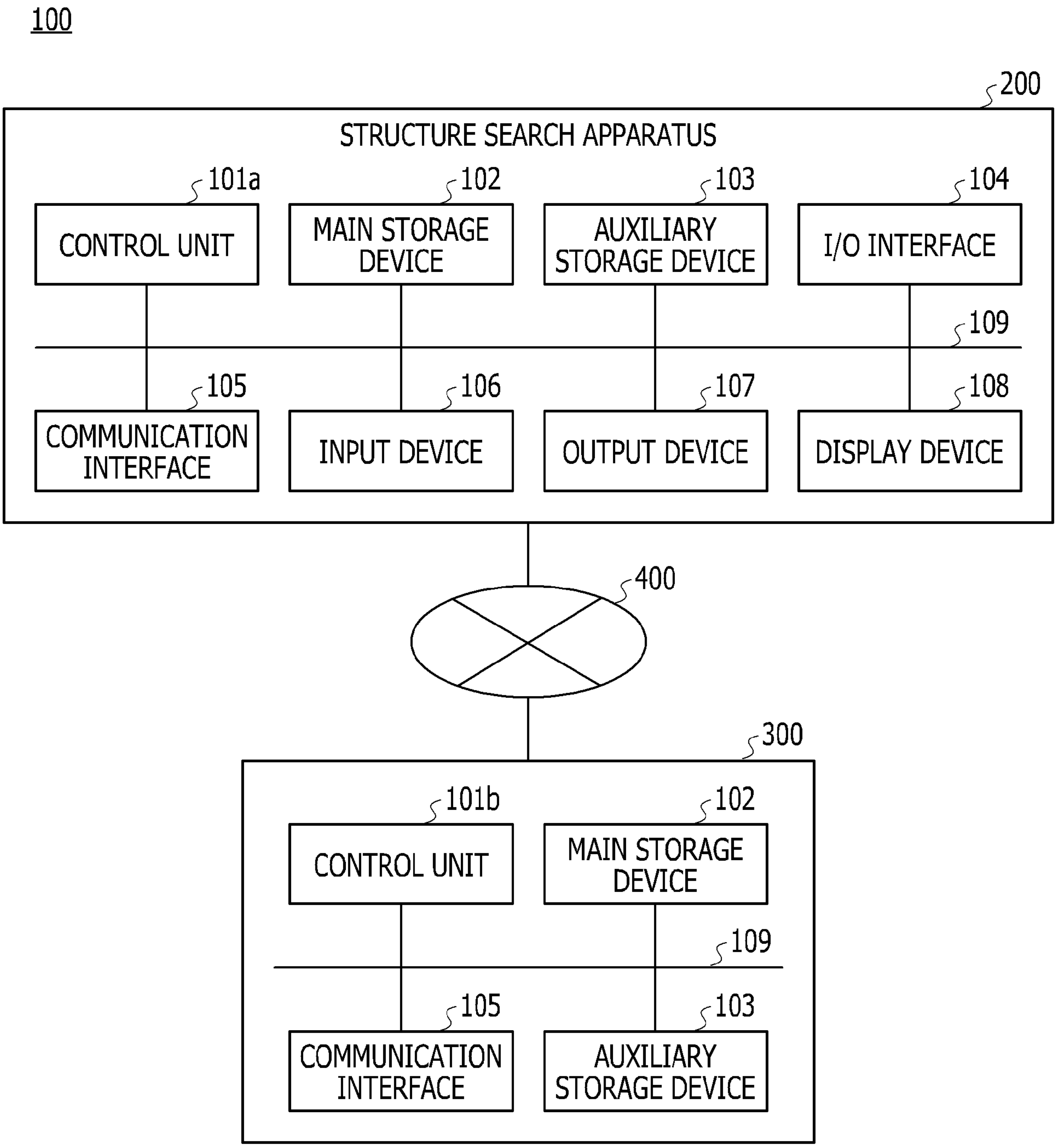
100
200
STRUCTURE SEARCH APPARATUS
101a
CONTROL UNIT
102
MAIN STORAGE DEVICE
103
AUXILIARY STORAGE DEVICE
104
I/O INTERFACE
109
105
COMMUNICATION INTERFACE
106
INPUT DEVICE
107
OUTPUT DEVICE
108
DISPLAY DEVICE
400
300
101b
CONTROL UNIT
102
MAIN STORAGE DEVICE
109
105
COMMUNICATION INTERFACE
103
AUXILIARY STORAGE DEVICE

FIG. 9

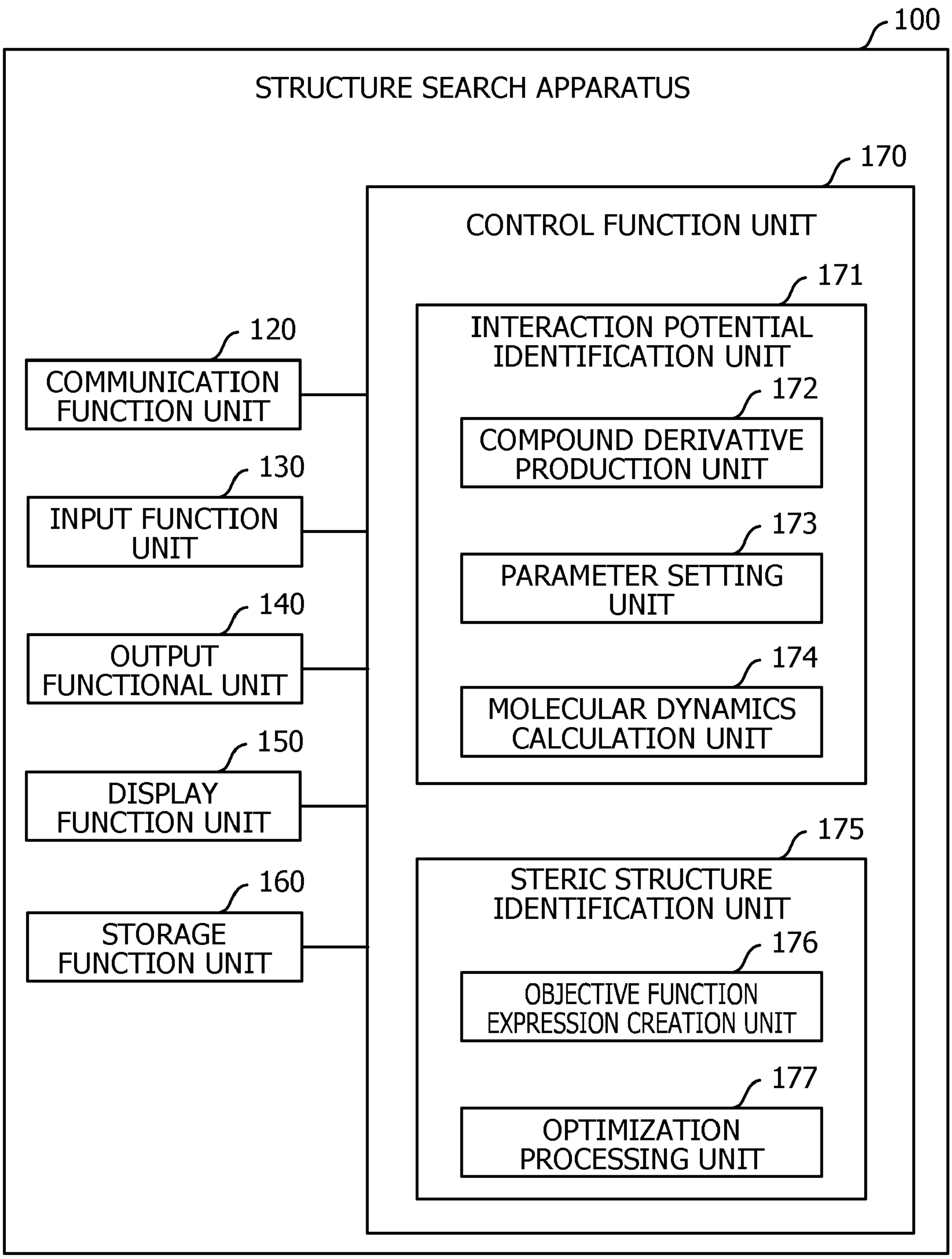
100
STRUCTURE SEARCH APPARATUS
170
CONTROL FUNCTION UNIT
171
INTERACTION POTENTIAL IDENTIFICATION UNIT
172
COMPOUND DERIVATIVE PRODUCTION UNIT
173
PARAMETER SETTING UNIT
174
MOLECULAR DYNAMICS CALCULATION UNIT
175
STERIC STRUCTURE IDENTIFICATION UNIT
176
OBJECTIVE FUNCTION EXPRESSION CREATION UNIT
177
OPTIMIZATION PROCESSING UNIT
120
COMMUNICATION FUNCTION UNIT
130
INPUT FUNCTION UNIT
140
OUTPUT FUNCTIONAL UNIT
150
DISPLAY FUNCTION UNIT
160
STORAGE FUNCTION UNIT

FIG. 10

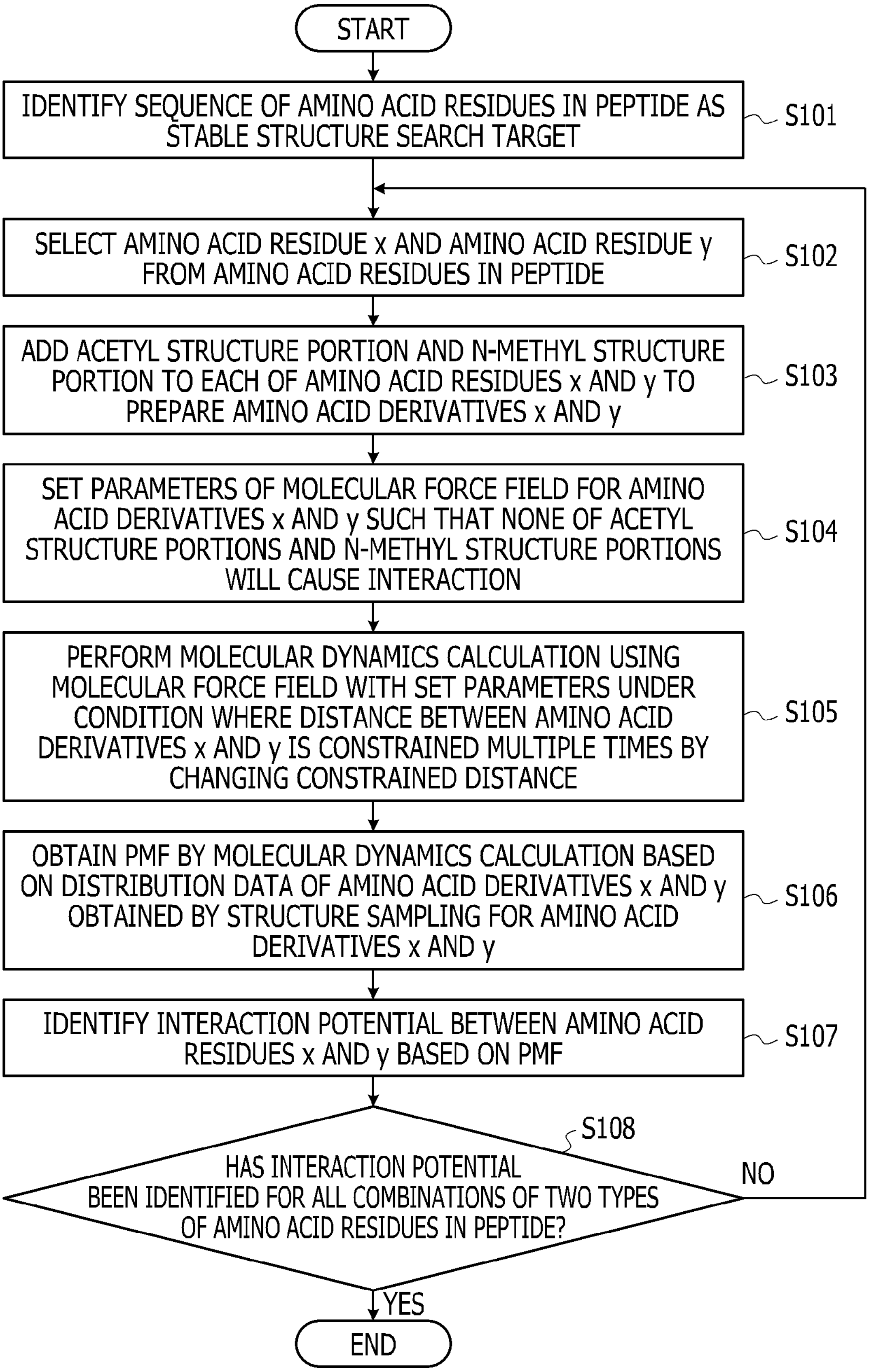
START
IDENTIFY SEQUENCE OF AMINO ACID RESIDUES IN PEPTIDE AS STABLE STRUCTURE SEARCH TARGET
S101
SELECT AMINO ACID RESIDUE x AND AMINO ACID RESIDUE y FROM AMINO ACID RESIDUES IN PEPTIDE
S102
ADD ACETYL STRUCTURE PORTION AND N-METHYL STRUCTURE PORTION TO EACH OF AMINO ACID RESIDUES x AND y TO PREPARE AMINO ACID DERIVATIVES x AND y
S103
SET PARAMETERS OF MOLECULAR FORCE FIELD FOR AMINO ACID DERIVATIVES x AND y SUCH THAT NONE OF ACETYL STRUCTURE PORTIONS AND N-METHYL STRUCTURE PORTIONS WILL CAUSE INTERACTION
S104
PERFORM MOLECULAR DYNAMICS CALCULATION USING MOLECULAR FORCE FIELD WITH SET PARAMETERS UNDER CONDITION WHERE DISTANCE BETWEEN AMINO ACID DERIVATIVES x AND y IS CONSTRAINED MULTIPLE TIMES BY CHANGING CONSTRAINED DISTANCE
S105
OBTAIN PMF BY MOLECULAR DYNAMICS CALCULATION BASED ON DISTRIBUTION DATA OF AMINO ACID DERIVATIVES x AND y OBTAINED BY STRUCTURE SAMPLING FOR AMINO ACID DERIVATIVES x AND y
S106
IDENTIFY INTERACTION POTENTIAL BETWEEN AMINO ACID RESIDUES x AND y BASED ON PMF
S107
S108
HAS INTERACTION POTENTIAL BEEN IDENTIFIED FOR ALL COMBINATIONS OF TWO TYPES OF AMINO ACID RESIDUES IN PEPTIDE?
NO
YES
END

FIG. 11

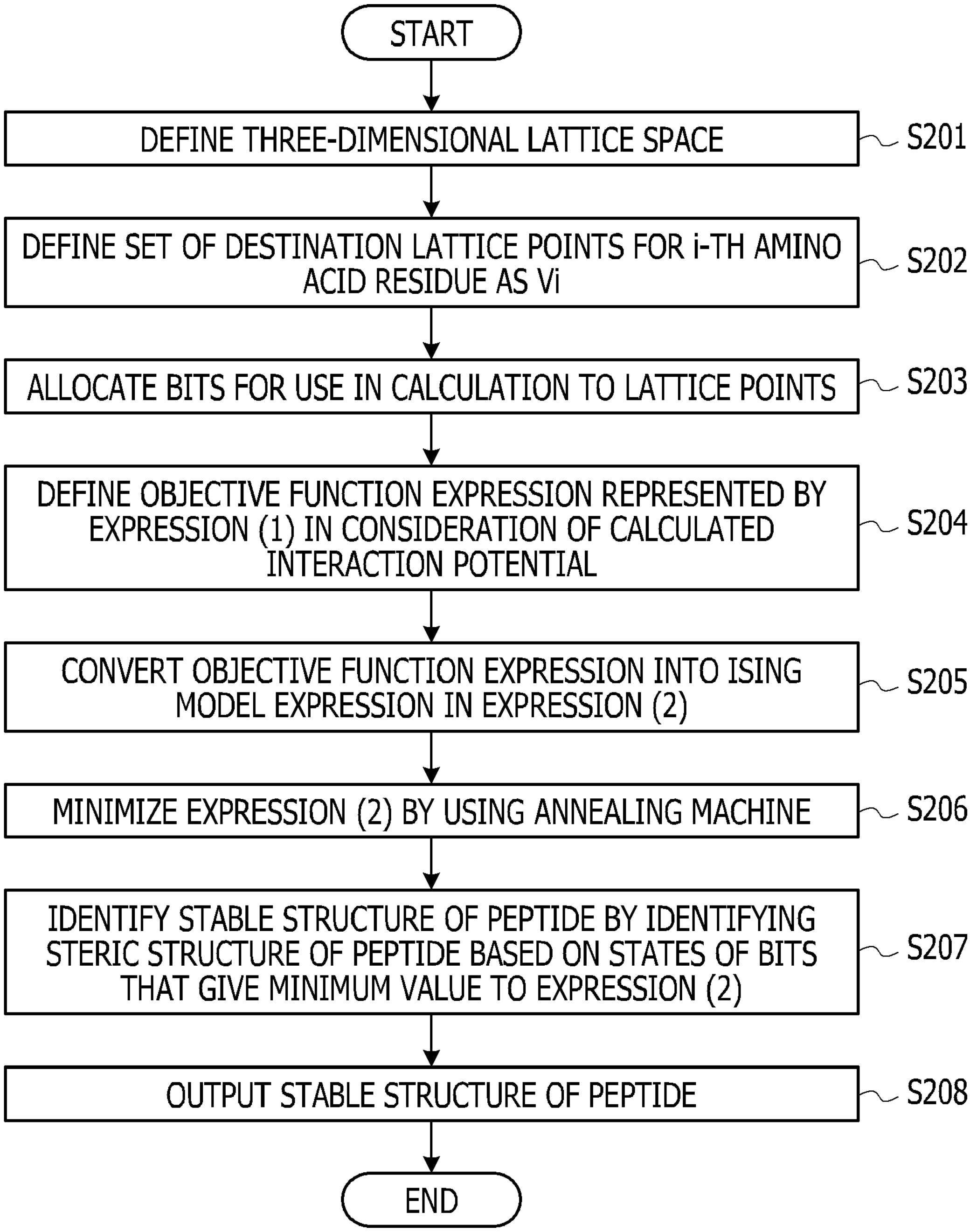
START
DEFINE THREE-DIMENSIONAL LATTICE SPACE
S201
DEFINE SET OF DESTINATION LATTICE POINTS FOR i-TH AMINO ACID RESIDUE AS Vi
S202
ALLOCATE BITS FOR USE IN CALCULATION TO LATTICE POINTS
S203
DEFINE OBJECTIVE FUNCTION EXPRESSION REPRESENTED BY EXPRESSION (1) IN CONSIDERATION OF CALCULATED INTERACTION POTENTIAL
S204
CONVERT OBJECTIVE FUNCTION EXPRESSION INTO ISING MODEL EXPRESSION IN EXPRESSION (2)
S205
MINIMIZE EXPRESSION (2) BY USING ANNEALING MACHINE
S206
IDENTIFY STABLE STRUCTURE OF PEPTIDE BY IDENTIFYING STERIC STRUCTURE OF PEPTIDE BASED ON STATES OF BITS THAT GIVE MINIMUM VALUE TO EXPRESSION (2)
S207
OUTPUT STABLE STRUCTURE OF PEPTIDE
S208
END

FIG. 12

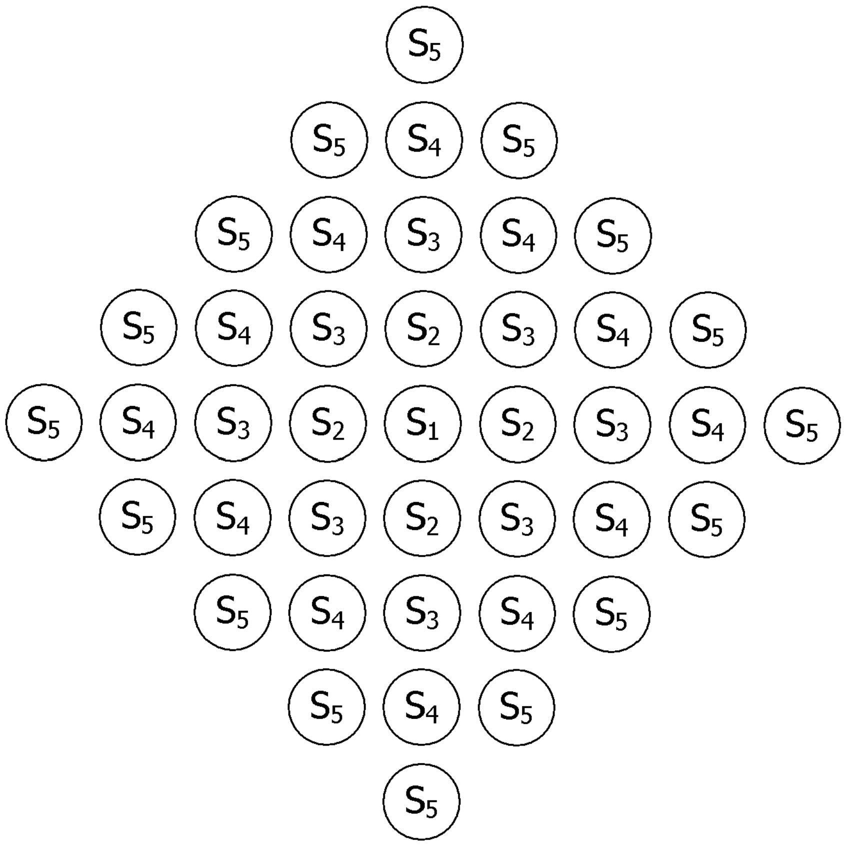
S5
S5 S4 S5
S5 S4 S3 S4 S5
S5 S4 S3 S2 S3 S4 S5
S5 S4 S3 S2 S1 S2 S3 S4 S5
S5 S4 S3 S2 S3 S4 S5
S5 S4 S3 S4 S5
S5 S4 S5
S5

FIG. 13A

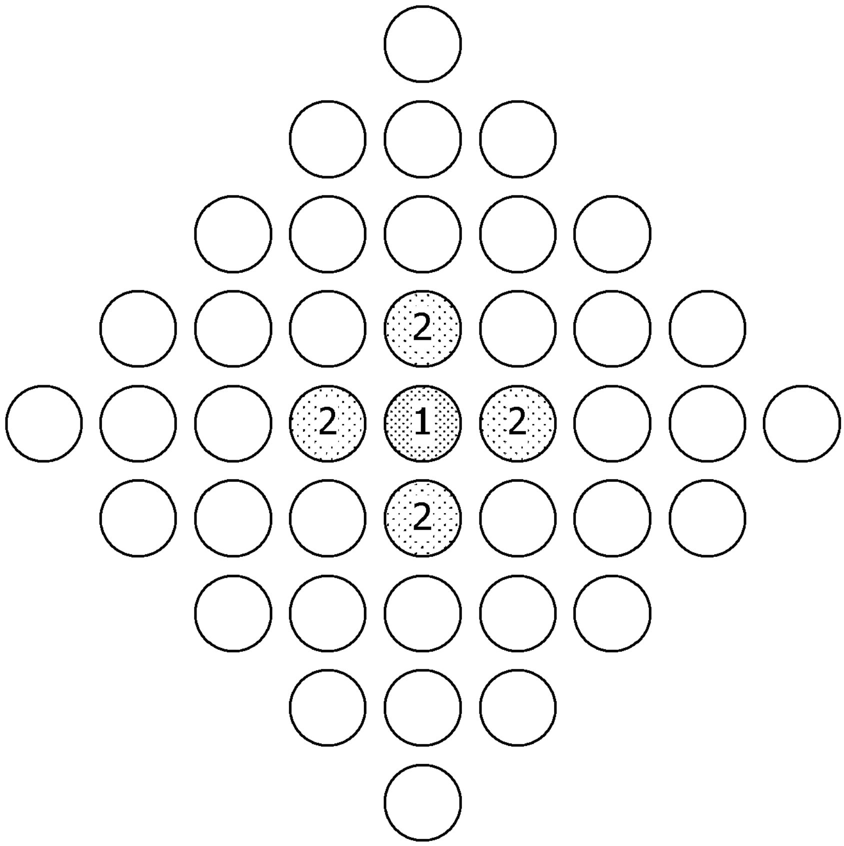

FIG. 13B

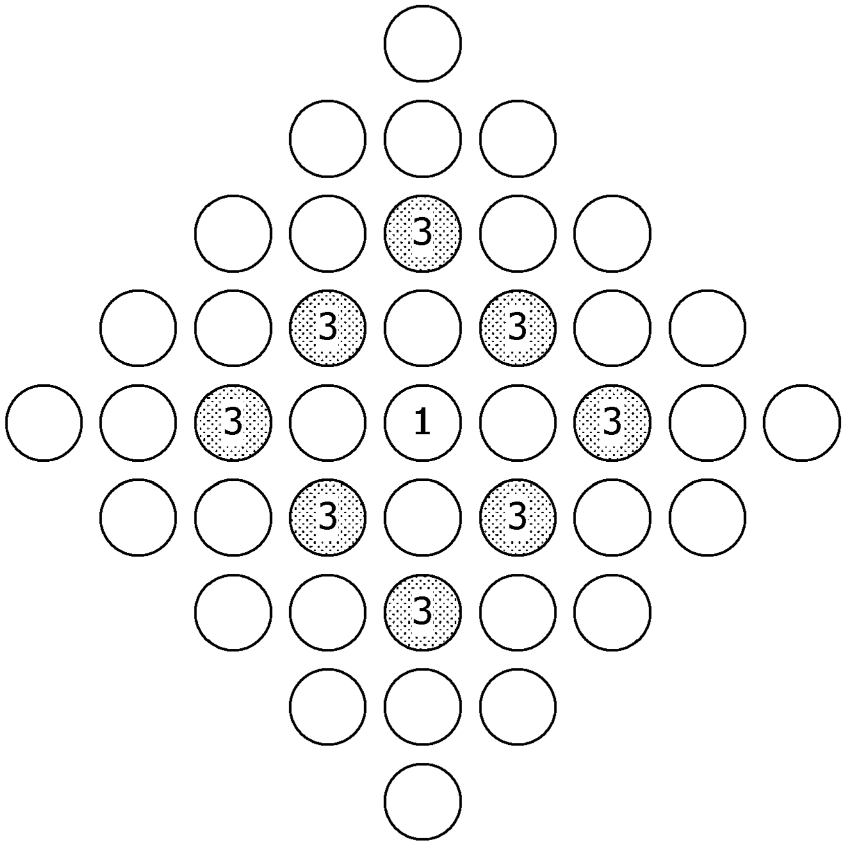

FIG. 13C

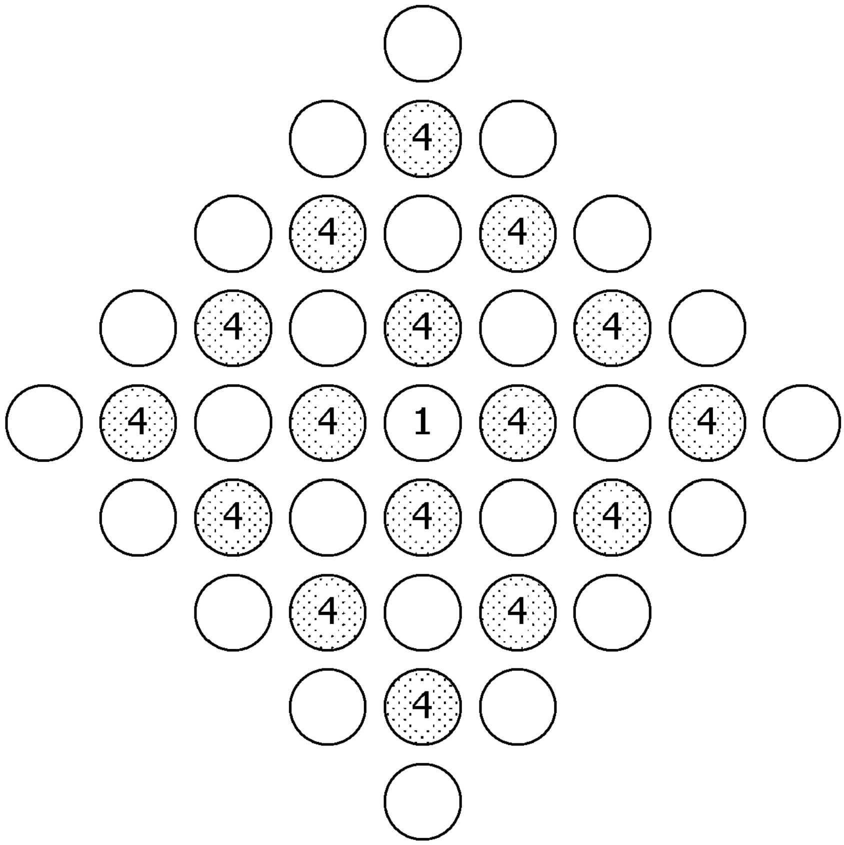

FIG. 13D

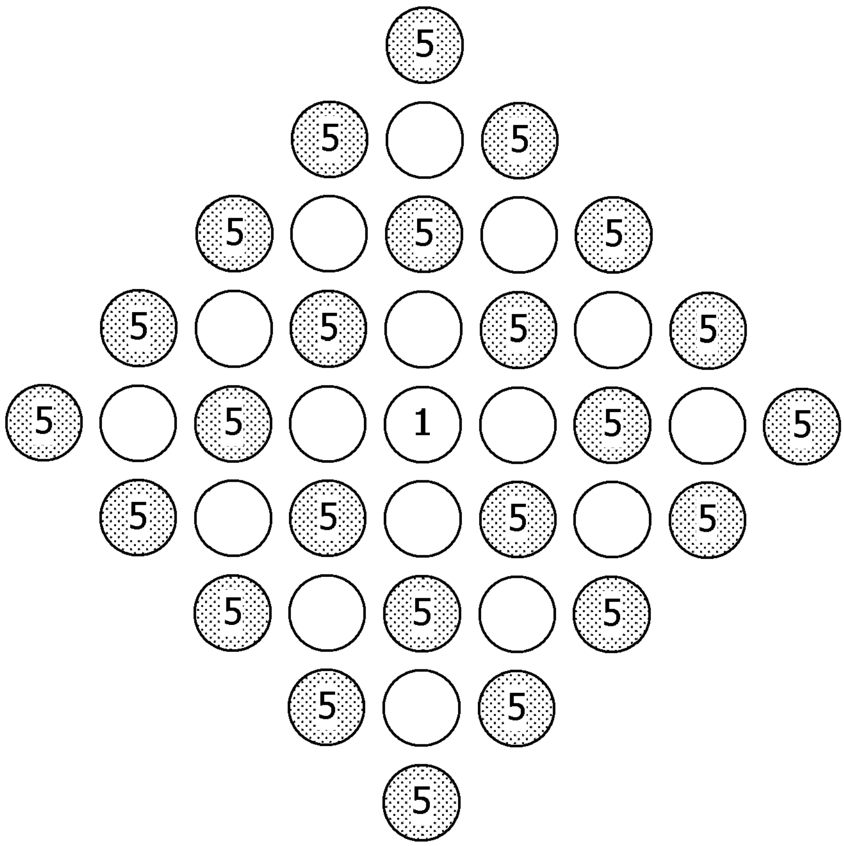

FIG. 14

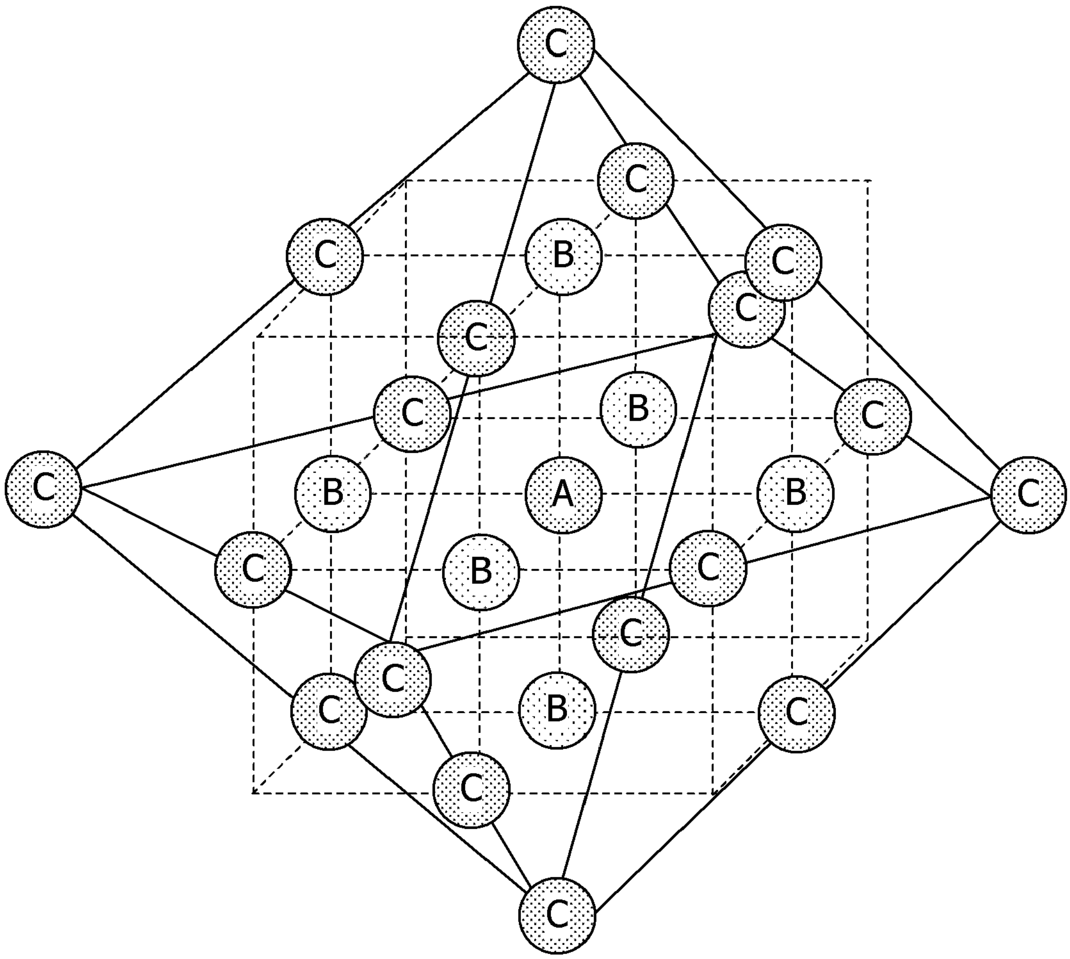

FIG. 15A

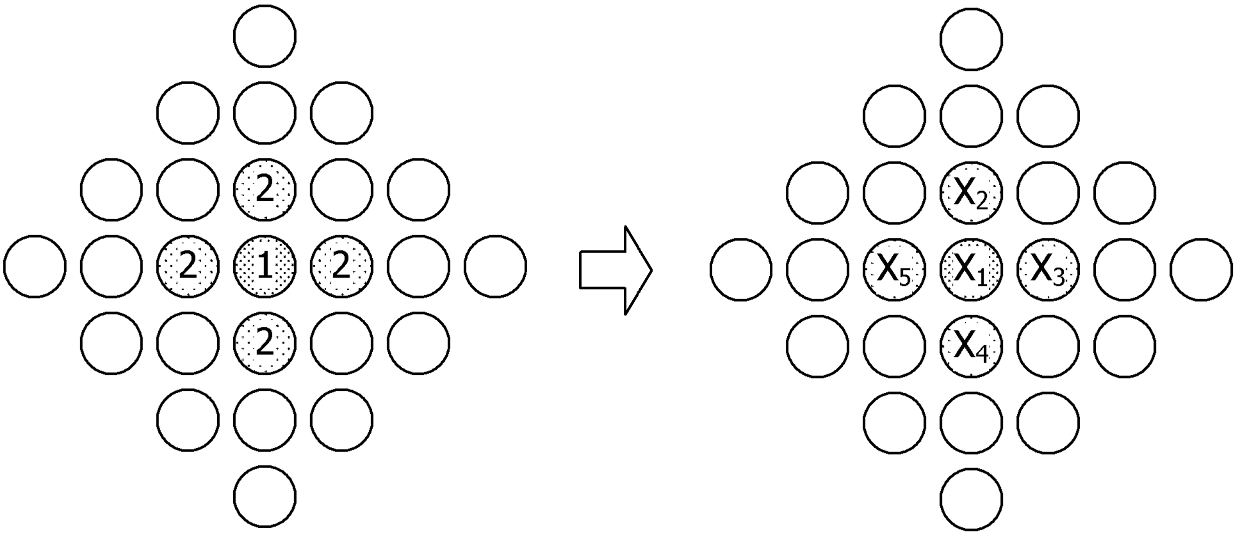


FIG. 15B

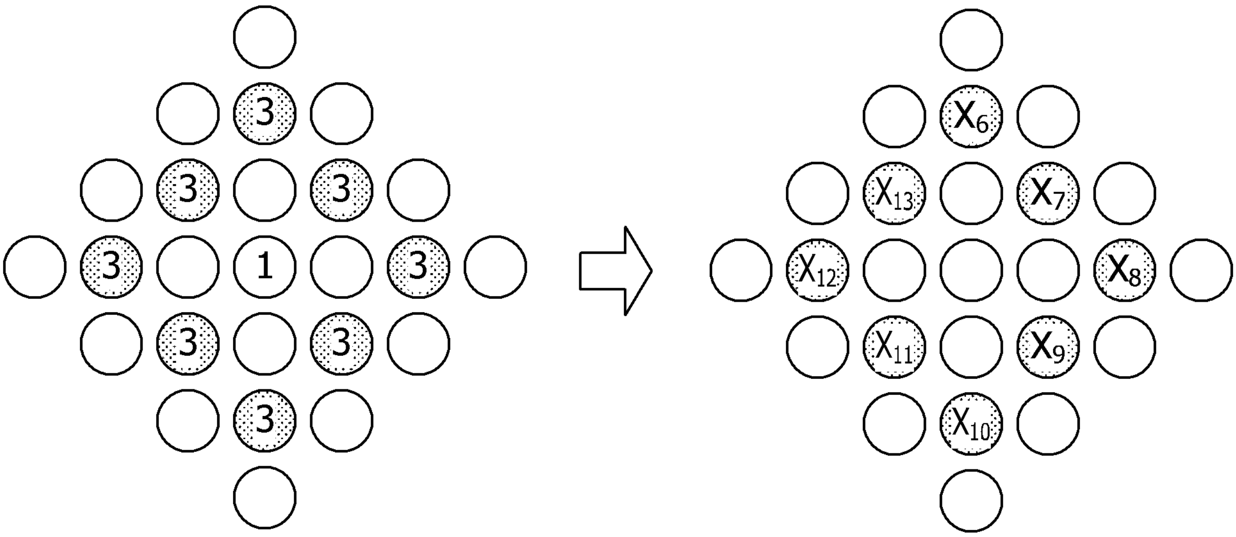

FIG. 15C

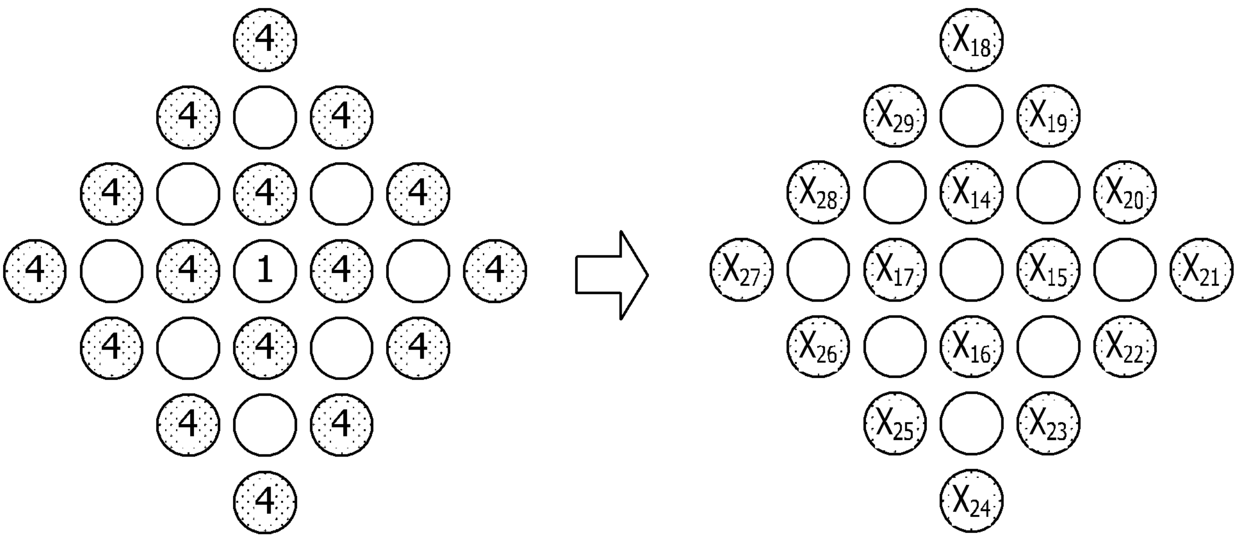
4
4
4
4
4
4
4
4
1
4
4
4
4
4
4
4
4
X18
X29
X19
X28
X14
X20
X27
X17
X15
X21
X26
X16
X22
X25
X23
X24

FIG. 16

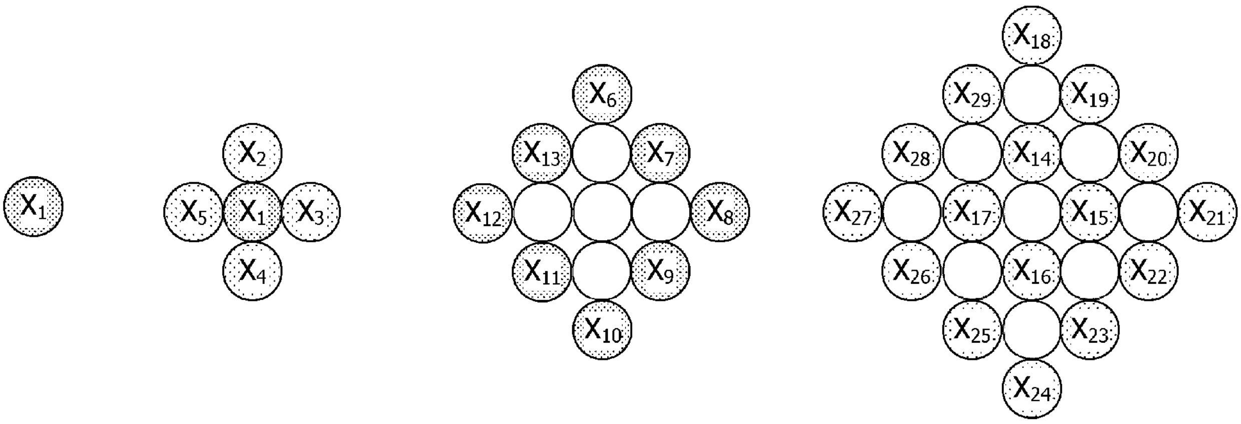
X1
X2
X5
X1
X3
X4
X6
X13
X7
X12
X8
X11
X9
X10
X18
X29
X19
X28
X14
X20
X27
X17
X15
X21
X26
X16
X22
X25
X23
X24

FIG. 17

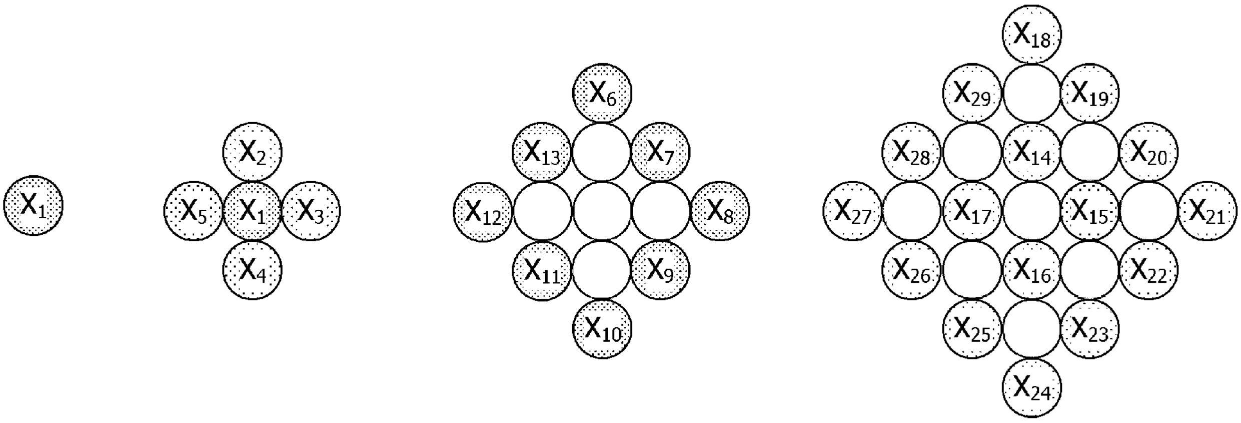
X1
X2
X5
X1
X3
X4
X6
X13
X7
X12
X8
X11
X9
X10
X18
X29
X19
X28
X14
X20
X27
X17
X15
X21
X26
X16
X22
X25
X23
X24

FIG. 18

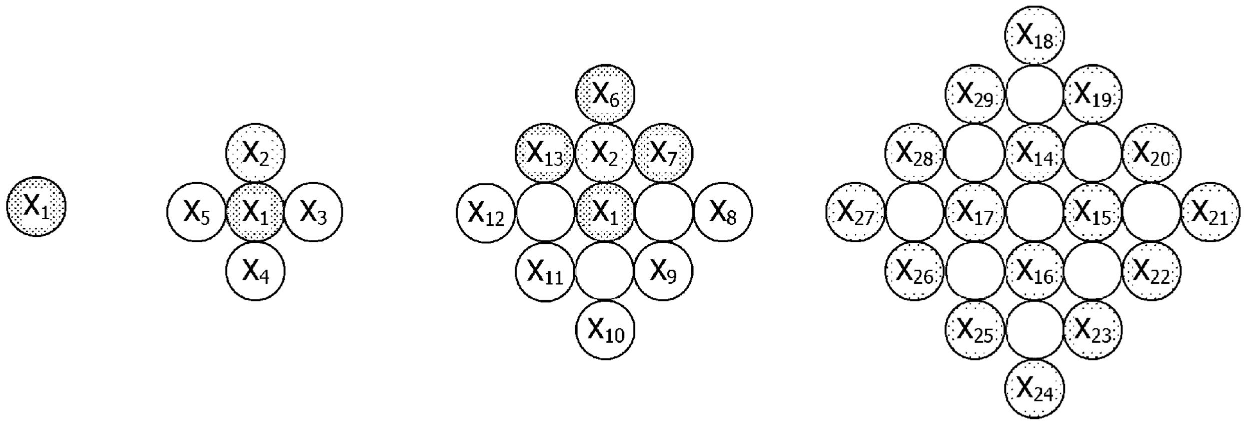
X1
X2
X5
X1
X3
X4
X6
X13
X2
X7
X12
X1
X8
X11
X9
X10
X18
X29
X19
X28
X14
X20
X27
X17
X15
X21
X26
X16
X22
X25
X23
X24

FIG. 19A

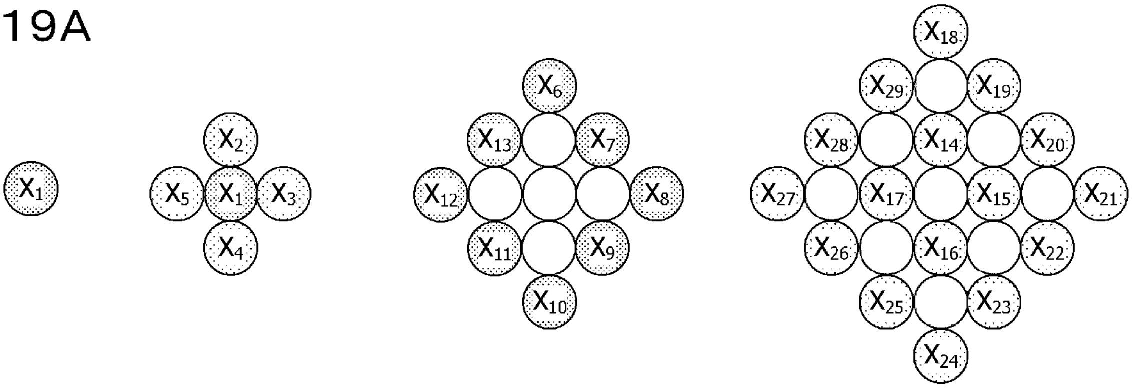


FIG. 19B

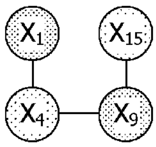

FIG. 20A

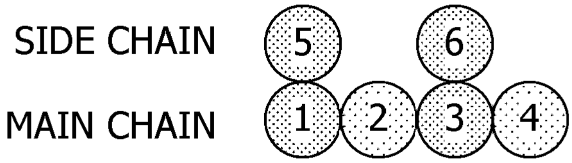


FIG. 20B

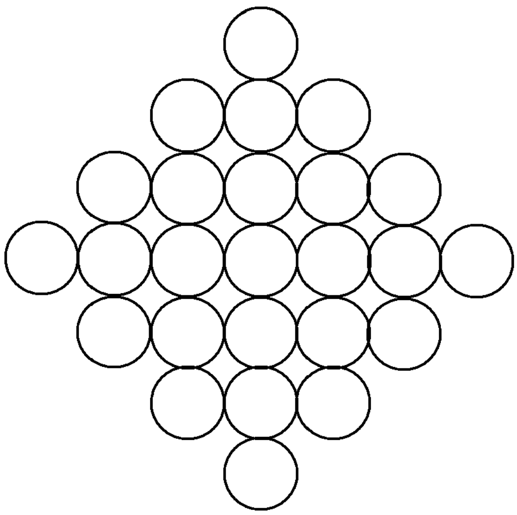

FIG. 20C

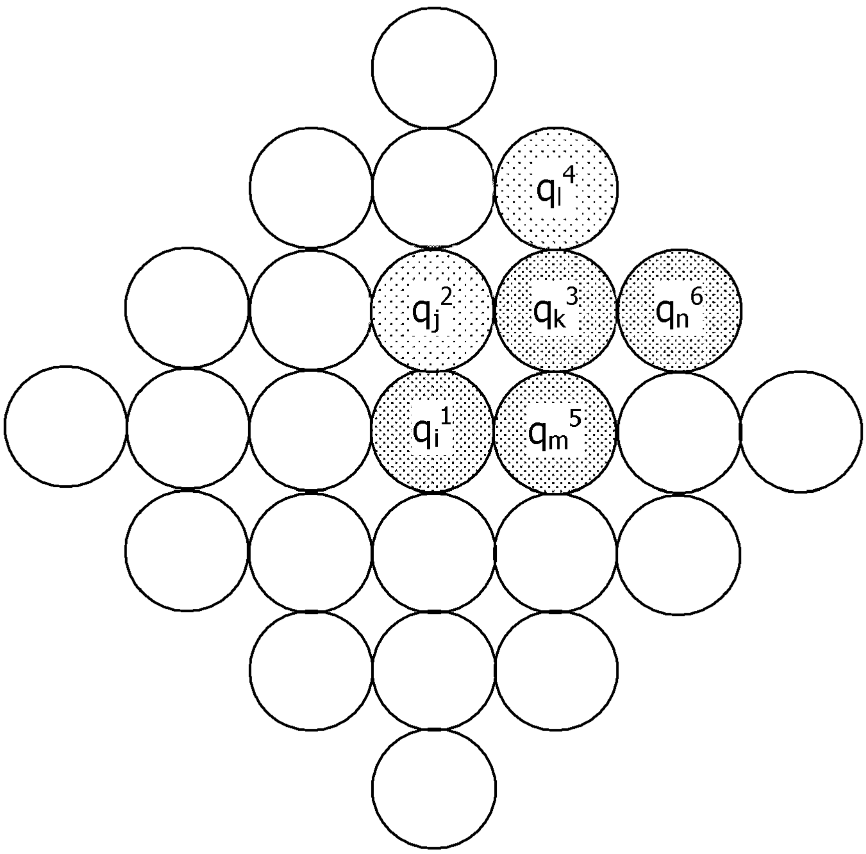

FIG. 21

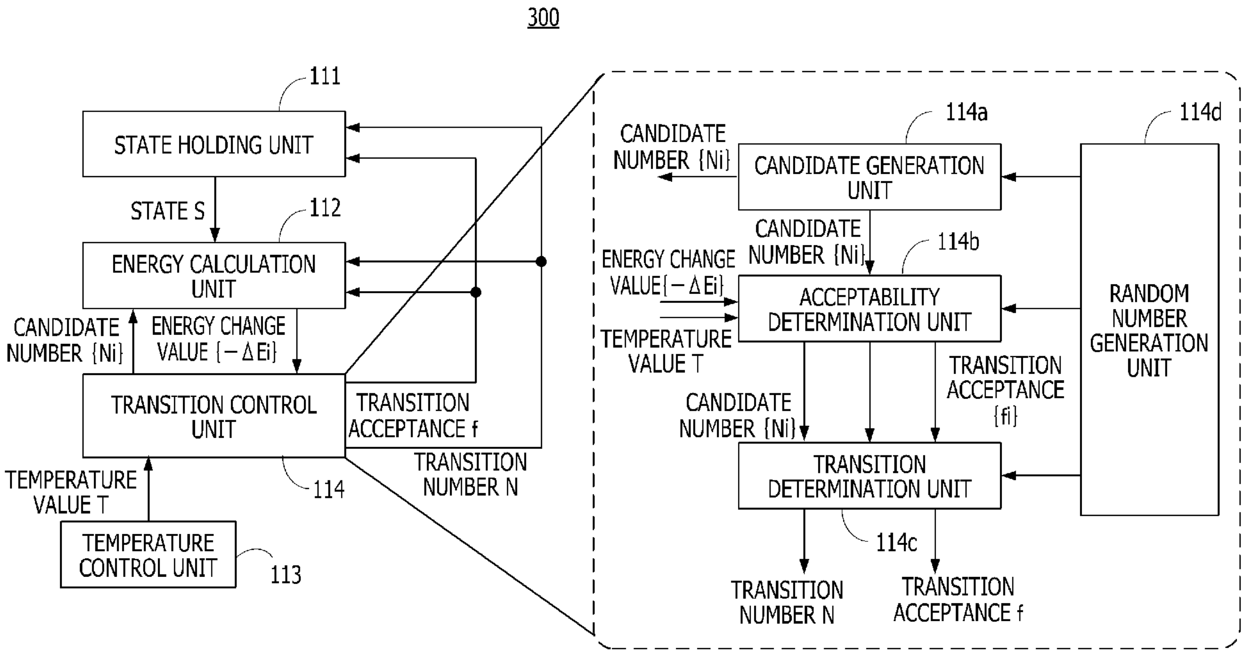
300
111
STATE HOLDING UNIT
STATE S
112
ENERGY CALCULATION UNIT
CANDIDATE NUMBER {Ni}
ENERGY CHANGE VALUE {−ΔEi}
TRANSITION CONTROL UNIT
TRANSITION ACCEPTANCE f
TRANSITION NUMBER N
114
TEMPERATURE VALUE T
TEMPERATURE CONTROL UNIT
113
114a
114d
CANDIDATE NUMBER {Ni}
CANDIDATE GENERATION UNIT
CANDIDATE NUMBER {Ni}
114b
ENERGY CHANGE VALUE{−ΔEi}
ACCEPTABILITY DETERMINATION UNIT
TEMPERATURE VALUE T
RANDOM NUMBER GENERATION UNIT
TRANSITION ACCEPTANCE {fi}
CANDIDATE NUMBER {Ni}
TRANSITION DETERMINATION UNIT
114c
TRANSITION NUMBER N
TRANSITION ACCEPTANCE f

FIG. 22

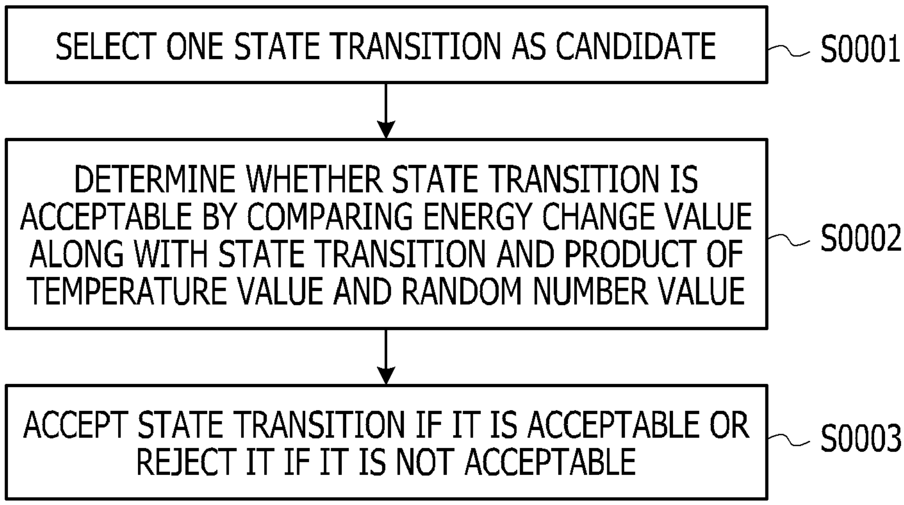
SELECT ONE STATE TRANSITION AS CANDIDATE
S0001
DETERMINE WHETHER STATE TRANSITION IS ACCEPTABLE BY COMPARING ENERGY CHANGE VALUE ALONG WITH STATE TRANSITION AND PRODUCT OF TEMPERATURE VALUE AND RANDOM NUMBER VALUE
S0002
ACCEPT STATE TRANSITION IF IT IS ACCEPTABLE OR REJECT IT IF IT IS NOT ACCEPTABLE
S0003

FIG. 23

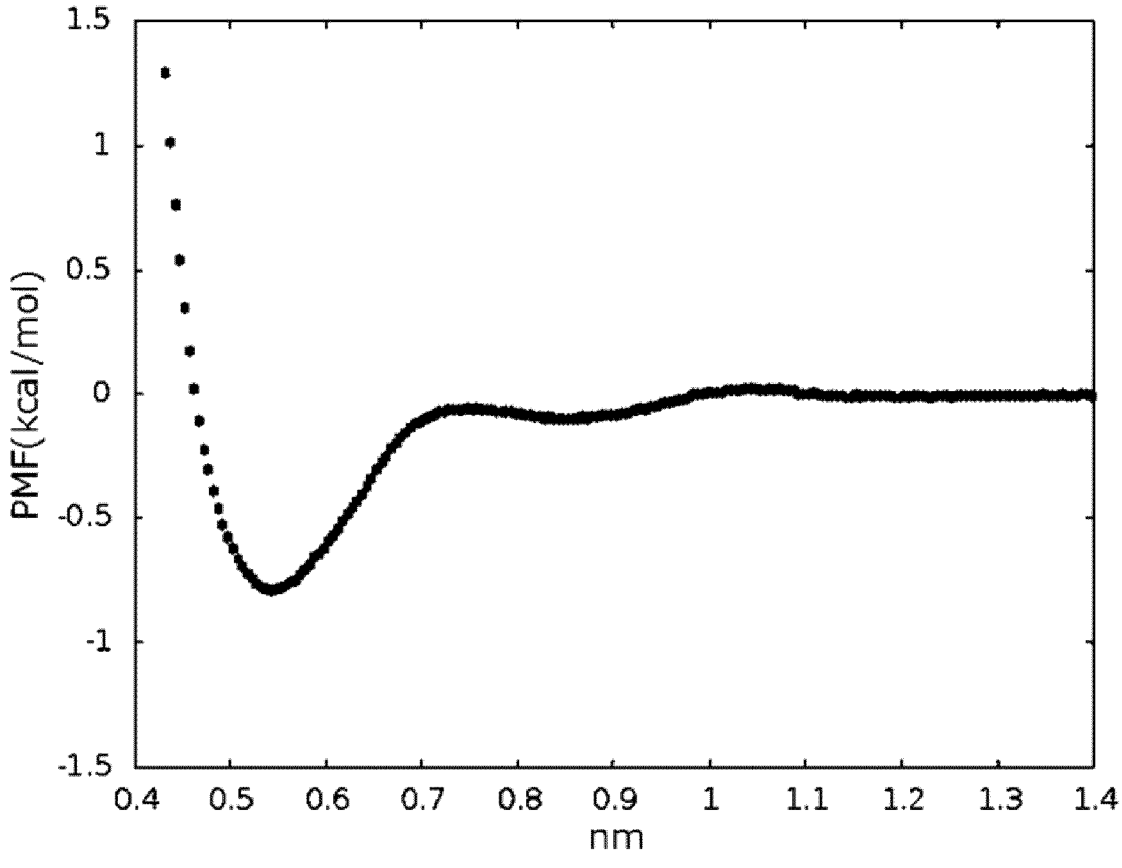

FIG. 24A

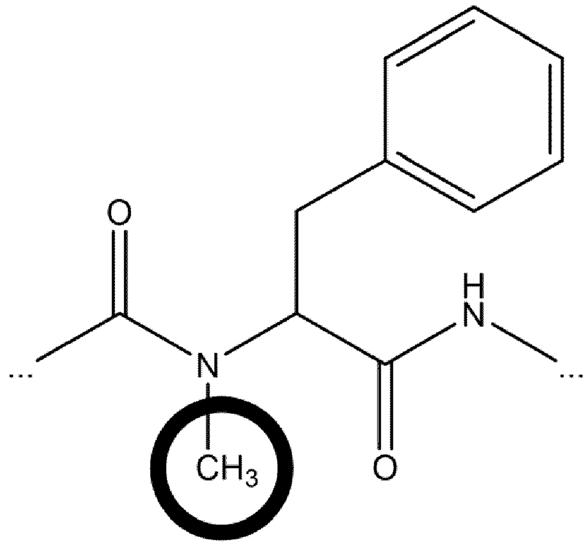

FIG. 24B

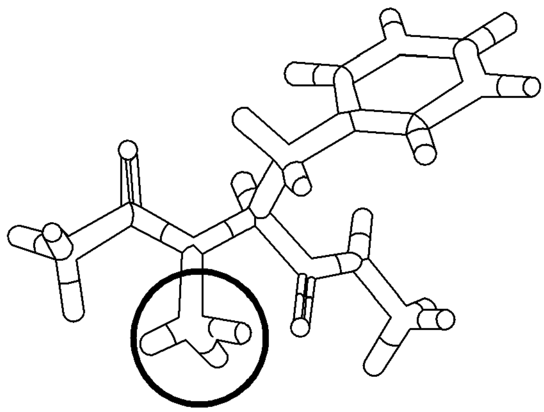

FIG. 25

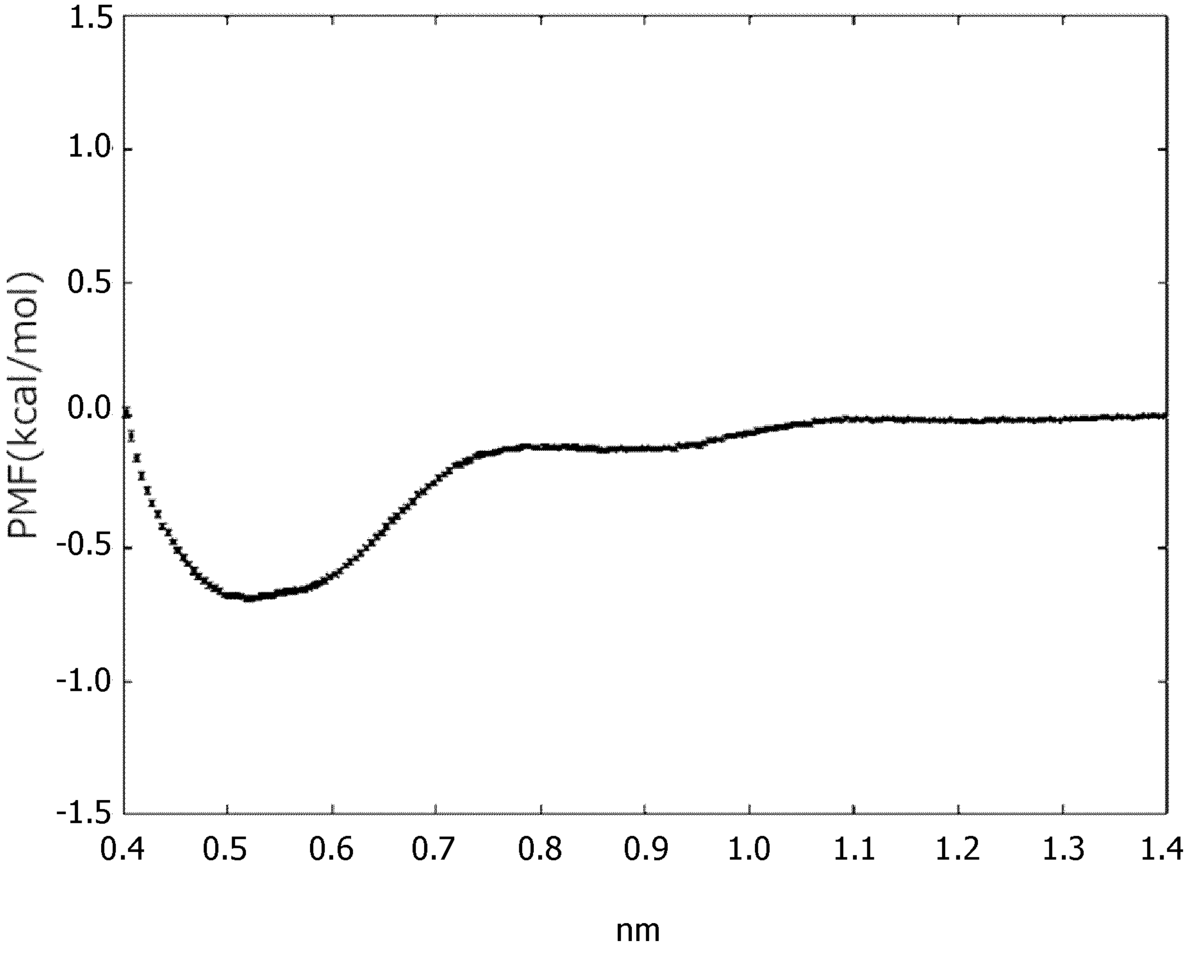

FIG. 26

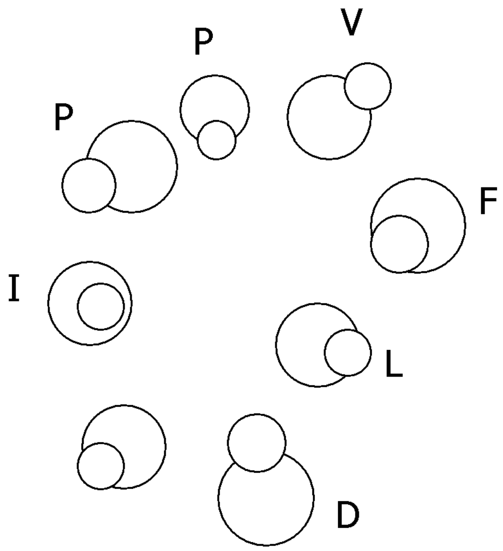

FIG. 27

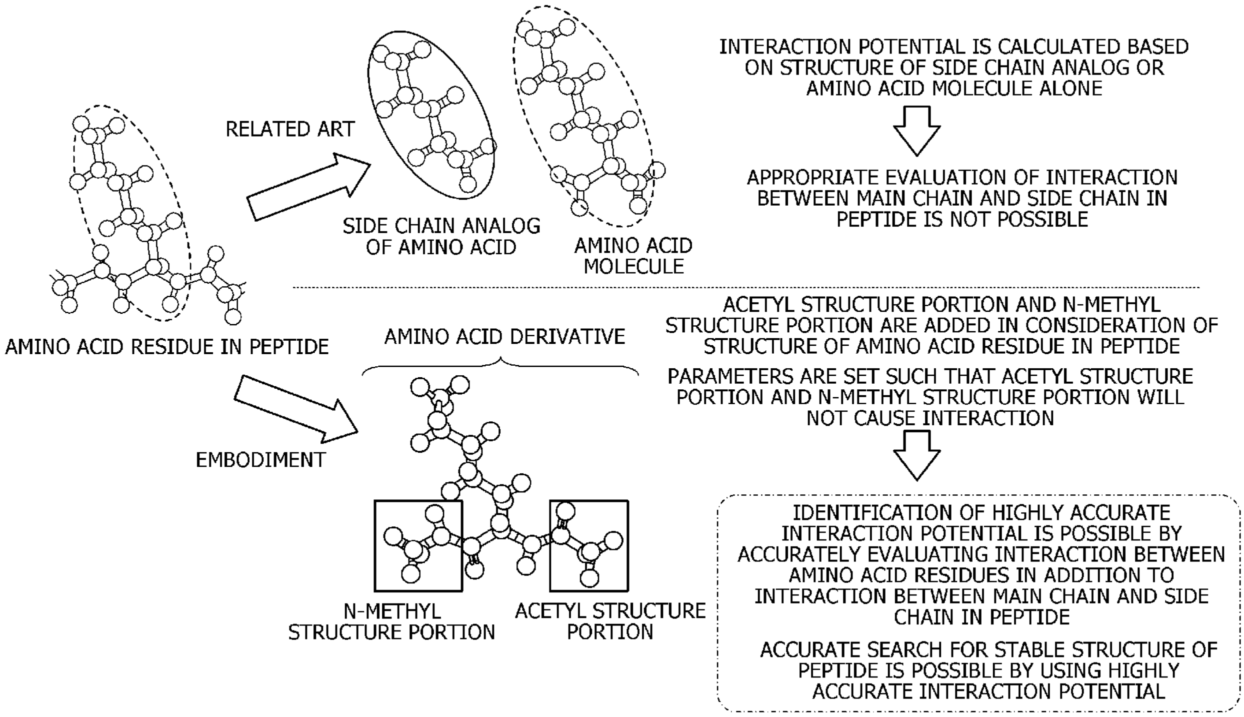
RELATED ART
SIDE CHAIN ANALOG OF AMINO ACID
AMINO ACID MOLECULE
INTERACTION POTENTIAL IS CALCULATED BASED ON STRUCTURE OF SIDE CHAIN ANALOG OR AMINO ACID MOLECULE ALONE
APPROPRIATE EVALUATION OF INTERACTION BETWEEN MAIN CHAIN AND SIDE CHAIN IN PEPTIDE IS NOT POSSIBLE
AMINO ACID RESIDUE IN PEPTIDE
AMINO ACID DERIVATIVE
ACETYL STRUCTURE PORTION AND N-METHYL STRUCTURE PORTION ARE ADDED IN CONSIDERATION OF STRUCTURE OF AMINO ACID RESIDUE IN PEPTIDE
PARAMETERS ARE SET SUCH THAT ACETYL STRUCTURE PORTION AND N-METHYL STRUCTURE PORTION WILL NOT CAUSE INTERACTION
EMBODIMENT
IDENTIFICATION OF HIGHLY ACCURATE INTERACTION POTENTIAL IS POSSIBLE BY ACCURATELY EVALUATING INTERACTION BETWEEN AMINO ACID RESIDUES IN ADDITION TO INTERACTION BETWEEN MAIN CHAIN AND SIDE CHAIN IN PEPTIDE
ACCURATE SEARCH FOR STABLE STRUCTURE OF PEPTIDE IS POSSIBLE BY USING HIGHLY ACCURATE INTERACTION POTENTIAL
N-METHYL STRUCTURE PORTION
ACETYL STRUCTURE PORTION

Europäisches Patentamt
European Patent Office
Office européen des brevets

# EUROPEAN SEARCH REPORT

Application Number
EP 21 15 8937

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2020/082904 A1 (UEMURA TAIKI [JP] ET AL) 12 March 2020 (2020-03-12)<br>* paragraphs [0068] - [0128], [0140] - [0144]; claims; figures *<br>----- | 1-20 | INV.<br>G16B15/20<br>G16C20/40 |
| Y | US 2020/176074 A1 (SATO HIROYUKI [JP]) 4 June 2020 (2020-06-04)<br>* paragraphs [0077] - [0115], [0127] - [0131]; claims; figures *<br>----- | 1-20 | |
| Y | TOMAS BABEJ ET AL: "Coarse-grained lattice protein folding on a quantum annealer",<br>ARXIV.ORG, CORNELL UNIVERSITY LIBRARY, 201 OLIN LIBRARY CORNELL UNIVERSITY ITHACA, NY 14853,<br>no. 1811.00713,<br>2 November 2018 (2018-11-02), XP080941413,<br>* Construction of Holap (p. 4); Construction of Hpair (p. 5); pages 2-3 *<br>----- | 1-20 | TECHNICAL FIELDS SEARCHED (IPC) |
| A | TOBI DROR ET AL: "Distance-dependent, pair potential for protein folding: Results from linear optimization",<br>PROTEINS: STRUCTURE, FUNCTION, AND BIOINFORMATICS,<br>vol. 41, no. 1,<br>1 October 2000 (2000-10-01), pages 40-46, XP55830998,<br>US<br>ISSN: 0887-3585, DOI: 10.1002/1097-0134(20001001)41:1<40::AID-PROT70>3.0.CO;2-U<br>* abstract *<br>-----<br>-/-- | 1-20 | G16B<br>G16C |

The present search report has been drawn up for all claims

1

| Place of search | Date of completion of the search | Examiner |
|---|---|---|
| Munich | 10 August 2021 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 15 8937

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SKOLNICK JEFFREY ET AL: "Derivation and testing of pair potentials for protein folding. When is the quasichemical approximation correct?", PROTEIN SCIENCE, vol. 6, no. 3, 1 March 1997 (1997-03-01), pages 676-688, XP55830924, US ISSN: 0961-8368, DOI: 10.1002/pro.5560060317 * abstract * ----- | 1-20 | |
| A | MIYAZAWA SANZO ET AL: "Estimation of effective interresidue contact energies from protein crystal structures: quasi-chemical approximation", MACROMOLECULES, vol. 18, no. 3, 1 March 1985 (1985-03-01), pages 534-552, XP55831084, Washington DC United States ISSN: 0024-9297, DOI: 10.1021/ma00145a039 * abstract * ----- | 1-20 | TECHNICAL FIELDS SEARCHED (IPC) |
| Y | VAITHEESWARAN S. ET AL: "Interactions between amino acid side chains in cylindrical hydrophobic nanopores with applications to peptide stability", PROCEEDINGS OF THE NATIONAL ACADEMY OF SCIENCES, vol. 105, no. 46, 18 November 2008 (2008-11-18), pages 17636-17641, XP55831134, US ISSN: 0027-8424, DOI: 10.1073/pnas.0803990105 Methods; * abstract * ----- -/-- | 1-20 | |

The present search report has been drawn up for all claims

1

| Place of search | Date of completion of the search | Examiner |
|---|---|---|
| Munich | 10 August 2021 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 15 8937

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FLOUDAS C A ET AL: "Advances in protein structure prediction and de novo protein design: A review", CHEMICAL ENGINEERING SCIENCE, OXFORD, GB, vol. 61, no. 3, 1 February 2006 (2006-02-01), pages 966-988, XP025012435, ISSN: 0009-2509, DOI: 10.1016/J.CES.2005.04.009 [retrieved on 2006-02-01] * the whole document * ----- | 1-20 | |
| X,P | EP 3 723 094 A1 (FUJITSU LTD [JP]) 14 October 2020 (2020-10-14) * claims; figures * ----- | 1-20 | |
| E | EP 3 826 022 A1 (FUJITSU LTD [JP]) 26 May 2021 (2021-05-26) * claims; figures * ----- | 1-20 | |
| | | | TECHNICAL FIELDS SEARCHED (IPC) |

1

The present search report has been drawn up for all claims

| Place of search | Date of completion of the search | Examiner |
|---|---|---|
| Munich | 10 August 2021 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 21 15 8937

This annex lists the patent family members relating to the patent documents cited in the above-mentioned European search report. The members are as contained in the European Patent Office EDP file on
The European Patent Office is in no way liable for these particulars which are merely given for the purpose of information.

10-08-2021

| Patent document cited in search report | Publication date | Patent family member(s) | Publication date |
| --- | --- | --- | --- |
| US 2020082904 A1 | 12-03-2020 | CN 110890126 A | 17-03-2020 |
| | | JP 2020042576 A | 19-03-2020 |
| | | US 2020082904 A1 | 12-03-2020 |
| US 2020176074 A1 | 04-06-2020 | CN 111261235 A | 09-06-2020 |
| | | EP 3667673 A1 | 17-06-2020 |
| | | JP 2020091518 A | 11-06-2020 |
| | | US 2020176074 A1 | 04-06-2020 |
| EP 3723094 A1 | 14-10-2020 | CN 111816257 A | 23-10-2020 |
| | | EP 3723094 A1 | 14-10-2020 |
| | | JP 2020173643 A | 22-10-2020 |
| | | US 2020327955 A1 | 15-10-2020 |
| EP 3826022 A1 | 26-05-2021 | CN 112837763 A | 25-05-2021 |
| | | EP 3826022 A1 | 26-05-2021 |
| | | JP 2021082165 A | 27-05-2021 |
| | | US 2021158891 A1 | 27-05-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BABBUSH RYAN et al.** Construction of Energy Functions for Lattice Heteropolymer Models: A Case Study in Constraint Satisfaction Programming and Adiabatic Quantum Optimization. *Advances in Chemical Physics,* vol. 155, 201-244
- **DROR TOBI.** Distance-Dependent, Pair Potential for Protein Folding: Results From Linear Optimization. *PROTEINS: Structure, Function, and Genetics,* 2000, vol. 41, 40-46
- **ANDREW POHORILLE.** Good Practices in Free-Energy Calculations. *J. Phys. Chem. B,* 2010, vol. 114, 10235-10253